(19) **Europäisches Patentamt / European Patent Office / Office européen des brevets**

(11) **EP 4 130 042 B1**

(12) # EUROPEAN PATENT SPECIFICATION

(45) Date of publication and mention of the grant of the patent:
**18.02.2026  Bulletin 2026/08**

(21) Application number: **21780305.5**

(22) Date of filing: **01.04.2021**

(51) International Patent Classification (IPC):
**C07K 16/28** *(2006.01)*    **C07K 16/46** *(2006.01)*
**C12N 15/13** *(2006.01)*    **C12N 15/63** *(2006.01)*
**A61K 39/395** *(2006.01)*    **A61P 35/00** *(2006.01)*
**A61P 35/02** *(2006.01)*

(52) Cooperative Patent Classification (CPC):
**A61P 35/00; A61P 35/02; C07K 16/28;**
**C07K 16/2803;** A01K 2207/12; A01K 2227/105;
A01K 2267/0331; A61K 2039/505; C07K 2317/24;
C07K 2317/73; C07K 2317/76; C07K 2317/92;
C07K 2317/94

(86) International application number:
**PCT/CN2021/084802**

(87) International publication number:
**WO 2021/197401 (07.10.2021 Gazette 2021/40)**

(54) **ANTIGEN-BINDING POLYPEPTIDE BINDING TO CD47, AND USE THEREOF**

AN CD47 BINDENDES ANTIGENBINDENDES POLYPEPTID UND VERWENDUNG DAVON

POLYPEPTIDE DE LIAISON À L'ANTIGÈNE SE LIANT À CD47, ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.04.2020  CN 202010255615**

(43) Date of publication of application:
**08.02.2023  Bulletin 2023/06**

(73) Proprietor: **CHIA TAI TIANQING PHARMACEUTICAL GROUP CO., LTD.**
**Lianyungang,**
**Jiangsu 222062 (CN)**

(72) Inventors:
• **GUI, Qiu**
**Nanjing, Jiangsu 211100 (CN)**
• **ZHU, Hong**
**Nanjing, Jiangsu 211100 (CN)**
• **WANG, Liangliang**
**Nanjing, Jiangsu 211100 (CN)**
• **ZHANG, Xiaoyun**
**Nanjing, Jiangsu 211100 (CN)**
• **ZOU, Xiaofang**
**Nanjing, Jiangsu 211100 (CN)**
• **SHEN, Hengqiao**
**Nanjing, Jiangsu 211100 (CN)**
• **XU, Hongjiang**
**Nanjing, Jiangsu 211100 (CN)**
• **YANG, Ling**
**Nanjing, Jiangsu 211100 (CN)**

(74) Representative: **Geling, Andrea**
**ZSP Patentanwälte PartG mbB**
**Hansastraße 32**
**80686 München (DE)**

(56) References cited:
CN-A- 105 102 479      CN-A- 108 779 179
CN-A- 110 066 336      CN-A- 110 872 350
US-A1- 2015 183 874

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 130 042 B1

- **D. TSENG ET AL: "Anti-CD47 antibody-mediated phagocytosis of cancer by macrophages primes an effective antitumor T-cell response", PROCEEDINGS OF THE NATIONAL ACADEMY OF SCIENCES, vol. 110, no. 27, 20 May 2013 (2013-05-20), pages 11103 - 11108, XP055245737, ISSN: 0027-8424, DOI: 10.1073/pnas.1305569110**
- **GHOLAMIN SHARAREH ET AL: "Disrupting the CD47-SIRPa anti-phagocytic axis by a humanized anti-CD47 antibody is an efficacious treatment for malignant pediatric brain tumors", 15 March 2017 (2017-03-15), XP093141173, Retrieved from the Internet <URL:https://pubmed.ncbi.nlm.nih.gov/28298418/> [retrieved on 20240313]**

Remarks:
> The complete document including Reference Table(s) and the Sequence Listing(s) can be downloaded from the EPO website

Remarks:

## Description

### TECHNICAL FIELD

The present disclosure relates to an antigen-binding polypeptide, in particular to an antigen-binding polypeptide that binds to CD47 and an antigen-binding portion thereof. Such antigen-binding polypeptides and antigen-binding portions thereof are further used in a method for reducing a tumor or inhibiting tumor cell growth in a subject, for treating cancer in a subject, or for promoting macrophage phagocytosis in a subject. The antigen-binding polypeptide is a monoclonal antibody.

### BACKGROUND

CD47, also known as integrin-associated protein, is a transmembrane glycoprotein widely expressed among a variety of species and tissues. It is a member of the immunoglobulin superfamily. CD47 weighs about 50 kD and comprises 1 extracellular Ig-like variable domain, 5 highly hydrophobic extended transmembrane segments, and 1 short alternatively spliced carboxy-terminal cytoplasmic tail.

Inhibitory receptor signal-regulatory protein $\alpha$ (SIRP$\alpha$) is one of the ligands of CD47, which binds to the $NH_2$-terminal IgV-like domain of SIRP$\alpha$. SIRP$\alpha$ is expressed primarily in myeloid-derived cells, including macrophages, granulocytes, DC cells, mast cells, and their precursors, including hematopoietic stem cells. For example, CD47 may inhibit the phagocytic function of macrophages by binding to the protein SIRP$\alpha$ on the macrophage surface. It has been shown that blocking CD47-mediated binding to SIRP$\alpha$ on phagocytes or loss of expression in CD47 knockout mice may cause the removal of viable cells and non-aged red cells. For those cells in which pre-phagocytic signals are still present, blocking SIRP$\alpha$ also allows the phagocytosis of targets that are not normally phagocytosed. Programmed cell death (PCD) and phagocytic removal are common ways in which organisms respond to remove damaged, precancerous or infected cells. Thus, there are ways for cells that survive this organism response (cancerous cells, chronically infected cells, etc.) to evade PCD and phagocytic removal. CD47 is constitutively upregulated in various types of diseased, cancerous and infected cells, thereby allowing these cells to evade phagocytosis. Anti-CD47 compounds that block the interaction between CD47 on one cell (cancerous cell, chronically infected cell, etc.) and SIRP$\alpha$ on another cell (phagocyte) can counteract the increase in CD47 expression and promote phagocytosis of cancer cells and/or infected cells.

CD47 expression and/or activity has been implicated in a number of diseases and disorders.

Tseng D. et al. (Proceedings of the National Academy of Sciences, Vol. 110, No. 27, May 20, 2013, pages 11103 to 11108) teach that anti-CD47 antibody-mediated phagocytosis of cancer by macrophages primes an effective antitumor T-cell response.

Cholamin, Shararch et al. (Science Translational Medicine, March 15, 2017) describe that disrupting the CD47-STRPa anti-phagocytic axis by a humanized anti-CD47 antibody is an efficacious treatment for malignant pediatric brain tumors.

US 2015/183874 A1 also relates to humanized or chimeric anti-CD47 monoclonal antibodies.

Different studies have shown that almost all tumor cells and tumor tissues highly express CD47. CD47 highly expressed on the tumor cell surface binds to SIRP$\alpha$ on the macrophage surface, sending a "don't eat me" signal, so that the macrophages in the tumor tissue infiltration area get along well with the tumor cells and even facilitate the expansion and growth of the tumor cells by facilitating the proliferation of blood vessels in tumors and suppressing effector T cells. There is thus a need for CD47-targeted therapy.

### SUMMARY

The present disclosure provides a novel isolated antigen-binding polypeptide that binds to CD47 and an antigen-binding portion thereof as a solution to CD47-targeted therapy.

The invention is set out in the appended set of claims.

Specifically, the present invention relates to an isolated antigen-binding polypeptide that binds to CD47 or an antigen-binding portion thereof, wherein the antigen-binding polypeptide comprises the following complementarity determining regions:

a heavy chain CDR1 comprising an amino acid sequence set forth in SEQ ID NO: 5;
a heavy chain CDR2 comprising an amino acid sequence set forth in SEQ ID NO: 10;
a heavy chain CDR3 comprising an amino acid sequence set forth in SEQ ID NO: 15;
a light chain CDR1 comprising an amino acid sequence set forth in SEQ ID NO: 20;
a light chain CDR2 comprising an amino acid sequence set forth in SEQ ID NO: 25; and
a light chain CDR3 comprising an amino acid sequence set forth in SEQ ID NO: 30,

wherein the antigen-binding polypeptide is a monoclonal antibody.

The present invention relates to an isolated antigen-binding polypeptide that binds to CD47 or an antigen-binding portion thereof, wherein the antigen-binding polypeptide comprises a heavy chain set forth in SEQ ID NO: 181 and a light chain set forth in SEQ ID NO: 183.

Further, the present invention relates to an immunoconjugate comprising the above antigen-binding polypeptide or the antigen-binding portion thereof and a therapeutic agent linked or conjugated to the antigen-binding polypeptide or the antigen-binding portion thereof.

Furthermore, the present invention relates to a composition comprising a component A and a pharmaceutically acceptable carrier, wherein the component A is the above antigen-binding polypeptide or the antigen-binding portion thereof or the above immunoconjugate.

The present invention also relates to an isolated nucleic acid encoding the above antigen-binding polypeptide or the antigen-binding portion thereof.

The present invention also relates to a vector comprising the above isolated nucleic acid.

The present invention also relates to a host cell comprising the above vector or into whose genome the above isolated nucleic acid is integrated.

In addition, the present invention relates to the above antigen-binding polypeptide or the antigen-binding portion thereof, the above immunoconjugate, or the above composition for use in a method for reducing a tumor or inhibiting tumor cell growth in a subject, in a method for treating cancer in a subject in need thereof, or in a method for promoting macrophage phagocytosis in a subject.

Further described herein, but not encompassed by the present invention, is an isolated antigen-binding polypeptide that binds to CD47 or an antigen-binding portion thereof, wherein the antigen-binding polypeptide comprises the following complementarity determining regions:

a heavy chain CDR1 comprising an amino acid sequence having at least 80% identity to an amino acid sequence selected from SEQ ID NOs: 1, 2, 3, 4 and 5;
a heavy chain CDR2 comprising an amino acid sequence having at least 80% identity to an amino acid sequence selected from SEQ ID NOs: 6, 7, 8, 9 and 10;
a heavy chain CDR3 comprising an amino acid sequence having at least 80% identity to an amino acid sequence selected from SEQ ID NOs: 11, 12, 13, 14 and 15;
a light chain CDR1 comprising an amino acid sequence having at least 80% identity to an amino acid sequence selected from SEQ ID NOs: 16, 17, 18, 19 and 20;
a light chain CDR2 comprising an amino acid sequence having at least 80% identity to an amino acid sequence selected from SEQ ID NOs: 21, 22, 23, 24 and 25; and
a light chain CDR3 comprising an amino acid sequence having at least 80% identity to an amino acid sequence selected from SEQ ID NOs: 26, 27, 28, 30 and 32;
wherein the sequence set forth in SEQ ID NO: 32 is QQFSX$_2$STWT, wherein X$_2$ is D or E.

Further described herein, but not encompassed by the present invention, is an isolated antigen-binding polypeptide that binds to CD47 or an antigen-binding portion thereof, wherein the antigen-binding polypeptide comprises the following complementarity determining regions:

a heavy chain CDR1 comprising an amino acid sequence selected from SEQ ID NOs: 1, 2, 3, 4 and 5 or a conservatively modified form thereof;
a heavy chain CDR2 comprising an amino acid sequence selected from SEQ ID NOs: 6, 7, 8, 9 and 10 or a conservatively modified form thereof;
a heavy chain CDR3 comprising an amino acid sequence selected from SEQ ID NOs: 11, 12, 13, 14 and 15 or a conservatively modified form thereof;
a light chain CDR1 comprising an amino acid sequence selected from SEQ ID NOs: 16, 17, 18, 19 and 20 or a conservatively modified form thereof;
a light chain CDR2 comprising an amino acid sequence selected from SEQ ID NOs: 21, 22, 23, 24 and 25 or a conservatively modified form thereof; and
a light chain CDR3 comprising an amino acid sequence selected from SEQ ID NOs: 26, 27, 28, 30 and 32 or a conservatively modified form thereof.

Further described herein, but not encompassed by the present invention, is an isolated antigen-binding polypeptide that binds to CD47 or an antigen-binding portion thereof, wherein the antigen-binding polypeptide comprises a heavy chain variable region comprising an amino acid sequence having at least 80% identity to an amino acid sequence selected from SEQ ID NOs: 33, 35, 37, 39, 41, 83, 85 and 87, and a light chain variable region comprising an amino acid sequence having at least 80% identity to an amino acid sequence selected from SEQ ID NOs: 34, 36, 38, 40, 42, 84, 86 and 88,

wherein the sequence set forth in SEQ ID NO: 83 is:
QVQLVQSGAEVKKPGSSVKVSCKASGYTFSRYWIEWVRQAPGQGLEWMGEFIPGSDTTNYAQKFQGRV TITA $X_3X_4STX_5$TAYMELSSLRSEDTAVYYCARGGLRRMDYWGQGTLVTVSS, wherein $X_3$ is D or E, $X_4$ is I or E, and $X_5$ is S or N;

the sequence set forth in SEQ ID NO: 84 is:
DIQMTQSPSSLSASVGDRVTITCRASSSVSSTYLHWYQQKPGKAPKL$X_6$IYTTSTLASGVPSRFSGSGSGTX $_7X_8$TLTISSLQPEDFATYYCQQFS$X_2$STWTFGQGTKLEIK, wherein $X_6$ is L or W, $X_7$ is D or S, $X_8$ is F or Y, and $X_2$ is D or E;

the sequence set forth in SEQ ID NO: 85 is:
QVQLVQSGAEVKKPGASVKVSCKASGYTFTNYGMNWVRQAPGQGL$X_9$WMGWINTNTGEPTYAQX$_{10}$L QGRVT MTX$_{11}$DTSTX$_{12}$TAYMELRSLRSDDTAVYYCX$_{13}$RFSHLRGPMDYWGQGTLVTVSS, wherein $X_9$ is E or K, $X_{10}$ is K or E, $X_{11}$ is L or T, $X_{12}$ is R or S, and $X_{13}$ is A or T;

the sequence set forth in SEQ ID NO: 86 is:
DX$_{14}$QMTQSPSSLSASVGDRVTITCRSSQSLVHSNGYTYLHWYQQKPGKAPKLLIYKVSNRFSGVPSRFSG SGSGTDFTLTISSLQPEDFATYYCSQSTHVPPTFGQGTKLEIK, wherein $X_{14}$ is I or A;

the sequence set forth in SEQ ID NO: 87 is:
QVQLVQSGAEVKKPGASVKVSCKX$_{15}$SGFNIEDDYIEWVRQAPGQGLEWMGRIDPANDKTKYAQKFQG RVTMT X$_{16}$DTSTX$_{17}$TVYMELSSLRSEDTAVYYCX$_{18}$RPGLRRYYSMDYWGQGTLVTVSS, wherein $X_{15}$ is A or V, $X_{16}$ is R or G, $X_{17}$ is S or N, and $X_{18}$ is A or T;

the sequence set forth in SEQ ID NO: 88 is:
DIQMTQSPSSLSASVGDRVTITCKASENVVSYVSWYQQKPGKAPKLLIYGASNRYTGVPSRFX$_{19}$GSGSX$_{20}$ TDFTLTISSLQPEDFATYYCGQSYSYPLTFGQGTKLEIK, wherein $X_{19}$ is S or I, and $X_{20}$ is S or G.

Further described herein, but not encompassed by the present invention, is an isolated antigen-binding polypeptide that binds to CD47 or an antigen-binding portion thereof, wherein the antigen-binding polypeptide or the antigen-binding portion thereof has one of, or a combination of several of, the following properties:

(a) binding to CD47 with a $K_D$ value of 1.89E-08 or less (e.g., 1.53E-08 or less);
(b) blocking binding of CD47 to SIRPα;
(c) promoting macrophage-mediated phagocytosis of CD47-expressing cells;
(d) not significantly inducing apoptosis of CD4+ T cells;
(e) not causing substantial hematocytopenia, anemia or red cell agglutination; and
(f) the antigen-binding polypeptide or the antigen-binding portion thereof having a melting temperature T $\geq$ 62 °C and an aggregation temperature Tagg $\geq$ 61 °C.

Further described herein, but not encompassed by the present invention, is an isolated antigen-binding polypeptide or an antigen-binding portion thereof, wherein the antigen-binding polypeptide or the antigen-binding portion thereof binds to the same epitope on CD47 as any of the exemplary antigen-binding polypeptides or the antigen-binding portions thereof provided herein.

Further described herein, but not encompassed by the present invention, is an isolated antigen-binding polypeptide or an antigen-binding portion thereof, wherein the antigen-binding polypeptide or the antigen-binding portion thereof competes for binding to CD47 with any of the exemplary antigen-binding polypeptides or the antigen-binding portions thereof provided herein, wherein the competition is measured by ELISA, flow cytometry or surface plasmon resonance assay.

Herein, the antigen-binding portion may be selected from Fab fragment, Fab' fragment, F(ab')$_2$ fragment, Fd fragment, Fv fragment, dAb fragment, isolated complementarity determining region and nanobody, but is not limited thereto.

Further described herein, but not encompassed by the present invention, is an immunoconjugate comprising the antigen-binding polypeptide that binds to CD47 or the antigen-binding portion thereof described herein and a therapeutic agent linked or conjugated to the antigen-binding polypeptide or the antigen-binding portion thereof. Herein, the antigen-binding polypeptide or the antigen-binding portion thereof may be linked to the therapeutic agent by a linker. The linker may be cleavable or non-cleavable.

Herein, the therapeutic agent may be selected from cytotoxic drugs, radioisotopes or immunomodulators, e.g., chemotherapeutic agents, immunosuppressive agents, immunostimulants, antimetabolites, alkylating agents, antibiotics, anti-angiogenic agents, antimitotic agents, toxins, and apoptotic agents.

Further described herein, but not encompassed by the present invention, is a composition comprising a component A and a pharmaceutically acceptable carrier, wherein the component A is the antigen-binding polypeptide that binds to CD47 or the antigen-binding portion thereof described herein, or the immunoconjugate described herein. Further described herein, but not encompassed by the present invention, is an isolated hybridoma cell line selected from the group consisting of 2B2, 2H8, 3F10, 16E5 and 14A9.

Further described herein, but not encompassed by the present invention, is an isolated nucleic acid encoding the antigen-binding polypeptide that binds to CD47 or the antigen-binding portion thereof described herein.

Further described herein, but not encompassed by the present invention, is a vector comprising the isolated nucleic acid described herein.

Further described herein, but not encompassed by the present invention, is a host cell comprising the vector described herein or into whose genome the isolated nucleic acid described herein is integrated. Preferably, the vector is an expression vector.

Further described herein, but not encompassed by the present invention, is a method for preparing the antigen-binding polypeptide that binds to CD47 or the antigen-binding portion thereof, which comprises: culturing the host cell described herein under conditions suitable for expression of the nucleic acid encoding the antigen-binding polypeptide that binds to CD47 or the antigen-binding portion thereof described herein, and isolating the antigen-binding polypeptide or the antigen-binding portion thereof expressed.

Further described herein, but not encompassed by the present invention, is a method for reducing a tumor or inhibiting tumor cell growth in a subject, which comprises administering to the subject a therapeutically effective amount of the antigen-binding polypeptide that binds to CD47 or the antigen-binding portion thereof described herein, the immunoconjugate described herein, or the composition described herein.

Further described herein, but not encompassed by the present invention, is a method for treating cancer in a subject in need thereof, which comprises administering to the subject a therapeutically effective amount of the antigen-binding polypeptide that binds to CD47 or the antigen-binding portion thereof described herein, the immunoconjugate described herein, or the composition described herein.

Further described herein, but not encompassed by the present invention, is a method for promoting phagocytosis by macrophages in a subject, which comprises administering to the subject an effective amount of the antigen-binding polypeptide that binds to CD47 or the antigen-binding portion thereof described herein, the immunoconjugate described herein, or the composition described herein.

Any references to methods of treatment in this description are to be interpreted as references to the compounds of the present invention for use in a method for treatment of the human (or animal) body by therapy.

## BRIEF DESCRIPTION OF THE DRAWINGS

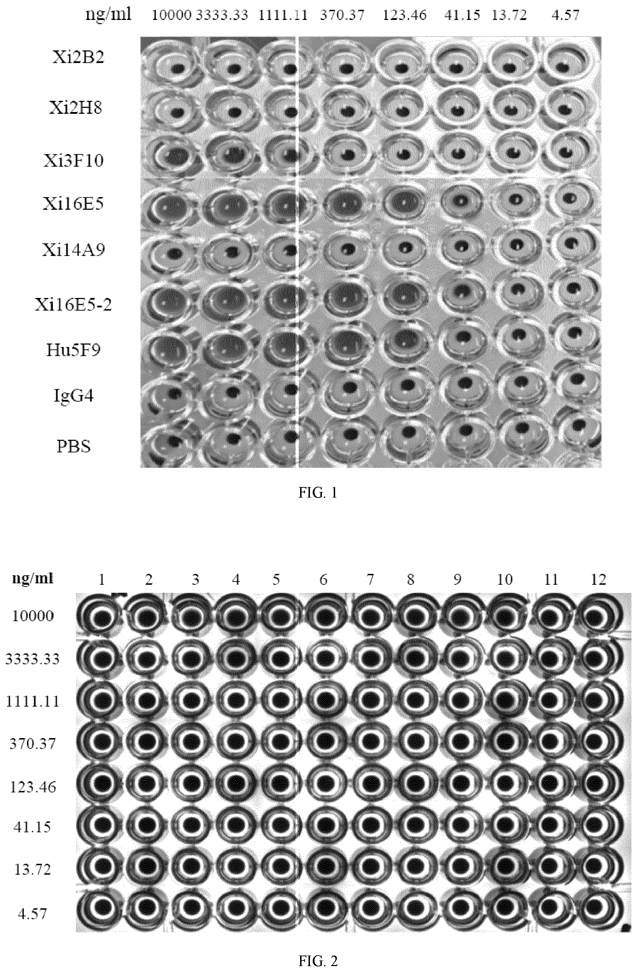

FIG. 1 shows the effects of anti-CD47 chimeric antibodies on red cell agglutination, wherein the samples (antibodies or controls) in the same row are of the same type but are at different concentrations, and the samples in the same column are at the same concentration but are of different types; the concentrations of the samples in different columns are, from left to right, 10,000 ng/mL, 3333.33 ng/mL, 1111.11 ng/mL, 370.37 ng/mL, 123.46 ng/mL, 41.15 ng/mL, 13.72 ng/mL and 4.57 ng/mL, respectively; the samples in different rows are, from top to bottom, Xi2B2, Xi2H8, Xi3F10, Xi16E5, Xi14A9, Xi16E5-2 (another replicate of Xi16E5), Hu5F9, IgG4 and PBS, respectively.

FIG. 2 shows the effects of some anti-CD47 humanized antibodies on red cell agglutination, wherein the samples in the same column are of the same type but are at different concentrations, and the samples in the same row are at the same concentration but are of different types; the concentrations of the antibodies in different rows are, from top to bottom, 10,000 ng/mL, 3333.33 ng/mL, 1111.11 ng/mL, 370.37 ng/mL, 123.46 ng/mL, 41.15 ng/mL, 13.72 ng/mL and 4.57 ng/mL, respectively; the antibodies in columns 1-12 are, from left to right, hz3F10-1.1, hz3F10-2.1, hz3F10-3.1, hz3F10-4.1, hz3F10-5.1, hz3F10-6.1, hz3F10-1.2, hz3F10-2.2, hz3F10-3.2, hz3F10-4.2, hz3F10-5.2 and hz3F10-6.2, respectively.

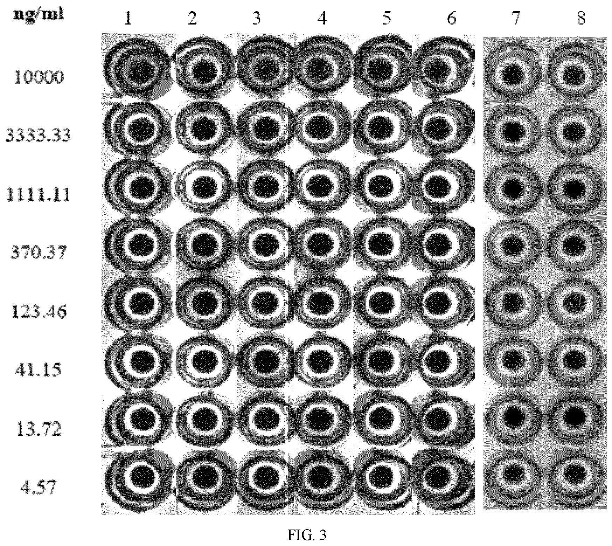

FIG. 3 shows the effects of some anti-CD47 humanized antibodies on red cell agglutination, wherein the samples in the same column are of the same type but are at different concentrations, and the samples in the same row are at the same concentration but are of different types; the concentrations of the antibodies in different rows are, from top to bottom, 10,000 ng/mL, 3333.33 ng/mL, 1111.11 ng/mL, 370.37 ng/mL, 123.46 ng/mL, 41.15 ng/mL, 13.72 ng/mL and 4.57 ng/mL, respectively; the antibodies in columns 1-8 are, from left to right, hz16E5-1.1, hz16E5-3.1, hz16E5-1.2, hz16E5-3.2, hz16E5-1.3, hz16E5-3.3, hz14A9-2.3 and hz14A9-2.4, respectively.

FIG. 4 shows the effects of anti-CD47 antibodies on mouse survival rate.

FIG. 5 shows the changes in mouse body weight over time following single administration of anti-CD47 antibodies.

FIG. 6 shows the rates of change in mouse body weight at different time points following single administration of anti-CD47 antibodies.

FIG. 7 shows the changes in mouse RBC over time following single administration of anti-CD47 antibodies.

FIG. 8 shows the rates of change in mouse RBC at different time points following single administration of anti-CD47 antibodies.

FIG. 9 shows the changes in mouse HGB content over time following single administration of anti-CD47 antibodies.

FIG. 10 shows the rates of change in mouse HGB content at different time points following single administration of anti-CD47 antibodies.

FIG. 11 shows the changes in mean tumor volume over time following administration of anti-CD47 antibodies in a mouse model of human acute myeloid leukemia cells MOLM-16 cells.

## DETAILED DESCRIPTION

The exemplary embodiments of the present disclosure will be described below; however, it will be understood by those skilled in the art that the protection scope of the present disclosure is not limited thereto, and that various modifications, alterations or changes can be made based on the spirit and conception of the present disclosure, and the content with these modifications, alterations or changes still falls within the scope of the present disclosure.

*Terminology*

As used herein, the term "antigen-binding polypeptide" refers to polypeptides and proteins having at least one antigen-binding domain. Examples of the antigen-binding polypeptide include, but are not limited to, monoclonal antibodies, multispecific antibodies or fusion proteins. As used herein, the term "antigen-binding portion" of an antigen-binding polypeptide refers to one or more fragments of the antigen-binding polypeptide that retain the ability to specifically bind to an antigen (e.g., CD47 protein). It has been shown that the antigen-binding function of antigen-binding polypeptides can be performed by fragments thereof. Examples encompassed by "antigen-binding portion" of an antigen-binding polypeptide include: (i) Fab fragments, monovalent fragments consisting of $V_L$, $V_H$, $C_L$ and $C_{H1}$ domains; (ii) "Fab' fragments", differing from the Fab fragments by having several additional residues, including one or more cysteines from the antibody hinge region, at the carboxy-terminus of the heavy chain $C_{H1}$ domain; (iii) F(ab')$_2$ fragments, bivalent fragments comprising two Fab fragments connected by a hinge region disulfide bridge; (iv) Fd fragments, consisting of $V_H$ and $C_{H1}$ domains; (v) Fv fragments, consisting of the VL and VH domains of an antibody single arm; (vi) dAb fragments (Ward et al., (1989) Nature 341:544-546), consisting of $V_H$ domains; (vii) isolated complementarity determining regions (CDRs); and (viii) nanobodies, comprising a heavy chain variable region of a single variable domain and two constant domains. Furthermore, although the two domains of the Fv fragment, $V_L$ and $V_H$, are encoded by different genes, they can be joined by a synthetic linker using recombinant methods, thereby forming a single protein chain, in which $V_L$ and $V_H$ regions are paired to form a monovalent molecule (known as single-chain Fv (scFv); see, e.g., Bird et al., (1988) Science 242: 423-426, and Huston et al., (1988) Proc. Natl. Acad. USA 85:5879-5883). Such single-chain antibodies are also intended to be encompassed within the term antigen-binding polypeptide. The antigen-binding portion described herein can be obtained using conventional techniques well known to those skilled in the art, and the fragments can be screened for utility in the same manner as intact antigen-binding polypeptides.

The term "isotype" refers to the class of antibodies encoded by the heavy chain constant region gene. The antigen-binding polypeptide that binds to CD47 and the antigen-binding portion thereof of the present disclosure can be derived from any species, including but not limited to, mice, rats, rabbits, non-human primates (e.g., chimpanzees, cynomolgus monkeys, spider monkeys, and macaques), llamas, and humans. The antigen-binding polypeptide that binds to CD47 and the antigen-binding portion thereof can be chimeric, humanized or fully human antibodies. In some embodiments, the antigen-binding polypeptide that binds to CD47 is an antibody produced by a mouse-derived hybridoma cell line. Thus, in some embodiments, the antigen-binding polypeptide that binds to CD47 is a murine antibody. In some other embodiments, the antigen-binding polypeptide that binds to CD47 is a chimeric antibody. In some embodiment, the chimeric antibody is a mouse-human chimeric antibody. In some other embodiments, the antigen-binding polypeptide that binds to CD47 is a humanized antibody. In some other embodiments, the antigen-binding polypeptide that binds to CD47 is derived from a murine antibody and is humanized.

The term "chimeric antibody" refers to an antibody having at least a portion of a heavy chain variable region and a portion of a light chain variable region derived from one species, and at least a portion of a constant region derived from another species. For example, in some embodiments, the chimeric antibody may comprise a murine variable region and a human constant region.

The term "humanized antibody" refers to an antibody comprising complementarity determining regions (CDRs) derived from a non-human antibody, and framework and constant regions derived from a human antibody. For example, the humanized antibody that binds to CD47 provided herein may comprise CDRs derived from one or more murine antibodies as well as human framework and constant regions. Thus, in some embodiments, the humanized antibody provided herein binds to the same epitope on CD47 as the murine antibody from which the CDRs of the humanized antibody are derived. Exemplary humanized antibodies are disclosed herein. Additional humanized antibodies that bind to CD47 or variants thereof comprising the heavy and light chain CDRs provided herein can be generated using any human framework sequences, and are also included in the present disclosure. In some embodiments, framework sequences suitable for use in the present disclosure include those similar in structure to the framework sequences provided herein. Additional modifications may be made in the framework regions to improve the properties of the antibodies provided herein. Such additional framework modifications may include: chemical modifications, point mutations for reducing immunogenicity or removing T cell epitopes, or modifications reverting the mutations to residues in original germline sequences. In some embodiments, such modifications include those corresponding to the mutations exemplified herein, including reversions to germline sequences. For example, in some embodiments, one or more amino acids in the human VH and/or VL framework regions of the humanized antibodies provided herein are reverted to the corresponding amino acids in the parent murine antibodies. For example, for the VH and VL of humanized 3F10, 14A9 and 16E5 antibodies, several sites of framework amino acids of the template human antibodies described above are reverted to the corresponding amino acid sequences in the mouse 3F10, 14A9 and 16E5 antibodies. In some embodiments, the amino acids at position 2 and/or position 33 and/or position 48 and/or position 63 and/or position 68 and/or position 71 and/or position 72 and/or position 94 of the light chain variable region are reverted to the corresponding amino acids found at the positions in mouse 3F10, 14A9 or 16E5 light chain variable region. In some other embodiments, the amino acids at position 24 and/or position 46 and/or position 63 and/or position 72 and/or position 73 and/or position 74 and/or position 77 and/or position 97 of the heavy chain variable region are reverted to the corresponding amino acids found at the positions in mouse 3F10, 14A9 or 16E5 heavy chain variable region. In some embodiments, the humanized 3F10 antibody comprises a heavy chain variable region, whose types of mutation are selected from one or more of the following mutations: position 73 where the amino acid is mutated from Asp (D) to Glu (E), position 74 where the amino acid is mutated from Glu (E) to Ile (I), and position 77 where the amino acid is mutated from Ser (S) to Asn (N); and the humanized 3F10 antibody comprises a light chain variable region, whose types of mutation are selected from one or more of the following mutations: position 48 where the amino acid is mutated from Leu (L) to Trp (W), position 71 where the amino acid is mutated from Asp (D) to Ser (S), position 72 where the amino acid is mutated from Phe (F) to Tyr (Y), and position 94 where the amino acid is mutated from Asp (D) to Glu (E). In some embodiments, the humanized 14A9 antibody comprises a heavy chain variable region, whose types of mutation are selected from one or more of the following mutations: position 24 where the amino acid is mutated from Ala (A) to Val (V), position 72 where the amino acid is mutated from Arg (R) to Gly (G), position 77 where the amino acid is mutated from Ser (S) to Asn (N), and position 97 where the amino acid is mutated from Ala (A) to Thr (T); and the humanized 14A9 antibody comprises a light chain variable region, whose types of mutation are selected from one or more of the following mutations: position 63 where the amino acid is mutated from Ser (S) to Ile (I), and position 68 where the amino acid is mutated from Gly (G) to Ser (S). In some embodiments, the humanized 16E5 antibody comprises a heavy chain variable region, whose types of mutation are selected from one or more of the following mutations: position 46 where the amino acid is mutated from Glu (E) to Lys (K), position 63 where the amino acid is mutated from Lys (K) to Glu (E), position 72 where the amino acid is mutated from Thr (T) to Leu (L), position 77 where the amino acid is mutated from Ser (S) to Arg (R), and position 97 where the amino acid is mutated from Ala (A) to Thr (T); and the humanized 16E5 antibody comprises a light chain variable region, whose types of mutation are selected from one or more of the following mutations: position 2 where the amino acid is mutated from Ile (I) to Ala (A), and position 33 where the amino acid is mutated from Asn (N) to Gln (Q). Unless otherwise indicated, the mutations described herein are numbered by counting from the first amino acid of the variable region in order. Additional or alternative reverse mutations can be made in the framework regions of the humanized antibodies provided herein to improve the properties of the antibodies. The present disclosure also includes humanized antibodies that bind to CD47 and comprise framework modifications corresponding to the exemplary modifications disclosed herein relative to any suitable framework sequence and other framework modifications that otherwise improve antibody properties.

As used herein, the term "derived", when used to refer to a molecule or polypeptide relative to a reference antibody or other binding proteins, means a molecule or polypeptide that can specifically bind to the same epitope as the reference antibody or other binding proteins.

The term "isolated" means that a compound of interest (e.g., an antibody or nucleic acid) has been isolated from its natural environment.

As used herein, the term "$EC_{50}$" refers to the effective concentration, 50% of the maximal response of an antibody. As used herein, the term "$IC_{50}$" refers to the inhibitory concentration, 50% of the maximal response of an antibody. Both $EC_{50}$ and $IC_{50}$ can be measured by ELISA or FACS assay or any other method known in the art.

The term "$K_D$" as used herein refers to the dissociation constant, expressed in molar concentration (M). The $K_D$ value of an antibody can be determined using methods well known in the art. One preferred method of determining antibody $K_D$ is to use surface plasmon resonance, more preferably to use a biosensor system, such as a Biacore system.

The term "treatment" refers to a measure taken to statistically significantly treat, cure, alleviate, relieve, alter, remedy, ameliorate, improve or affect an illness (e.g., a disease) and a symptom of the illness, or prevent or delay the onset of the symptom, a complication and biochemical indicators, or otherwise prevent or inhibit the further progression of the disease, illness or disorder.

As used herein, the term "therapeutically effective amount" refers to the amount of a compound or composition necessary to provide a therapeutic and/or prophylactic benefit to a subject.

As used herein, the term "subject" includes any human or non-human animal. The term "non-human animal" includes all vertebrates, e.g., mammals and non-mammals, such as non-human primates, sheep, dog, cat, horse, cow, chicken, amphibians, and reptiles. Preferably, the subject according to the present disclosure is a human. The terms "patient" and "subject" are used interchangeably unless otherwise indicated.

As used herein, "about" means being within an acceptable error range determined by those of ordinary skill in the art for a particular value, which will depend in part on how the value is measured or determined, i.e., the limitation by the measurement system. For example, "about" may mean being within 1 or more than 1 standard deviation, as practiced in the art. Alternatively, "about" may mean a range of up to $\pm5\%$, for example, fluctuating within a particular numerical range given $\pm 2\%$, $\pm 1\%$ or $\pm 0.5\%$. When a particular value is given in the present disclosure or in the claims, unless otherwise stated, "about" should be considered to mean being within an acceptable error range for that particular value. In this context, unless otherwise indicated, the values of the parameters or conditions in a step are all modified by "about" by default.

The term "identity" is also known as consistency. The percent identity between two sequences is a function of identical positions shared by the sequences (i.e., %identity = number of identical positions/total number of positions $\times$ 100), where the number of gaps that need to be introduced to produce an optimal alignment of the two sequences and the length of each gap should be taken into consideration. As shown in the following non-limiting examples, comparison of sequences and determination of percent identity between two sequences can be accomplished using a mathematical algorithm. The percent identity between two amino acid sequences can be determined using the algorithm of E. Meyers and W. Miller (Comput. Appl. Biosci., 4:11-17 (1988)), which has been incorporated into the ALIGN program (version 2.0) and uses a PAM120 residue weight table with a gap length penalty of 12 and a gap penalty of 4. In addition, the percent identity of two amino acid sequences can be determined using the algorithm of Needleman and Wunsch (J. Mol. Biol. 484-453 (1970)), which has been incorporated into the GAP program in the GCG software package (available at www.gcg.com) and uses the Blossum 62 matrix or the PAM250 matrix with a gap weight of 16, 14, 12, 10, 8, 6 or 4 and a length weight of 1, 2, 3, 4, 5 or 6.

The terms "Xn" and "Xaa" are equivalent and refer to an unspecified amino acid whose scope is specified by the subsequent definitions in the relevant description.

The term "include", "contain" or "comprise" and variations thereof should be understood as "including but not limited to", which means that other elements, components and steps that are not specified are encompassed in addition to the elements, components and steps that are listed.

Herein, singular terms encompass plural referents and vice versa, unless otherwise specified clearly in the context.

*Antigen-Binding Polypeptide That Binds to CD47 and Antigen-Binding Portion Thereof*

Herein, the terms "CD47", "integrin-associated protein", "IAP", "OA3" and "MER6" are used interchangeably. The terms "human CD47" and "hCD47" and the like are used interchangeably herein and refer to human CD47 and variants or isotypes of human CD47. Herein, "antibody that binds to CD47" and "anti-CD47 antibody" are used interchangeably.

**EP 4 130 042 B1**

The present disclosure provides an antigen-binding polypeptide that binds to CD47 or an antigen-binding portion thereof, providing a new solution to CD47-targeted therapy. The antigen-binding polypeptide provided herein also has a number of other desirable features for targeted therapy, in addition to binding to human CD47 or/and cynomolgus monkey CD47. Specifically, these desirable features may be, for example, at least one of the following: binding to CD47 with a $K_D$ value of 1.53E-08 or less; blocking binding of CD47 to SIRP$\alpha$; promoting macrophage-mediated phagocytosis of CD47-expressing cells; not significantly inducing apoptosis of CD4+ T cells; not causing substantial hematocytopenia, anemia or red cell agglutination; and the antigen-binding polypeptide or the antigen-binding portion thereof having a melting temperature T $\geq$ 62 °C and an aggregation temperature Tagg $\geq$ 61 °C. In some embodiments, the antigen-binding polypeptide that binds to CD47 has a better therapeutic effect; in some embodiments, the antigen-binding polypeptide that binds to CD47 has reduced side effects.

In one aspect, the present disclosure provides an antigen-binding polypeptide that binds to CD47 and an antigen-binding portion thereof comprising specific sequences.

In some embodiments, the present disclosure provides an isolated antigen-binding polypeptide that binds to CD47 or an antigen-binding portion thereof comprising one or more CDRs selected from the group consisting of SEQ ID NOs: 1-32.

In some embodiments, the isolated antigen-binding polypeptide that binds to CD47 or the antigen-binding portion thereof comprises a heavy chain CDR1 sequence comprising an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 1, 2, 3, 4 and 5.

In some embodiments, the isolated antigen-binding polypeptide that binds to CD47 or the antigen-binding portion thereof comprises a heavy chain CDR2 comprising an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 6, 7, 8, 9 and 10.

In some embodiments, the isolated antigen-binding polypeptide that binds to CD47 or the antigen-binding portion thereof comprises a heavy chain CDR3 comprising an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 11, 12, 13, 14 and 15.

In some embodiments, the isolated antigen-binding polypeptide that binds to CD47 or the antigen-binding portion thereof comprises a light chain CDR1 comprising an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 16, 17, 18, 19 and 20.

In some embodiments, the isolated antigen-binding polypeptide that binds to CD47 or the antigen-binding portion thereof comprises a light chain CDR2 comprising an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 21, 22, 23, 24 and 25.

In some embodiments, the isolated antigen-binding polypeptide that binds to CD47 or the antigen-binding portion thereof comprises a light chain CDR3 comprising an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 26, 27, 28, 30 and 32.

Those skilled in the art will understand that the heavy chain CDRs and light chain CDRs of the antigen-binding polypeptide provided herein can be independently selected or mixed and matched to form an antigen-binding polypeptide or an antigen-binding portion thereof comprising any heavy chain CDR1, CDR2 and CDR3, and any light chain CDR1, CDR2 and CDR3 of the antigen-binding polypeptide provided herein. Thus, in some embodiments, the present disclosure provides an isolated antigen-binding polypeptide that binds to CD47, which comprises three heavy chain CDRs (a heavy chain CDR1, a heavy chain CDR2, and a heavy chain CDR3) and three light chain CDRs (a light chain CDR1, a light chain CDR2, and a light chain CDR3), wherein:

the heavy chain CDR1 comprises an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 1, 2, 3, 4 and 5; the heavy chain CDR2 comprises an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 6, 7, 8, 9 and 10; the heavy chain CDR3 comprises an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 11, 12, 13, 14 and 15; the light chain CDR1 comprises an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 16, 17, 18, 19 and 20; the light chain CDR2 comprises an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 21, 22, 23, 24 and 25; and the light chain CDR3 comprises an amino acid sequence having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 26, 27, 28, 30 and 32.

In some embodiments, the isolated antigen-binding polypeptide that binds to CD47 comprises the following complementarity determining regions: a heavy chain CDR1 comprising an amino acid sequence selected from SEQ ID NOs: 1, 2, 3, 4 and 5; a heavy chain CDR2 comprising an amino acid sequence selected from SEQ ID NOs: 6, 7, 8, 9 and 10; a heavy chain CDR3 comprising an amino acid sequence selected from SEQ ID NOs: 11, 12, 13, 14 and 15; a light chain CDR1 comprising an amino acid sequence selected from SEQ ID NOs: 16, 17, 18, 19 and 20; a light chain CDR2 comprising an amino acid sequence selected from SEQ ID NOs: 21, 22, 23, 24 and 25; and a light chain CDR3 comprising an amino acid sequence selected from SEQ ID NOs: 26, 27, 28, 30 and 32.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein the heavy chain CDR1, the heavy chain CDR2, and the heavy chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 1, 6 and 11, respectively; the light chain CDR1, the light chain CDR2, and the light chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 16, 21 and 26, respectively. In some embodiments, the isolated antigen-binding polypeptide that binds to CD47 comprises the following complementarity determining regions: a heavy chain CDR1 comprising the amino acid sequence of SEQ ID NO: 1; a heavy chain CDR2 comprising the amino acid sequence of SEQ ID NO: 6; a heavy chain CDR3 comprising the amino acid sequence of SEQ ID NO: 11; a light chain CDR1 comprising the amino acid sequence of SEQ ID NO: 16; a light chain CDR2 comprising the amino acid sequence of SEQ ID NO: 21; and a light chain CDR3 comprising the amino acid sequence of SEQ ID NO: 26.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein

the heavy chain CDR1, the heavy chain CDR2, and the heavy chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 2, 7 and 12, respectively; the light chain CDR1, the light chain CDR2, and the light chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 17, 22 and 27, respectively. In some embodiments, the isolated antigen-binding polypeptide that binds to CD47 comprises the following complementarity determining regions: a heavy chain CDR1 comprising the amino acid sequence of SEQ ID NO: 2; a heavy chain CDR2 comprising the amino acid sequence of SEQ ID NO: 7; a heavy chain CDR3 comprising the amino acid sequence of SEQ ID NO: 12; a light chain CDR1 comprising the amino acid sequence of SEQ ID NO: 17; a light chain CDR2 comprising the amino acid sequence of SEQ ID NO: 22; and a light chain CDR3 comprising the amino acid sequence of SEQ ID NO: 27.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein the heavy chain CDR1, the heavy chain CDR2, and the heavy chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 3, 8 and 13, respectively; the light chain CDR1, the light chain CDR2, and the light chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 18, 23 and 28, respectively. In some embodiments, the isolated antigen-binding polypeptide that binds to CD47 comprises the following complementarity determining regions: a heavy chain CDR1 comprising the amino acid sequence of SEQ ID NO: 3; a heavy chain CDR2 comprising the amino acid sequence of SEQ ID NO: 8; a heavy chain CDR3 comprising the amino acid sequence of SEQ ID NO: 13; a light chain CDR1 comprising the amino acid sequence of SEQ ID NO: 18; a light chain CDR2 comprising the amino acid sequence of SEQ ID NO: 23; and a light chain CDR3 comprising the amino acid sequence of SEQ ID NO: 28.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein the heavy chain CDR1, the heavy chain CDR2, and the heavy chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 4, 9 and 14, respectively; the light chain CDR1, the light chain CDR2, and the light chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 19, 24 and 32, respectively. In some embodiments, the isolated antigen-binding polypeptide that binds to CD47 comprises the following complementarity determining regions: a heavy chain CDR1 comprising the amino acid sequence of SEQ ID NO: 4; a heavy chain CDR2 comprising the amino acid sequence of SEQ ID NO: 9; a heavy chain CDR3 comprising the amino acid sequence of SEQ ID NO: 14; a light chain CDR1 comprising the amino acid sequence of SEQ ID NO: 19; a light chain CDR2 comprising the amino acid sequence of SEQ ID NO: 24; and a light chain CDR3 comprising the amino acid sequence of SEQ ID NO: 32.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein the heavy chain CDR1, the heavy chain CDR2, and the heavy chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 5, 10 and 15, respectively; the light chain CDR1, the light chain CDR2, and the light chain CDR3 comprise amino acid sequences having at least 80% identity, at least 81% identity, at least 82% identity, at least 83% identity, at least 84% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequences set forth in SEQ ID NOs: 20, 25 and 30, respectively. In some embodiments, the isolated antigen-binding polypeptide that binds to CD47 comprises the following complementarity determining regions: a heavy chain CDR1 comprising the amino acid sequence of SEQ ID NO: 5; a heavy chain CDR2 comprising the amino acid sequence of SEQ ID NO: 10; a heavy chain CDR3 comprising the amino acid sequence of SEQ ID NO: 15; a light chain CDR1 comprising the amino acid sequence of SEQ ID NO: 20; a light chain CDR2 comprising the amino acid sequence of SEQ ID NO: 25; and a light chain CDR3 comprising the amino acid sequence of SEQ ID NO: 30.

The present disclosure also provides a conservatively modified form of the antigen-binding polypeptide and the antigen-binding portion thereof. Those skilled in the art will recognize that a conservative amino acid substitution refers to the substitution of one amino acid with another amino acid having a similar structure or similar chemical properties (e.g., a similar side chain). Exemplary conservative substitutions are described in the art, for example, in Watson et al., Molecular Biology of the Gene, the Bengamin/Cummings Publication Company, 4th Edition (1987). In some embodiments, the isolated antigen-binding polypeptide that binds to CD47 comprises: a heavy chain CDR1 comprising an amino acid sequence selected from SEQ ID NOs: 1, 2, 3, 4 and 5 or a conservatively modified form thereof; a heavy chain CDR2 comprising an amino acid sequence selected from SEQ ID NOs: 6, 7, 8, 9 and 10 or a conservatively modified form thereof; a heavy chain CDR3 comprising an amino acid sequence selected from SEQ ID NOs: 11, 12, 13, 14 and 15 or a conservatively modified form thereof; a light chain CDR1 comprising an amino acid sequence selected from SEQ ID NOs: 16, 17, 18, 19 and 20 or a conservatively modified form thereof; a light chain CDR2 comprising an amino acid sequence selected from SEQ ID NOs: 21, 22, 23, 24 and 25 or a conservatively modified form thereof; and a light chain CDR3 comprising an amino acid sequence selected from SEQ ID NOs: 26, 27, 28, 30 and 32 or a conservatively modified form thereof. Conservative modifications exist in any one or more of the light chain CDRs or the heavy chain CDRs.

In some embodiments, the isolated antigen-binding polypeptide that binds to CD47 comprises: a heavy chain CDR1 comprising an amino acid sequence selected from SEQ ID NOs: 3, 4 and 5; a heavy chain CDR2 comprising an amino acid sequence selected from SEQ ID NOs: 8, 9 and 10; a heavy chain CDR3 comprising an amino acid sequence selected from SEQ ID NOs: 13, 14 and 15; a light chain CDR1 comprising an amino acid sequence selected from SEQ ID NOs: 18, 19 and 20; a light chain CDR2 comprising an amino acid sequence selected from SEQ ID NOs: 23, 24 and 25; and a light chain CDR3 comprising an amino acid sequence selected from SEQ ID NOs: 28, 30 and 32.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein the heavy chain CDR1 comprises the amino acid sequence of SEQ ID NO: 1 or a conservatively modified form thereof; the heavy chain CDR2 comprises the amino acid sequence of SEQ ID NO: 6 or a conservatively modified form thereof; the heavy chain CDR3 comprises the amino acid sequence of SEQ ID NO: 11 or a conservatively modified form thereof; the light chain CDR1 comprises the amino acid sequence of SEQ ID NO: 16 or a conservatively modified form thereof; the light chain CDR2 comprising the amino acid sequence of SEQ ID NO: 21 or a conservatively modified form thereof; and the light chain CDR3 comprises the amino acid sequence of SEQ ID NO: 26 or a conservatively modified form thereof.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein the heavy chain CDR1 comprises the amino acid sequence of SEQ ID NO: 2 or a conservatively modified form thereof; the heavy chain CDR2 comprises the amino acid sequence of SEQ ID NO: 7 or a conservatively modified form thereof; the heavy chain CDR3 comprises the amino acid sequence of SEQ ID NO: 12 or a conservatively modified form thereof; the light chain CDR1 comprises the amino acid sequence of SEQ ID NO: 17 or a conservatively modified form thereof; the light chain CDR2 comprising the amino acid sequence of SEQ ID NO: 22 or a conservatively modified form thereof; and the light chain CDR3 comprises the amino acid sequence of SEQ ID NO: 27 or a conservatively modified form thereof.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein the heavy chain CDR1 comprises the amino acid sequence of SEQ ID NO: 3 or a conservatively modified form thereof; the heavy chain CDR2 comprises the amino acid sequence of SEQ ID NO: 8 or a conservatively modified form thereof; the heavy chain CDR3 comprises the amino acid sequence of SEQ ID NO: 13 or a conservatively modified form thereof; the light chain CDR1 comprises the amino acid sequence of SEQ ID NO: 18 or a conservatively modified form thereof; the light chain CDR2 comprising the amino acid sequence of SEQ ID NO: 23 or a conservatively modified form thereof; and the light chain CDR3 comprises the amino acid sequence of SEQ ID NO: 28 or a conservatively modified form thereof.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein

the heavy chain CDR1 comprises the amino acid sequence of SEQ ID NO: 4 or a conservatively modified form thereof; the heavy chain CDR2 comprises the amino acid sequence of SEQ ID NO: 9 or a conservatively modified form thereof; the heavy chain CDR3 comprises the amino acid sequence of SEQ ID NO: 14 or a conservatively modified form thereof; the light chain CDR1 comprises the amino acid sequence of SEQ ID NO: 19 or a conservatively modified form thereof; the light chain CDR2 comprising the amino acid sequence of SEQ ID NO: 24 or a conservatively modified form thereof; and the light chain CDR3 comprises the amino acid sequence of SEQ ID NO: 32 or a conservatively modified form thereof.

In some embodiments, the isolated antigen-binding polypeptide that binds to CD47 comprises a heavy chain CDR1, a heavy chain CDR2, a heavy chain CDR3, a light chain CDR1, a light chain CDR2, and a light chain CDR3, wherein the heavy chain CDR1 comprises the amino acid sequence of SEQ ID NO: 5 or a conservatively modified form thereof; the heavy chain CDR2 comprises the amino acid sequence of SEQ ID NO: 10 or a conservatively modified form thereof; the heavy chain CDR3 comprises the amino acid sequence of SEQ ID NO: 15 or a conservatively modified form thereof; the light chain CDR1 comprises the amino acid sequence of SEQ ID NO: 20 or a conservatively modified form thereof; the light chain CDR2 comprising the amino acid sequence of SEQ ID NO: 25 or a conservatively modified form thereof; and the light chain CDR3 comprises the amino acid sequence of SEQ ID NO: 30 or a conservatively modified form thereof.

In some embodiments, the isolated antigen-binding polypeptide that binds to CD47 provided herein comprises a heavy chain variable region and a light chain variable region; the heavy and light chain variable regions comprise CDR regions and framework regions FRs (including FR1, FR2, FR3 and FR4), which form a variable region sequence in the order FR1-CDR1-FR2-CDR2-FR3-CDR3-FR4.

Thus, in another aspect, the present disclosure provides an isolated antigen-binding polypeptide that binds to CD47 or an antigen-binding portion thereof, the antigen-binding polypeptide comprising a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 33, 35, 37, 39, 41, 83, 85 and 87; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to an amino acid sequence selected from SEQ ID NOs: 34, 36, 38, 40, 42, 84, 86 and 88.

wherein the sequence set forth in SEQ ID NO: 83 is:
QVQLVQSGAEVKKPGSSVKVSCKAS<u>GYTFSRYWIE</u>WVRQAPGQGLEWMG<u>EFIPGSDTT</u>NYAQKFQGRV TITA $X_3X_4$ST$X_5$TAYMELSSLRSEDTAVYYCAR<u>GGLRRMDY</u>WGQGTLVTVSS, wherein $X_3$ is D or E, $X_4$ is I or E, and $X_5$ is S or N;

the sequence set forth in SEQ ID NO: 84 is:
DIQMTQSPSSLSASVGDRVTITC<u>RASSSVSSTYLH</u>WYQQKPGKAPKL$X_6$<u>IYTTSTLASG</u>VPSRFSGSGSGT$X_7$$X_8$TLTISSLQPEDFATYYC<u>QQFS$X_2$STWT</u>$_6$ is L or W, $X_7$ is D or S, $X_8$ is F or Y, and $X_2$ is D or E;

the sequence set forth in SEQ ID NO: 85 is:
QVQLVQSGAEVKKPGASVKVSCKAS<u>GYTFTNYGMN</u>WVRQAPGQGL$X_9$WMG<u>WINTNTGEPTYAQX$_{10}$L</u> QGRVT MT$X_{11}$DTST$X_{12}$TAYMELRSLRSDDTAVYYC$X_{13}$R<u>FSHLRGPMDY</u>WGQGTLVTVSS, wherein $X_9$ is E or K, $X_{10}$ is K or E, $X_{11}$ is L or T, $X_{12}$ is R or S, and $X_{13}$ is A or T;

the sequence set forth in SEQ ID NO: 86 is:
D$X_{14}$QMTQSPSSLSASVGDRVTITC<u>RSSQSLVHSNGYTYLH</u>WYQQKPGKAPKLLIY<u>KVSNRFS</u>GVPSRFSG SGSGTDFTLTISSLQPEDFATYYC<u>SQSTHVPPT</u>FGQGTKLEIK, wherein $X_{14}$ is I or A;

the sequence set forth in SEQ ID NO: 87 is:
QVQLVQSGAEVKKPGASVKVSCK$X_{15}$<u>SGFNIEDDYIE</u>WVRQAPGQGLEWMG<u>RIDPANDKTK</u>YAQKFQG RVTMT $X_{16}$DTST$X_{17}$TVYMELSSLRSEDTAVYYC$X_{18}$<u>RPGLRRYYSMDY</u>WGQGTLVTVSS, wherein $X_{15}$ is A or V, $X_{16}$ is R or G, $X_{17}$ is S or N, and $X_{18}$ is A or T;

the sequence set forth in SEQ ID NO: 88 is:
DIQMTQSPSSLSASVGDRVTITC<u>KASENVVS</u>YVSWYQQKPGKAPKLLIY<u>GASNRYT</u>GVPSRF$X_{19}$GSGS$X_{20}$ TDFTLTISSLQPEDFATYYC<u>GQSYSYPLT</u>FGQGTKLEIK, wherein $X_{19}$ is S or I, and $X_{20}$ is S or G.

In some embodiments, the isolated antigen-binding polypeptide that binds to CD47 comprises a heavy chain variable region and a light chain variable region, wherein the heavy chain variable region comprises or consists of the following sequence: an amino acid sequence selected from SEQ ID NOs: 33, 35, 37, 39, 41, 83, 85 and 87; and the light chain variable region comprises or consists of the following sequence: an amino acid sequence selected from SEQ ID NOs: 34, 36, 38, 40, 42, 84, 86 and 88.

In some embodiments, the isolated antigen-binding polypeptide that binds to CD47 comprises a heavy chain variable region and a light chain variable region, wherein the heavy chain variable region comprises or consists of the following sequence: an amino acid sequence selected from SEQ ID NOs: 35, 39, 41, 83, 85 and 87; and the light chain variable region comprises or consists of the following sequence: an amino acid sequence selected from SEQ ID NOs: 36, 40, 42, 84, 86 and 88. In some embodiments, the isolated antigen-binding polypeptide that binds to CD47 comprises a heavy chain variable region and a light chain variable region, wherein the heavy chain variable region comprises or consists of the amino acid sequence of SEQ ID NO: 79; and the light chain variable region comprises or consists of the amino acid sequence of SEQ ID NO: 80. In some embodiments, the isolated antigen-binding polypeptide that binds to CD47 comprises a heavy chain variable region and a light chain variable region, wherein the heavy chain variable region comprises or consists of the amino acid sequence of SEQ ID NO: 81; and the light chain variable region comprises or consists of the amino acid sequence of SEQ ID NO: 82.

In some embodiments, the isolated antigen-binding polypeptide that binds to CD47 comprises a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 33; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 34. In some embodiments, the isolated antigen-binding polypeptide that binds to CD47 comprises a heavy chain variable region and a light chain variable region, wherein the heavy chain variable region comprises the amino acid sequence of SEQ ID NO: 33, and the light chain variable region comprises the amino acid sequence of SEQ ID NO: 34.

In some embodiments, the isolated antigen-binding polypeptide that binds to CD47 comprises a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 35; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 36. In some embodiments, the isolated antigen-binding polypeptide that binds to CD47 comprises a heavy chain variable region and a light chain variable region, wherein the heavy chain variable region comprises the amino acid sequence of SEQ ID NO: 35, and the light chain variable region comprises the amino acid sequence of SEQ ID NO: 36.

In some embodiments, the isolated antigen-binding polypeptide that binds to CD47 comprises a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 37; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 38. In some embodiments, the isolated antigen-binding polypeptide that binds to CD47 provided herein comprises a heavy chain variable region and a light chain variable region, wherein the heavy chain variable region comprises the amino acid sequence of SEQ ID NO: 37, and the light chain variable region comprises the amino acid sequence of SEQ ID NO: 38.

In some embodiments, the isolated antigen-binding polypeptide that binds to CD47 comprises a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 39; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 40. In some embodiments, the isolated antigen-binding polypeptide that binds to CD47 provided herein comprises a heavy chain variable region and a light chain variable region, wherein the heavy chain variable region comprises the amino acid sequence of SEQ ID NO: 39, and the light chain variable region comprises the amino acid sequence of SEQ ID NO: 40.

In some embodiments, the isolated antigen-binding polypeptide that binds to CD47 comprises a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 41; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 42. In some embodiments, in the isolated antigen-binding polypeptide that binds to CD47 provided herein, the heavy chain variable region comprises the amino acid sequence of SEQ ID NO: 41, and the light chain variable region comprises the amino acid sequence of SEQ ID NO: 42.

In some embodiments, the isolated antigen-binding polypeptide that binds to CD47 comprises a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 83; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 84. In some embodiments, the isolated antigen-binding polypeptide that binds to CD47 comprises a heavy chain variable region and a light chain variable region, wherein the heavy chain variable region comprises the amino acid sequence of SEQ ID NO: 83, and the light chain variable region comprises the amino acid sequence of SEQ ID NO: 84.

In some embodiments, the isolated antigen-binding polypeptide that binds to CD47 comprises a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 85; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 86. In some embodiments, the isolated antigen-binding polypeptide that binds to CD47 comprises a heavy chain variable region and a light chain variable region, wherein the heavy chain variable region comprises the amino acid sequence of SEQ ID NO: 85, and the light chain variable region comprises the amino acid sequence of SEQ ID NO: 86.

In some embodiments, the isolated antigen-binding polypeptide that binds to CD47 comprises a heavy chain variable region and a light chain variable region, wherein the heavy chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 87; and the light chain variable region comprises an amino acid sequence having at least 80% identity, at least 85% identity, at least 86% identity, at least 87% identity, at least 88% identity, at least 89% identity, at least 90% identity, at least 91% identity, at least 92% identity, at least 93% identity, at least 94% identity, at least 95% identity, at least 96% identity, at least 97% identity, at least 98% identity, at least 99% identity or 100% identity to the amino acid sequence of SEQ ID NO: 88. In some embodiments, the isolated antigen-binding polypeptide that binds to CD47 comprises a heavy chain variable region and a light chain variable region, wherein the heavy chain variable region comprises the amino acid sequence of SEQ ID NO: 87, and the light chain variable region comprises the amino acid sequence of SEQ ID NO: 88.

In some embodiments, the heavy chain variable region and the light chain variable region described herein comprise the sequence characteristics of the heavy chain CDR1, the heavy chain CDR2, the heavy chain CDR3, the light chain CDR1, the light chain CDR2 and the light chain CDR3 described herein.

In some embodiments, the isolated antigen-binding polypeptide that binds to CD47 provided herein comprises a heavy chain and a light chain that comprise a constant region in addition to the heavy chain variable region and the light chain variable region described herein. In some embodiments, the constant region is humanized. In some embodiments, the framework regions FRs of both the constant region and the variable regions are humanized. Immunogenicity is reduced by constructing chimeric antibodies (e.g., humanized constant regions and non-human variable regions) or humanized antibodies (e.g., humanized constant regions and FRs).

In some embodiments, the light chain constant region of the antigen-binding polypeptide is a human κ chain constant region. In some embodiments, the light chain constant region of the antigen-binding polypeptide is a human λ chain constant region.

The heavy chain constant region of the antigen-binding polypeptide may be from any type of constant region, e.g., IgG, IgM, IgD, IgA and IgE, and from any subclass, e.g., IgG1, IgG2, IgG3 and IgG4. In some embodiments, the isolated antigen-binding polypeptide that binds to CD47 is an IgG1 subclass. In some embodiments, the isolated antigen-binding polypeptide that binds to CD47 is an IgG4 subclass.

In some embodiments, the antigen-binding polypeptide comprises a modified constant region. In some embodiments, the hinge region within the human IgG4 constant region is modified to avoid or reduce chain swapping; for example, an IgG4 antibody has a Ser228Pro (S228P) mutation according to the EU numbering index. In some embodiments, the isolated antigen-binding polypeptide that binds to CD47 is a chimeric antibody comprising a heavy chain variable region and a light chain variable region, wherein the heavy chain variable region comprises or consists of the following sequence: an amino acid sequence selected from SEQ ID NOs: 33, 35, 37, 39 and 41; and the light chain variable region comprises or consists of the following sequence: an amino acid sequence selected from SEQ ID NOs: 34, 36, 38, 40 and 42.

In some embodiments, the isolated antigen-binding polypeptide that binds to CD47 is a chimeric antibody comprising a heavy chain and a light chain, wherein the heavy chain has an amino acid sequence having at least 80% identity, at least 85% identity, at least 90% identity, at least 92% identity, at least 93% identity, at least 94% identity, at least 95% identity, at least 96% identity, at least 97% identity, at least 98% identity, at least 99% identity or 100% identity to an amino acid selected from SEQ ID NOs: 89, 93, 97, 101 and 105; and the light chain has an amino acid sequence having at least 80% identity, at least 85% identity, at least 90% identity, at least 92% identity, at least 93% identity, at least 94% identity, at least 95% identity, at least 96% identity, at least 97% identity, at least 98% identity, at least 99% identity or 100% identity to an amino acid selected from SEQ ID NOs: 91, 95, 99, 103 and 107. In one embodiment, the isolated antigen-binding polypeptide that binds to CD47 is a chimeric antibody comprising a heavy chain according to SEQ ID NO: 89 and a light chain according to SEQ ID NO: 91. In one embodiment, the isolated antigen-binding polypeptide that binds to CD47 is a chimeric antibody comprising a heavy chain according to SEQ ID NO: 93 and a light chain according to SEQ ID NO: 95. In one embodiment, the isolated antigen-binding polypeptide that binds to CD47 is a chimeric antibody comprising a heavy chain according to SEQ ID NO: 97 and a light chain according to SEQ ID NO: 99. In one embodiment, the isolated antigen-binding polypeptide that binds to CD47 is a chimeric antibody comprising a heavy chain according to SEQ ID NO: 101 and a light chain according to SEQ ID NO: 103. In one embodiment, the isolated antigen-binding polypeptide that binds to CD47 is a chimeric antibody comprising a heavy chain according to SEQ ID NO: 105 and a light chain according to SEQ ID NO: 107.

In some embodiments, the isolated antigen-binding polypeptide that binds to CD47 is a humanized antibody comprising a heavy chain variable region and a light chain variable region, wherein the heavy chain variable region comprises or consists of the following sequence: an amino acid sequence selected from SEQ ID NOs: 83, 85 and 87; and the light chain variable region comprises or consists of the following sequence: an amino acid sequence selected from SEQ ID NOs: 84, 86 and 88.

In some embodiments, the isolated antigen-binding polypeptide that binds to CD47 is a humanized antibody, wherein the heavy chain has an amino acid sequence having at least 80% identity, at least 85% identity, at least 90% identity, at least 92% identity, at least 93% identity, at least 94% identity, at least 95% identity, at least 96% identity, at least 97% identity, at least 98% identity, at least 99% identity or 100% identity to an amino acid selected from SEQ ID NOs: 109, 113, 117, 121, 125, 129, 133, 137, 141, 145, 149, 153, 157, 161, 165, 169, 173, 177, 181 and 185; and the light chain has an amino acid sequence having at least 80% identity, at least 85% identity, at least 90% identity, at least 92% identity, at least 93% identity, at least 94% identity, at least 95% identity, at least 96% identity, at least 97% identity, at least 98% identity, at least 99% identity or 100% identity to an amino acid selected from SEQ ID NOs: 111, 115, 119, 123, 127, 131, 135, 139, 143, 147, 151, 155, 159, 163, 167, 171, 175, 179, 183 and 187. In one embodiment, the isolated antigen-binding polypeptide that binds to CD47 is a humanized antibody comprising a heavy chain according to SEQ ID NO: 109 and a light chain according to SEQ ID NO: 111. In one embodiment, the isolated antigen-binding polypeptide that binds to CD47 is a humanized antibody comprising a heavy chain according to SEQ ID NO: 113 and a light chain according to SEQ ID NO: 115. In one embodiment, the isolated antigen-binding polypeptide that binds to CD47 is a humanized antibody comprising a heavy chain according to SEQ ID NO: 117 and a light chain according to SEQ ID NO: 119. In one embodiment, the isolated antigen-binding polypeptide that binds to CD47 is a humanized antibody comprising a heavy chain according to SEQ ID NO: 121 and a light chain according to SEQ ID NO: 123. In one embodiment, the isolated antigen-binding polypeptide that binds to CD47 is a humanized antibody comprising a heavy chain according to SEQ ID NO: 125 and a light chain according to SEQ ID NO: 127. In one embodiment, the isolated antigen-binding polypeptide that binds to CD47 is a humanized antibody comprising a heavy chain according to SEQ ID NO: 129 and a light chain according to SEQ ID NO: 131. In one embodiment, the isolated antigen-binding polypeptide that binds to CD47 is a humanized antibody comprising a heavy chain according to SEQ ID NO: 133 and a light chain according to SEQ ID NO: 135. In one embodiment, the isolated antigen-binding polypeptide that binds to CD47 is a humanized antibody comprising a heavy chain according to SEQ ID NO: 137 and a light chain according to SEQ ID NO: 139. In one embodiment, the isolated antigen-binding polypeptide that binds to CD47 is a humanized antibody comprising a heavy chain according to SEQ ID NO: 141 and a light chain according to SEQ ID NO: 143. In one embodiment, the isolated antigen-binding polypeptide that binds to CD47 is a humanized antibody comprising a heavy chain according to SEQ ID NO: 145 and a light chain according to SEQ ID NO: 147. In one embodiment, the isolated antigen-binding polypeptide that binds to CD47 is a humanized antibody comprising a heavy chain according to SEQ ID NO: 149 and a light chain according to SEQ ID NO: 151. In one embodiment, the isolated antigen-binding polypeptide that binds to CD47 is a humanized antibody comprising a heavy chain according to SEQ ID NO: 153 and a light chain according to SEQ ID NO: 155. In one embodiment, the isolated antigen-binding polypeptide that binds to CD47 is a humanized antibody comprising a heavy chain according to SEQ ID NO: 157 and a light chain according to SEQ ID NO: 159. In one embodiment, the isolated antigen-binding polypeptide that binds to CD47 is a humanized antibody comprising a heavy chain according to SEQ ID NO: 161 and a light chain according to SEQ ID NO: 163. In one embodiment, the isolated antigen-binding polypeptide that binds to CD47 is a humanized antibody comprising a heavy chain according to SEQ ID NO: 165 and a light chain according to SEQ ID NO: 167. In one embodiment, the isolated antigen-binding polypeptide that binds to CD47 is a humanized antibody comprising a heavy chain according to SEQ ID NO: 169 and a light chain according to SEQ ID NO: 171. In one embodiment, the isolated antigen-binding polypeptide that binds to CD47 is a humanized antibody comprising a heavy chain according to SEQ ID NO: 173 and a light chain according to SEQ ID NO: 175. In one embodiment, the isolated antigen-binding polypeptide that binds to CD47 is a humanized antibody comprising a heavy chain according to SEQ ID NO: 177 and a light chain according to SEQ ID NO: 179. In one embodiment, the isolated antigen-binding polypeptide that binds to CD47 is a humanized antibody comprising a heavy chain according to SEQ ID NO: 181 and a light chain according to SEQ ID NO: 183. In one embodiment, the isolated antigen-binding polypeptide that binds to CD47 is a humanized antibody comprising a heavy chain according to SEQ ID NO: 185 and a light chain according to SEQ ID NO: 187.

In one embodiments, the isolated antigen-binding polypeptide that binds to CD47 comprises a heavy chain and a light chain, wherein the heavy chain comprises or consists of the following sequence: an amino acid sequence selected from SEQ ID NOs: 93, 101, 105, 109, 113, 117, 121, 125, 129, 133, 137, 141, 145, 149, 153, 157, 161, 165, 169, 173, 177, 181 and 185; and the light chain comprises or consists of the following sequence: an amino acid sequence selected from SEQ ID NOs: 95, 103, 107, 111, 115, 119, 123, 127, 131, 135, 139, 143, 147, 151, 155, 159, 163, 167, 171, 175, 179, 183 and 187.

In some embodiments, the isolated antigen-binding polypeptide that binds to CD47 or/and the antigen-binding portion thereof bind to CD47 with a $K_D$ value of 1.53E-08 M or less. In some embodiments, the antigen-binding polypeptide or/and the antigen-binding portion thereof bind to human CD47. In some other embodiments, the antigen-binding polypeptide or/and the antigen-binding portion thereof bind to cynomolgus monkey CD47. In some other embodiments, the antigen-binding polypeptide or/and the antigen-binding portion thereof bind to human CD47 and cynomolgus monkey CD47. In some embodiments, the antigen-binding polypeptide or/and the antigen-binding portion thereof have binding affinities ($K_D$) in the range of about 1E-12 M to about 1E-05 M, about 1E-12 M to about 1.89E-06 M, about 1E-12 M to about 1.89E-07 M, about 1E-11 M to about 1.89E-07 M, about 9.46E-10 M to about 1.89E-07 M, about 9.46E-10 M to about 1.89E-08 M, about 2.04E-10 M to about 1.53E-07 M or about 2.04E-10 M to about 1.89E-08 M for human CD47. In some embodiments, the antigen-binding polypeptide or/and the antigen-binding portion thereof have binding affinities ($K_D$) of about 1E-05 M or less, about 1.89E-06 M or less, about 1.89E-07 M or less, about 1.89E-08 M or less, about 1.53E-08 M or less, about 9.31E-08 M or less, about 9.31E-09 M or less, about 8.55E-09 M or less, about 2.35E-09 M or less or about 2.04E-010 M or less for human CD47. In some embodiments, the antigen-binding polypeptide or/and the antigen-binding portion thereof have binding affinities ($K_D$) of about 7.48E-09 M, about 3.50E-10 M, about 7.67E-10 M, about 9.06E-09 M, about 2.35E-09 M, about 9.46E-10 M, about 8.36E-10 M, about 1.53E-09 M, about 1.42E-09 M, about 9.60E-10 M, about 5.92E-10 M, about 9.04E-10 M, about 7.79E-10 M, about 1.15E-09 M, about 1.34E-09 M, about 8.96E-10 M, about 7.65E-10 M, about 1.89E-08 M, about 6.28E-09 M, about 9.31E-09 M, about 1.71E-08 M, about 1.53E-08 M, about 7.13E-09 M, about 2.04E-10 M or about 8.55E-09 M for human CD47. In some embodiments, the binding affinity $k_D$ of the antigen-binding polypeptide that binds to CD47 and the antigen-binding portion thereof provided herein is measured by Biacore.

In some embodiments, the antigen-binding polypeptide that binds to CD47 or/and the antigen-binding portion thereof have binding $EC_{50}$ of about 1 ng/mL to about 2000 ng/mL, about 1 ng/mL to about 1500 ng/mL, about 1 ng/mL to about 1106 ng/mL, about 1 ng/mL to about 699 ng/mL, about 1 ng/mL to about 340 ng/mL, about 30 ng/mL to about 340 ng/mL, about 50 ng/mL to about 340 ng/mL or about 100 ng/mL to about 340 ng/mL for human CD47. In some embodiments, the antigen-binding polypeptide that binds to CD47 or/and the antigen-binding portion thereof have binding $EC_{50}$ of about 2000 ng/mL or less, about 1500 ng/mL or less, about 1106 ng/mL or less, about 699 ng/mL or less or about 340 ng/mL or less for human CD47. In some embodiments, the $EC_{50}$ of the antigen-binding polypeptide that binds to CD47 and the antigen-binding portion thereof provided herein is measured by ELISA or FACS.

In some embodiments, the antigen-binding polypeptide that binds to CD47 or/and the antigen-binding portion thereof block the binding of CD47 to SIRP$\alpha$. Signal-regulatory protein $\alpha$ (SIRP$\alpha$) is one of the ligands that bind to CD47, and is expressed on hematopoietic cells including macrophages and dendritic cells. CD47 interacts with SIRP$\alpha$ on macrophages, sending a "don't eat me" signal to the macrophages. In the case of treatment, using an antigen-binding polypeptide that binds to CD47 or an antigen-binding portion thereof to block the interaction of SIRP$\alpha$ with CD47 can facilitate the uptake and clearing of cancer cells by the host's immune system. Therefore, the ability to block the interaction of CD47 with IRP$\alpha$ and thus to enable macrophages to phagocytose CD47-expressing tumor cells is an important functional feature of some antigen-binding polypeptides that bind to CD47. In some embodiments, the antigen-binding polypeptide (e.g., chimeric antibodies Xi2B2, Xi2H8, Xi3F10, Xi16E5, Xi14A9) or/and the antigen-binding portion thereof have blocking $IC_{50}$ of about 358.5 ng/mL to about 26,966 ng/mL, about 358.5 ng/mL to about 4861 ng/mL, about 358.5 ng/mL to about 631.2 ng/mL or about 358.5 ng/mL to about 1283 ng/mL, based on Jurkat cells and flow cytometry assay. In some embodiments, the antigen-binding polypeptide or/and the antigen-binding portion thereof have blocking $IC_{50}$ of about 26,966 ng/mL or less, about 15,000 ng/mL or less, about 10,000 ng/mL or less, about 8000 ng/mL or less, about 6000 ng/mL or less, about 4861 ng/mL or less, about 3000 ng/mL or less, about 2500 ng/mL or less, about 2000 ng/mL or less, about 1500 ng/mL or less, about 1283 ng/mL or less, about 1000 ng/mL or less, about 800 ng/mL or less, about 500 ng/mL or less or about 358.5 ng/mL or less.

In some embodiments, based on ELISA analysis, the antigen-binding polypeptide that binds to CD47 (e.g., chimeric antibodies Xi2B2, Xi2H8 and Xi3F10 or humanized antibodies thereof, Xi16E5 or a humanized antibody thereof, and Xi14A9 or a humanized antibody thereof) or/and the antigen-binding portion thereof have blocking $IC_{50}$ of about 4.682 ng/mL to about 15,246 ng/mL, about 621.1 ng/mL to about 15,246 ng/mL, about 621.1 ng/mL to about 7910 ng/mL, about 621.1 ng/mL to about 2939 ng/mL, about 621.1 ng/mL to about 2500 ng/mL, about 621.1 ng/mL to about 2051 ng/mL, about 621.1 ng/mL to about 1738 ng/mL, about 621.1 ng/mL to about 1500 ng/mL, about 621.1 ng/mL to about 1400 ng/mL, about 621.1 ng/mL to about 1300 ng/mL, about 621.1 ng/mL to about 1200 ng/mL, about 621.1 ng/mL to about 1100 ng/mL, about 621.1 ng/mL to about 1000 ng/mL, about 621.1 ng/mL to about 900 ng/mL, about 621.1 ng/mL to about 800 ng/mL, or about 621.1 ng/mL to about 703.2 ng/mL. In some embodiments, based on ELISA analysis, the antigen-binding polypeptide or/and the antigen-binding portion thereof have blocking $IC_{50}$ of about 15,246 ng/mL or less, about 7910 ng/mL or less, about 2939 ng/mL or less, about 2500 ng/mL or less, about 2051 ng/mL or less, about 1738 ng/mL or less, about 1500 ng/mL or less, about 1400 ng/mL or less, about 1300 ng/mL or less, about 1200 ng/mL or less, about 1100 ng/mL or less, about 1000 ng/mL or less, about 900 ng/mL or less, about 800 ng/mL or less, about 703.2 ng/mL or less, about 621.1 ng/mL or less, about 450 ng/mL or less, about 350 ng/mL or less, about 250 ng/mL or less, about 150 ng/mL or less, about 100 ng/mL or less, about 50 ng/mL or less, about 40 ng/mL or less, about 30 ng/mL or less, about 20 ng/mL or less, about 10 ng/mL or less, or about 4.682 ng/mL or less.

In some embodiments, the antigen-binding polypeptide that binds to CD47 or/and the antigen-binding portion thereof promote macrophage-mediated phagocytosis of CD47-expressing cells. As previously described, the phagocytosis of CD47-expressing cells such as cancer cells (specific examples are HL60 cells and the like) by macrophages is increased by blocking the binding of SIRP$\alpha$ to CD47 with an antigen-binding polypeptide that binds to CD47. In some embodiments, under an antigen-binding polypeptide that binds to CD47 at a concentration of about 1 $\mu$g/mL to about 10 $\mu$g/mL, a macrophage phagocytosis rate is about 3.5% to about 65.7%, about 11.5% to about 65.7%, about 28.8% to about 65.7%, or about 44.67% to about 65.7%. In some embodiments, under an antigen-binding polypeptide at a concentration of about 1 $\mu$g/mL to about 10 $\mu$g/mL, the macrophage phagocytosis rate is about 1.34% to about 81.66%, about 2% to about 81.66%, about 11.95% to about 81.66%, about 24.19% to about 81.66%, or about 71.48% to about 81.66%.

In some embodiments, the antigen-binding polypeptide that binds to CD47 or/and the antigen-binding portion thereof do not significantly induce apoptosis of CD4+ T cells. In some embodiments, an apoptosis assay was performed by flow cytometry. The significant apoptosis herein means that there is no statistically significant difference ($P \leq 0.05$) from a negative control within an acceptable range (e.g., having no effect on a subject or within a controllable range); for example, in some embodiments, there is no significant difference from a negative control IgG.

In some embodiments, the antigen-binding polypeptide that binds to CD47 or/and the antigen-binding portion thereof have a Tm (melting temperature) of about 62 °C or higher, about 63 °C or higher, about 64 °C or higher, about 65 °C or higher, about 66 °C or higher, about 67 °C or higher, about 68 °C or higher, about 69 °C or higher, or about 70 °C or higher. In some embodiments, the antibody or/and the fragment thereof have a Tagg (aggregation temperature) of about 61 °C or higher, about 62 °C or higher, about 63 °C or higher, about 64 °C or higher, about 65 °C or higher, about 66 °C or higher, about 67 °C or higher, about 68 °C or higher, about 69 °C or higher, about 70 °C or higher, about 71 °C or higher, about 72 °C or higher, or about 73 °C or higher. In some embodiments, the antigen-binding polypeptide or/and the antigen-binding portion thereof have a Tm of about 62 °C to about 75 °C, about 63 °C to about 70.6 °C, about 64 °C to about 70.6 °C, about 65 °C to about 70.6 °C, about 67 °C to about 70.6 °C, or about 68 °C to about 70.6 °C. In some embodiments, the antibody or/and the fragment thereof have a Tagg of about 60 °C to about 73.81 °C, about 61 °C to about 73.81 °C, about 62 °C to about 73.81 °C, about 63 °C to about 73.81 °C, about 64 °C to about 73.81 °C, about 65 °C to about 73.81 °C, about 67 °C to about 73.81 °C, or about 68 °C to about 73.81 °C. In some embodiments, Tm and Tagg are determined by nano DSF.

In some preferred embodiments, the antigen-binding polypeptide that binds to CD47 or/and the antigen-binding portion thereof do not cause substantial hematocytopenia, anemia or red cell agglutination. The terms "red blood cell" and "red cell" are synonymous and are used interchangeably. The term "agglutination" means clumping of cells. Since red cells also express CD47, certain antigen-binding polypeptides that bind to CD47 bind to red cells, thereby causing agglutination. Thus, avoiding or reducing the deleterious effects of antigen-binding polypeptides that bind to CD47 on red cells is expected to reduce side effects in treatment.

Thus, in another aspect, the present disclosure provides an antigen-binding polypeptide that binds to CD47 and an antigen-binding portion thereof having one of, or a combination of several of, the following properties:

(a) binding to CD47 with a $K_D$ value of 1.89E-08 or less (e.g., 1.53E-08);
(b) blocking binding of CD47 to SIRP$\alpha$;
(c) promoting macrophage-mediated phagocytosis of CD47-expressing cells;
(d) not significantly inducing apoptosis of CD4+ T cells;
(e) not causing substantial hematocytopenia, anemia or red cell agglutination; and
(f) the antigen-binding polypeptide or the antigen-binding portion thereof having a melting temperature $T \geq 62$ °C and an aggregation temperature Tagg $\geq 61$ °C.

In some embodiments, the antigen-binding polypeptide that binds to CD47 or/and the antigen-binding portion thereof have one, two, three, four, five or six of the properties (a)-(f). In some embodiments, the antigen-binding polypeptide or the antigen-binding portion thereof has the properties (a), (b) and (c). In some embodiments, the antigen-binding polypeptide or the antigen-binding portion thereof has the properties (a), (b) and (e). In some embodiments, the antigen-binding polypeptide or the antigen-binding portion thereof has the properties (a), (c) and (e). In some embodiments, the antigen-binding polypeptide or the antigen-binding portion thereof has the properties (a), (b), (c) and (d). In some embodiments, the antigen-binding polypeptide or the antigen-binding portion thereof has the properties (a), (b), (c) and (e). In some embodiments, the antigen-binding polypeptide or the antigen-binding portion thereof has the properties (a), (b), (c) and (f). In some embodiments, the antigen-binding polypeptide or the antigen-binding portion thereof has the properties (a), (b), (c), (d) and (f). In some embodiments, the antigen-binding polypeptide or the antigen-binding portion thereof has the properties (a), (b), (c), (d), (e) and (f).

In some embodiments, the antigen-binding polypeptide that binds to CD47 and the antigen-binding portion thereof are an anti-CD47 antibody or an antigen-binding portion thereof.

In some embodiments, the isolated antigen-binding polypeptide that binds to CD47 provided herein is a monoclonal antibody.

In some embodiments, the isolated antibody that binds to CD47 provided herein is a monospecific antibody.

In some embodiments, the isolated antibody that binds to CD47 provided herein is a multispecific antibody, e.g., a bispecific antibody or trispecific antibody.

In some embodiments, the present disclosure provides an antigen-binding polypeptide that binds to CD47 or an antigen-binding portion thereof that binds to the same epitope on CD47 as any of the exemplary antibodies provided herein, e.g., binds to the same epitope as a chimeric or humanized antibody of 16E5, e.g., binds to the same epitope as a chimeric or humanized antibody of 3F10, and e.g., binds to the same epitope as a chimeric or humanized antibody of 14A9. In some embodiments, the antigen-binding polypeptide or the antigen-binding portion thereof competes with any of the exemplary antibodies provided herein for binding to CD47, e.g., competes with the chimeric or humanized antibody of 16E5 for binding to CD47, e.g., competes with the chimeric or humanized antibody of 3F10 for binding to CD47, and e.g., competes with the chimeric or humanized antibody of 14A9 for binding to CD47. The binding to CD47 can be measured by ELISA, flow cytometry or surface plasmon resonance (SPR) assay, or any other method known in the art.

The present disclosure provides some exemplary monoclonal antibodies that bind to CD47, including chimeric versions of the 2B2, 2H8, 3F10, 16E5 and 14A9 antibodies (Xi2B2, Xi2H8, Xi3F10, Xi16E5 and Xi14A9) and humanized variants of 3F10, 16E5 and 14A9. The amino acid sequences of the heavy chain CDRs (the heavy chain CDR1, the heavy chain CDR2 and the heavy chain CDR3) of the exemplary antibodies that bind to CD47 provided herein are provided in Table S1 below. The amino acid sequences of the light chain CDRs (the light chain CDR1, the light chain CDR2 and the light chain CDR3) are provided in Table S2 below. The amino acid sequences of the variable regions and the full-length heavy and light chains are provided in Tables S3 and S4 below.

Table S1: CDR sequences of heavy chains of anti-CD47 antibodies

| Name of antibody | Heavy chain CDR1 | Heavy chain CDR2 | Heavy chain CDR3 |
|---|---|---|---|
| Xi2H8 | GFSLTNYGVH (SEQ ID NO: 1) | IIWAGGSTN (SEQ ID NO: 6) | DDYASMDY (SEQ ID NO: 11) |
| Xi2B2 | GFTFSNYYMS (SEQ ID NO: 2) | YISSGGGSTY (SEQ ID NO: 7) | FAY (SEQ ID NO: 12) |
| Xi16E5 | GYTFTNYGMN (SEQ ID NO: 3) | WINTNTGEPT (SEQ ID NO: 8) | FSHLRGPMDY (SEQ ID NO: 13) |
| Xi3F10 | GYTFSRYWIE (SEQ ID NO: 4) | EFIPGSDT (SEQ ID NO: 9) | GGLRRMDY (SEQ ID NO: 14) |
| Xi14A9 | GFNIEDDYIE (SEQ ID NO: 5) | RIDPANDKTK (SEQ ID NO: 10) | PGLRRYYSMDY (SEQ ID NO: 15) |
| hz3F10-1.1 /2.1 /3.1 / 4.1 / 5.1 / 6.1 / 1.2 / 2.2 / 3.2 / 4.2 / 5.2 / 6.2 | GYTFSRYWIE (SEQ ID NO: 4) | EFIPGSDT (SEQ ID NO: 9) | GGLRRMDY (SEQ ID NO: 14) |
| hz16E5-1.1 / 1.3 / 1.2 / 3.2 / 3.1 / 3.3 | GYTFTNYGMN (SEQ ID NO: 3) | WINTNTGEPT (SEQ ID NO: 8) | FSHLRGPMDY (SEQ ID NO: 13) |
| hz14A9-2.3 / 2.4 | GFNIEDDYIE (SEQ ID NO: 5) | RIDPANDKTK (SEQ ID NO: 10) | PGLRRYYSMDY (SEQ ID NO: 15) |

Table S2: CDR sequences of light chains of anti-CD47 antibodies

| Name | Light chain CDR1 | Light chain CDR2 | Light chain CDR3 |
|---|---|---|---|
| Xi2H8 | RSSQSLVHSNGNTYLH (SEQ ID NO: 16) | KVSNRFS (SEQ ID NO: 21) | SQSTHVPYT (SEQ ID NO: 26) |
| Xi2B2 | KSSQSLLDSDGKTYLN (SEQ ID NO: 17) | LVSKLDS (SEQ ID NO: 22) | GTHFPLT (SEQ ID NO: 27) |
| Xi16E5 | RSSQSLVHSNGYTYLH (SEQ ID NO: 18) | KVSNRFS (SEQ ID NO: 23) | SQSTHVPPT (SEQ ID NO: 28) |
| Xi3F10 | RASSSVSSTYLH (SEQ ID NO: 19) | TTSTLAS (SEQ ID NO: 24) | QQFSDSTWT (SEQ ID NO: 29) |
| Xi14A9 | KASENVVSYVS (SEQ ID NO: 20) | GASNRYT (SEQ ID NO: 25) | GQSYSYPLT (SEQ ID NO: 30) |
| hz3F10-1.1 / 3.1 / 5.1 / 1.2 / 3.2 / 5.2 | RASSSVSSTYLH (SEQ ID NO: 19) | TTSTLAS (SEQ ID NO: 24) | QQFSDSTWT (SEQ ID NO: 29) |
| hz3F10-2.1 / 4.1 / 6.1 / 2.2 / 4.2 / 6.2 | RASSSVSSTYLH (SEQ ID NO: 19) | TTSTLAS (SEQ ID NO: 24) | QQFSESTWT (SEQ ID NO: 31) |
| hz16E5-1.1 / 1.3 / 1.2 / 3.2 / 3.1 / 3.3 | RSSQSLVHSNGYTYLH (SEQ ID NO: 18) | KVSNRFS (SEQ ID NO: 23) | SQSTHVPPT (SEQ ID NO: 28) |
| hz14A9-2.3 / 2.4 | KASENVVSYVS (SEQ ID NO: 20) | GASNRYT (SEQ ID NO: 25) | GQSYSYPLT (SEQ ID NO: 30) |
| Humanized 3F10 (common) | RASSSVSSTYLH (SEQ ID NO: 19) | TTSTLAS (SEQ ID NO: 24) | QQFSX$_2$STWT, wherein X$_2$ is D or E (SEQ ID NO: 32) |

Table S3: Variable region sequences of anti-CD47 antibodies

| Name | Region | Amino acid sequence | SEQ ID NO: |
|---|---|---|---|
| Xi2H8 | VH | QVQLKESGPGLVAPSQSLSITCTVS<u>GFSLTNYGVH</u>WVRQPPGKGLE WLG<u>IIWAGGSTN</u>YNSALMSRLSISKDNSKSQVFLKMNSLQTDDTA MYYCAR<u>DDYASMDY</u>WGQGTSVTVSS | 33 |
| | VL | DAFMTQTPLSLPVSLGDQASISC<u>RSSQSLVHSNGNTYLH</u>WYLQKPG QSPKLLIY<u>KVSNRFS</u>GVPDRFSGSGSGTDFTLKISRVEAEDLGVYFC <u>SQSTHVPYT</u>LGGGTKLEIK | 34 |
| Xil6E5 | VH | EVQLQQSGPELKKPGETVKISCKAS<u>GYTFTNYGMN</u>WVKQVPGKGL KWMG<u>WINTNTGEPT</u>YAEEFKGRFAFSLETSARTAFLQINNLKNEDT ATYFCTR<u>FSHLRGPMDY</u>WGQGASVTVSS | 35 |
| | VL | DAVMTQTPLSLPVSLGDQASISC<u>RSSQSLVHSNGYTYLH</u>WYLQKPG QSPKLLIY<u>KVSNRFS</u>GVPDRFSGSGSGTDFTLKISRLEAEDLGVYFC <u>SQSTHVPPTF</u>GGGTKLEIK | 36 |
| Xi2B2 | VH | EVMLVESGGGLVQPGGSLKLSCAAS<u>GFTFSNYYMS</u>WVRQTPEKRL EWVA<u>YISSGGGSTY</u>YPDSVKGRFTISRDNAKNTLYLQMSSLKSEDT AMYYCAW<u>FAY</u>WGQGTLVTVSA | 37 |
| | VL | DAVVTQTPLTLSVTIGQPASISC<u>KSSQSLLDSDGKTYLN</u>WLLQRPGQ SPKRLIY<u>LVSKLDS</u>GVPDRFTGSGSGTDFTLKISRVEAEDLGVYYC W<u>QGTHFPLT</u>FGAGTKLELK | 38 |
| Xi3F10 | VH | EVQLQQSVAELMKPGASVKIYCKAT<u>GYTFSRYWIE</u>WVKQRPGHGL EWIG<u>EFIPGSDT</u>TNFNEKFKGKATFTAEISSNTAYMQLSSLTSEDSA VYFCAR<u>GGLRRMDY</u>WGQGTSVTVSS | 39 |
| | VL | DIVMTQTPAIMSASPGEKVTMTC<u>RASSSVSSTYLH</u>WYQQKSGASPK LWIY<u>TTSTLAS</u>GVPARFSGSGSGTSYSLTISSVEAEDAATYYC<u>QQFS DSTWT</u>FGGGTKLEIK | 40 |
| Xi14A9 | VH | EVQLQQSGAEVARPGASVKLSCTVS<u>GFNIEDDYIE</u>WVKQRPEQGLE WIG<u>RIDPANDKTK</u>YAPKFQDKATITGDTSSNTAYLQLSSLTSEDTA VYYCTR<u>PGLRRYYSMDY</u>WGQGTSVTVSS | 41 |
| | VL | NIVLTQSPKSMSMSVGERVTLSCKAS<u>ENVVSYVS</u>WYQQKPEQSPK LLIY<u>GASNRYT</u>GVPDRFIGSGSSTDFTLIISSFQPEDLADYHC<u>GQSYS YPLT</u>FGAGTKLELK | 42 |
| hz3F10-1.1 | VH | QVQLVQSGAEVKKPGSSVKVSCKAS<u>GYTFSRYWIE</u>WVRQAPGQG LEWMG<u>EFIPGSDT</u>TNYAQKFQGRVTITADISTSTAYMELSSLRSEDT AVYYCAR<u>GGLRRMDY</u>WGQGTLVTVSS | 43 |
| | VL | DIQMTQSPSSLSASVGDRVTITC<u>RASSSVSSTYLH</u>WYQQKPGKAPK LLIY<u>TTSTLAS</u>GVPSRFSGSGSGTDFTLTISSLQPEDFATYYC<u>QQFSD STWT</u>FGQGTKLEIK | 44 |
| hz3F10-2.1 | VH | QVQLVQSGAEVKKPGSSVKVSCKAS<u>GYTFSRYWIE</u>WVRQAPGQG LEWMG<u>EFIPGSDT</u>TNYAQKFQGRVTITADISTSTAYMELSSLRSEDT AVYYCAR<u>GGLRRMDY</u>WGQGTLVTVSS | 45 |
| | VL | DIQMTQSPSSLSASVGDRVTITC<u>RASSSVSSTYLH</u>WYQQKPGKAPK LLIY<u>TTSTLAS</u>GVPSRFSGSGSGTDFTLTISSLQPEDFATYYC<u>QQFSES TWT</u>FGQGTKLEIK | 46 |

(continued)

| Name | Region | Amino acid sequence | SEQ ID NO: |
|---|---|---|---|
| hz3F10-3.1 | VH | QVQLVQSGAEVKKPGSSVKVSCKAS<u>GYTFSRYWIE</u>WVRQAPGQG LEWMG<u>EFIPGSD</u>TTNYAQKFQGRVTITADISTSTAYMELSSLRSEDT AVYYCAR<u>GGLRRMD</u>YWGQGTLVTVSS | 47 |
| | VL | DIQMTQSPSSLSASVGDRVTITC<u>RASSSVSSTYLH</u>WYQQKPGKAPK LLIY<u>TTSTLAS</u>GVPSRFSGSGSGTSYTLTISSLQPEDFATYYC<u>QQFSD STW</u>TFGQGTKLEIK | 48 |
| hz3F10-4.1 | VH | QVQLVQSGAEVKKPGSSVKVSCKAS<u>GYTFSRYWIE</u>WVRQAPGQG LEWMG<u>EFIPGSD</u>TTNYAQKFQGRVTITADISTSTAYMELSSLRSEDT AVYYCAR<u>GGLRRMD</u>YWGQGTLVTVSS | 49 |
| | VL | DIQMTQSPSSLSASVGDRVTITC<u>RASSSVSSTYLH</u>WYQQKPGKAPK LLIY<u>TTSTLAS</u>GVPSRFSGSGSGTSYTLTISSLQPEDFATYYC<u>QQFSES TW</u>TFGQGTKLEIK | 50 |
| hz3F10-5.1 | VH | QVQLVQSGAEVKKPGSSVKVSCKAS<u>GYTFSRYWIE</u>WVRQAPGQG LEWMG<u>EFIPGSD</u>TTNYAQKFQGRVTITADISTSTAYMELSSLRSEDT AVYYCAR<u>GGLRRMD</u>YWGQGTLVTVSS | 51 |
| | VL | DIQMTQSPSSLSASVGDRVTITC<u>RASSSVSSTYLH</u>WYQQKPGKAPK LWIY<u>TTSTLAS</u>GVPSRFSGSGSGTSYTLTISSLQPEDFATYYC<u>QQFSD STW</u>TFGQGTKLEIK | 52 |
| hz3F10-6.1 | VH | QVQLVQSGAEVKKPGSSVKVSCKAS<u>GYTFSRYWIE</u>WVRQAPGQG LEWMG<u>EFIPGSD</u>TTNYAQKFQGRVTITADISTSTAYMELSSLRSEDT AVYYCAR<u>GGLRRMD</u>YWGQGTLVTVSS | 53 |
| | VL | DIQMTQSPSSLSASVGDRVTITC<u>RASSSVSSTYLH</u>WYQQKPGKAPK LWIY<u>TTSTLAS</u>GVPSRFSGSGSGTSYTLTISSLQPEDFATYYC<u>QQFSE STW</u>TFGQGTKLEIK | 54 |
| hz3F10-1.2 | VH | QVQLVQSGAEVKKPGSSVKVSCKAS<u>GYTFSRYWIE</u>WVRQAPGQG LEWMG<u>EFIPGSD</u>TTNYAQKFQGRVTITAEISTNTAYMELSSLRSEDT AVYYCAR<u>GGLRRMD</u>YWGQGTLVTVSS | 55 |
| | VL | DIQMTQSPSSLSASVGDRVTITC<u>RASSSVSSTYLH</u>WYQQKPGKAPK LLIY<u>TTSTLAS</u>GVPSRFSGSGSGTDFTLTISSLQPEDFATYYC<u>QQFSD STW</u>TFGQGTKLEIK | 56 |
| hz3F10-2.2 | VH | QVQLVQSGAEVKKPGSSVKVSCKAS<u>GYTFSRYWIE</u>WVRQAPGQG LEWMG<u>EFIPGSD</u>TTNYAQKFQGRVTITAEISTNTAYMELSSLRSEDT AVYYCAR<u>GGLRRMD</u>YWGQGTLVTVSS | 57 |
| | VL | DIQMTQSPSSLSASVGDRVTITC<u>RASSSVSSTYLH</u>WYQQKPGKAPK LLIY<u>TTSTLAS</u>GVPSRFSGSGSGTDFTLTISSLQPEDFATYYC<u>QQFSES TW</u>TFGQGTKLEIK | 58 |
| hz3F10-3.2 | VH | QVQLVQSGAEVKKPGSSVKVSCKAS<u>GYTFSRYWIE</u>WVRQAPGQG LEWMG<u>EFIPGSD</u>TTNYAQKFQGRVTITAEISTNTAYMELSSLRSEDT AVYYCAR<u>GGLRRMD</u>YWGQGTLVTVSS | 59 |
| | VL | DIQMTQSPSSLSASVGDRVTITC<u>RASSSVSSTYLH</u>WYQQKPGKAPK LLIY<u>TTSTLAS</u>GVPSRFSGSGSGTSYTLTISSLQPEDFATYYC<u>QQFSD STW</u>TFGQGTKLEIK | 60 |

(continued)

| Name | Region | Amino acid sequence | SEQ ID NO: |
|---|---|---|---|
| hz3F10-4.2 | VH | QVQLVQSGAEVKKPGSSVKVSCKAS<u>GYTFSRYWIE</u>WVRQAPGQG LEWMG<u>EFIPGSD</u>TTNYAQKFQGRVTITAEISTNTAYMELSSLRSEDT AVYYCAR<u>GGLRRMD</u>YWGQGTLVTVSS | 61 |
| | VL | DIQMTQSPSSLSASVGDRVTITC<u>RASSSVSSTYLH</u>WYQQKPGKAPK LLIY<u>TTSTLAS</u>GVPSRFSGSGSGTSYTLTISSLQPEDFATYYC<u>QQFSES TW</u>TFGQGTKLEIK | 62 |
| hz3F10-5.2 | VH | QVQLVQSGAEVKKPGSSVKVSCKAS<u>GYTFSRYWIE</u>WVRQAPGQG LEWMG<u>EFIPGSD</u>TTNYAQKFQGRVTITAEISTNTAYMELSSLRSEDT AVYYCAR<u>GGLRRMD</u>YWGQGTLVTVSS | 63 |
| | VL | DIQMTQSPSSLSASVGDRVTITC<u>RASSSVSSTYLH</u>WYQQKPGKAPK LWIY<u>TTSTLAS</u>GVPSRFSGSGSGTSYTLTISSLQPEDFATYYC<u>QQFSD STW</u>TFGQGTKLEIK | 64 |
| hz3F10-6.2 | VH | QVQLVQSGAEVKKPGSSVKVSCKAS<u>GYTFSRYWIE</u>WVRQAPGQG LEWMG<u>EFIPGSD</u>TTNYAQKFQGRVTITAEISTNTAYMELSSLRSEDT AVYYCAR<u>GGLRRMD</u>YWGQGTLVTVSS | 65 |
| | VL | DIQMTQSPSSLSASVGDRVTITC<u>RASSSVSSTYLH</u>WYQQKPGKAPK LWIY<u>TTSTLAS</u>GVPSRFSGSGSGTSYTLTISSLQPEDFATYYC<u>QQFSE STW</u>TFGQGTKLEIK | 66 |
| hz16E5-1.1 | VH | QVQLVQSGAEVKKPGASVKVSCKAS<u>GYTFTNYGMN</u>WVRQAPGQ GLEWMG<u>WINTNTGEPT</u>YAQKLQGRVTMTLDTSTRTAYMELRSLR SDDTAVYYCAR<u>FSHLRGPMD</u>YWGQGTLVTVSS | 67 |
| | VL | DIQMTQSPSSLSASVGDRVTITC<u>RSSQSLVHSNGYTYLH</u>WYQQKPG KAPKLLIY<u>KVSNRFS</u>GVPSRFSGSGSGTDFTLTISSLQPEDFATYYC<u>S QSTHVPPT</u>FGQGTKLEIK | 68 |
| hz16E5-1.3 | VH | QVQLVQSGAEVKKPGASVKVSCKAS<u>GYTFTNYGMN</u>WVRQAPGQ GLKWMG<u>WINTNTGEPT</u>YAQELQGRVTMTLDTSTRTAYMELRSLR SDDTAVYYCTR<u>FSHLRGPMD</u>YWGQGTLVTVSS | 69 |
| | VL | DIQMTQSPSSLSASVGDRVTITC<u>RSSQSLVHSNGYTYLH</u>WYQQKPG KAPKLLIY<u>KVSNRFS</u>GVPSRFSGSGSGTDFTLTISSLQPEDFATYYC<u>S QSTHVPPT</u>FGQGTKLEIK | 70 |
| hz16E5-1.2 | VH | QVQLVQSGAEVKKPGASVKVSCKAS<u>GYTFTNYGMN</u>WVRQAPGQ GLKWMG<u>WINTNTGEPT</u>YAQELQGRVTMTLDTSTRTAYMELRSLR SDDTAVYYCAR<u>FSHLRGPMD</u>YWGQGTLVTVSS | 71 |
| | VL | DIQMTQSPSSLSASVGDRVTITC<u>RSSQSLVHSNGYTYLH</u>WYQQKPG KAPKLLIY<u>KVSNRFS</u>GVPSRFSGSGSGTDFTLTISSLQPEDFATYYC<u>S QSTHVPPT</u>FGQGTKLEIK | 72 |
| hz16E5-3.2 | VH | QVQLVQSGAEVKKPGASVKVSCKAS<u>GYTFTNYGMN</u>WVRQAPGQ GLKWMG<u>WINTNTGEPT</u>YAQELQGRVTMTLDTSTRTAYMELRSLR SDDTAVYYCAR<u>FSHLRGPMD</u>YWGQGTLVTVSS | 73 |
| | VL | DAQMTQSPSSLSASVGDRVTITC<u>RSSQSLVHSNGYTYLH</u>WYQQKP GKAPKLLIY<u>KVSNRFS</u>GVPSRFSGSGSGTDFTLTISSLQPEDFATYYC <u>SQSTHVPPT</u>FGQGTKLEIK | 74 |

(continued)

| Name | Region | Amino acid sequence | SEQ ID NO: |
|---|---|---|---|
| hz16E5-3.1 | VH | QVQLVQSGAEVKKPGASVKVSCKAS<u>GYTFTNYGMN</u>WVRQAPGQGLEWMG<u>WINTNTGEPT</u>YAQKLQGRVTMTLDTSTRTAYMELRSLRSDDTAVYYCARF<u>SHLRGPMD</u>YWGQGTLVTVSS | 75 |
| | VL | DAQMTQSPSSLSASVGDRVTITC<u>RSSQSLVHSNGYTYLH</u>WYQQKPGKAPKLLIY<u>KVSNRFS</u>GVPSRFSGSGSGTDFTLTISSLQPEDFATYYC<u>SQSTHVPPT</u>FGQGTKLEIK | 76 |
| hz16E5-3.3 | VH | QVQLVQSGAEVKKPGASVKVSCKAS<u>GYTFTNYGMN</u>WVRQAPGQGLKWMG<u>WINTNTGEPT</u>YAQELQGRVTMTLDTSTRTAYMELRSLRSDDTAVYYCTRF<u>SHLRGPMD</u>YWGQGTLVTVSS | 77 |
| | VL | DAQMTQSPSSLSASVGDRVTITC<u>RSSQSLVHSNGYTYLH</u>WYQQKPGKAPKLLIY<u>KVSNRFS</u>GVPSRFSGSGSGTDFTLTISSLQPEDFATYYC<u>SQSTHVPPT</u>FGQGTKLEIK | 78 |
| hz14A9-2.3 | VH | QVQLVQSGAEVKKPGASVKVSCKVS<u>GFNIEDDYIE</u>WVRQAPGQGLEWMG<u>RIDPANDKTK</u>YAQKFQGRVTMTGDTSTNTVYMELSSLRSEDTAVYYCARP<u>GLRRYYSMD</u>YWGQGTLVTVSS | 79 |
| | VL | DIQMTQSPSSLSASVGDRVTITC<u>KASENVVSYVS</u>WYQQKPGKAPKLLIY<u>GASNRYT</u>GVPSRFIGSGSSTDFTLTISSLQPEDFATYYC<u>GQSYSYPLT</u>FGQGTKLEIK | 80 |
| hz14A9-2.4 | VH | QVQLVQSGAEVKKPGASVKVSCKVS<u>GFNIEDDYIE</u>WVRQAPGQGLEWMG<u>RIDPANDKTK</u>YAQKFQGRVTMTGDTSTNTVYMELSSLRSEDTAVYYC**T**RP<u>GLRRYYSMD</u>YWGQGTLVTVSS | 81 |
| | VL | DIQMTQSPSSLSASVGDRVTITC<u>KASENVVSYVS</u>WYQQKPGKAPKLLIY<u>GASNRYT</u>GVPSRFIGSGSSTDFTLTISSLQPEDFATYYC<u>GQSYSYPLT</u>FGQGTKLEIK | 82 |
| Humanized 3F10 (common) | VH | QVQLVQSGAEVKKPGSSVKVSCKAS<u>GYTFSRYWIE</u>WVRQAPGQGLEWMG<u>EFIPGSD</u>TTNYAQKFQGRVTITAX$_3$X$_4$STX$_5$TAYMELSSLRSEDTAVYYCAR<u>GGLRRMD</u>YWGQGTLVTVSS<br>wherein X$_3$ is D or E, X$_4$ is I or E, and X$_5$ is S or N | 83 |
| | VL | DIQMTQSPSSLSASVGDRVTITC<u>RASSSVSSTYLH</u>WYQQKPGKAPKLX$_6$IY<u>TTSTLAS</u>GVPSRFSGSGSGTX$_7$X$_8$TLTISSLQPEDFATYYC<u>QQFSX$_2$STWT</u>FGQGTKLEIK<br>wherein X$_6$ is L or W, X$_7$ is D or S, X$_8$ is F or Y, and X$_2$ is D or E | 84 |
| Humanized 16E5 (common) | VH | QVQLVQSGAEVKKPGASVKVSCKAS<u>GYTFTNYGMN</u>WVRQAPGQGLX$_9$WMG<u>WINTNTGEPT</u>YAQX$_{10}$LQGRVTMTX$_{11}$DTSTX$_{12}$TAYMELRSLRSDDTAVYYC X$_{13}$RF<u>SHLRGPMD</u>YWGQGTLVTVSS<br>wherein X$_9$ is E or K, X$_{10}$ is K or E, X$_{11}$ is L or T, X$_{12}$ is R or S, and X$_{13}$ is A or T | 85 |
| | VL | DX$_{14}$QMTQSPSSLSASVGDRVTITC<u>RSSQSLVHSNGYTYLH</u>WYQQKPGKAPKLLIY<u>KVSNRFS</u>GVPSRFSGSGSGTDFTLTISSLQPEDFATYYC<u>SQSTHVPPT</u>FGQGTKLEIK<br>wherein X$_{14}$ is I or A | 86 |

(continued)

| Name | Region | Amino acid sequence | SEQ ID NO: |
|---|---|---|---|
| Humanized 14A9 (common) | VH | QVQLVQSGAEVKKPGASVKVSCKX$_{15}$SGFNIEDDYIEWVRQAPGQG LEWMGRIDPANDKTKYAQKFQGRVTMTX$_{16}$DTSTX$_{17}$TVYMELSSL RSEDTAVYYC X$_{18}$RPGLRRYYSMDYWGQGTLVTVSS<br><br>wherein X$_{15}$ is A or V, X$_{16}$ is R or G, X$_{17}$ is S or N, X$_{18}$ is A or T | 87 |
| | VL | DIQMTQSPSSLSASVGDRVTITCKASENVVSYVSWYQQKPGKAPKL LIYGASNRYTGVPSRFX$_{19}$GSGSX$_{20}$TDFTLTISSLQPEDFATYYCGQS YSYPLTFGQGTKLEIK<br><br>wherein X$_{19}$ is S or I, and X$_{20}$ is S or G | 88 |

Table S4: Sequences of full-length heavy chains and full-length light chains of anti-CD47 antibodies

| Name | Region | Amino acid sequence | SEQ ID NO: |
|---|---|---|---|
| Xi2H8 | Heavy chain | QVQLKESGPGLVAPSQSLSITCTVSGFSLTNYGVHWVRQPPGKGLEWLGI IWAGGSTNYNSALMSRLSISKDNSKSQVFLKMNSLQTDDTAMYYCARDD YASMDYWGQGTSVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 89 |
| | Light chain | DAFMTQTPLSLPVSLGDQASISCRSSQSLVHSNGNTYLHWYLQKPGQSPK LLIYKVSNRFSGVPDRFSGSGSGTDFTLKISRVEAEDLGVYFCSQSTHVPY TLGGGTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 91 |
| Xil6E5 | Heavy chain | EVQLQQSGPELKKPGETVKISCKASGYTFTNYGMNWVKQVPGKGLKWM GWINTNTGEPTYAEEFKGRFAFSLETSARTAFLQINNLKNEDTATYFCTRF SHLRGPMDYWGQGASVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 93 |
| | Light chain | DAVMTQTPLSLPVSLGDQASISCRSSQSLVHSNGYTYLHWYLQKPGQSPK LLIYKVSNRFSGVPDRFSGSGSGTDFTLKISRLEAEDLGVYFCSQSTHVPPT FGGGTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 95 |

(continued)

| Name | Region | Amino acid sequence | SEQ ID NO: |
|------|--------|---------------------|------------|
| Xi2B2 | Heavy chain | EVMLVESGGGLVQPGGSLKLSCAASGFTFSNYYMSWVRQTPEKRLEWV AYISSGGGSTYYPDSVKGRFTISRDNAKNTLYLQMSSLKSEDTAMYYCA WFAYWGQGTLVTVSA ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 97 |
| | Light chain | DAVVTQTPLTLSVTIGQPASISCKSSQSLLDSDGKTYLNWLLQRPGQSPKR LIYLVSKLDSGVPDRFTGSGSGTDFTLKISRVEAEDLGVYYCWQGTHFPL TFGAGTKLELK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 99 |
| Xi3F10 | Heavy chain | EVQLQQSVAELMKPGASVKIYCKATGYTFSRYWIEWVKQRPGHGLEWIG EFIPGSDTTNFNEKFKGKATFTAEISSNTAYMQLSSLTSEDSAVYFCARGG LRRMDYWGQGTSVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 101 |
| | Light chain | DIVMTQTPAIMSASPGEKVTMTCRASSSVSSTYLHWYQQKSGASPKLWIY TTSTLASGVPARFSGSGSGTSYSLTISSVEAEDAATYYCQQFSDSTWTFGG GTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 103 |
| Xi14A9 | Heavy chain | EVQLQQSGAEVARPGASVKLSCTVSGFNIEDDYIEWVKQRPEQGLEWIGR IDPANDKTKYAPKFQDKATITGDTSSNTAYLQLSSLTSEDTAVYYCTRPG LRRYYSMDYWGQGTSVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 105 |
| | Light chain | NIVLTQSPKSMSMSVGERVTLSCKASENVVSYVSWYQQKPEQSPKLLIYG ASNRYTGVPDRFIGSGSSTDFTLIISSFQPEDLADYHCGQSYSYPLTFGAGT KLELK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 107 |

(continued)

| Name | Region | Amino acid sequence | SEQ ID NO: |
|------|--------|---------------------|------------|
| hz3F10-1.1 | Heavy chain | QVQLVQSGAEVKKPGSSVKVSCKAS<u>GYTFSRYWIE</u>WVRQAPGQGLEWM G<u>EFIPGSDT</u>TNYAQKFQGRVTITADISTSTAYMELSSLRSEDTAVYYCAR<u>G GLRRMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 109 |
| | Light chain | DIQMTQSPSSLSASVGDRVTITC<u>RASSSVSSTYLH</u>WYQQKPGKAPKLLIY<u>T TSTLAS</u>GVPSRFSGSGSGTDFTLTISSLQPEDFATYYC<u>QQFSDSTWT</u>FGQG TKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 111 |
| hz3F10-2.1 | Heavy chain | QVQLVQSGAEVKKPGSSVKVSCKAS<u>GYTFSRYWIE</u>WVRQAPGQGLEWM G<u>EFIPGSDT</u>TNYAQKFQGRVTITADISTSTAYMELSSLRSEDTAVYYCAR<u>G GLRRMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 113 |
| | Light chain | DIQMTQSPSSLSASVGDRVTITC<u>RASSSVSSTYLH</u>WYQQKPGKAPKLLIY<u>T TSTLAS</u>GVPSRFSGSGSGTDFTLTISSLQPEDFATYYC<u>QQFSESTWT</u>FGQGT KLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 115 |
| hz3F10-3.1 | Heavy chain | QVQLVQSGAEVKKPGSSVKVSCKAS<u>GYTFSRYWIE</u>WVRQAPGQGLEWM G<u>EFIPGSDT</u>TNYAQKFQGRVTITADISTSTAYMELSSLRSEDTAVYYCAR<u>G GLRRMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 117 |
| | Light chain | DIQMTQSPSSLSASVGDRVTITC<u>RASSSVSSTYLH</u>WYQQKPGKAPKLLIY<u>T TSTLAS</u>GVPSRFSGSGSGTSYTLTISSLQPEDFATYYC<u>QQFSDSTWT</u>FGQG TKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 119 |

(continued)

| Name | Region | Amino acid sequence | SEQ ID NO: |
|------|--------|---------------------|------------|
| hz3F10-4.1 | Heavy chain | QVQLVQSGAEVKKPGSSVKVSCKASGYTFSRYWIEWVRQAPGQGLEWM GEFIPGSDTTNYAQKFQGRVTITADISTSTAYMELSSLRSEDTAVYYCARG GLRRMDYWGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 121 |
| | Light chain | DIQMTQSPSSLSASVGDRVTITCRASSSVSSTYLHWYQQKPGKAPKLLIYT TSTLASGVPSRFSGSGSGTSYTLTISSLQPEDFATYYCQQFSESTWTFGQGT KLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 123 |
| hz3F10-5.1 | Heavy chain | QVQLVQSGAEVKKPGSSVKVSCKASGYTFSRYWIEWVRQAPGQGLEWM GEFIPGSDTTNYAQKFQGRVTITADISTSTAYMELSSLRSEDTAVYYCARG GLRRMDYWGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 125 |
| | Light chain | DIQMTQSPSSLSASVGDRVTITCRASSSVSSTYLHWYQQKPGKAPKLWIY TTSTLASGVPSRFSGSGSGTSYTLTISSLQPEDFATYYCQQFSDSTWTFGQ GTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 127 |
| hz3F10-6.1 | Heavy chain | QVQLVQSGAEVKKPGSSVKVSCKASGYTFSRYWIEWVRQAPGQGLEWM GEFIPGSDTTNYAQKFQGRVTITADISTSTAYMELSSLRSEDTAVYYCARG GLRRMDYWGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 129 |
| | Light chain | DIQMTQSPSSLSASVGDRVTITCRASSSVSSTYLHWYQQKPGKAPKLWIY TTSTLASGVPSRFSGSGSGTSYTLTISSLQPEDFATYYCQQFSESTWTFGQG TKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 131 |

(continued)

| Name | Region | Amino acid sequence | SEQ ID NO: |
|------|--------|---------------------|------------|
| hz3F10-1.2 | Heavy chain | QVQLVQSGAEVKKPGSSVKVSCKAS<u>GYTFSRYWIE</u>WVRQAPGQGLEWM<u>GEFIPGSD</u>TTNYAQKFQGRVTITAEISTNTAYMELSSLRSEDTAVYYCAR<u>GGLRRMD</u>YWGQGTLVTVSS ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK | 133 |
| | Light chain | DIQMTQSPSSLSASVGDRVTITC<u>RASSSVSSTYLH</u>WYQQKPGKAPKLLIY<u>TTSTLAS</u>GVPSRFSGSGSGTDFTLTISSLQPEDFATYYC<u>QQFSDSTWT</u>FGQGTKLEIK RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 135 |
| hz3F10-2.2 | Heavy chain | QVQLVQSGAEVKKPGSSVKVSCKAS<u>GYTFSRYWIE</u>WVRQAPGQGLEWM<u>GEFIPGSD</u>TTNYAQKFQGRVTITAEISTNTAYMELSSLRSEDTAVYYCAR<u>GGLRRMD</u>YWGQGTLVTVSS ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK | 137 |
| | Light chain | DIQMTQSPSSLSASVGDRVTITC<u>RASSSVSSTYLH</u>WYQQKPGKAPKLLIY<u>TTSTLAS</u>GVPSRFSGSGSGTDFTLTISSLQPEDFATYYC<u>QQFSESTWT</u>FGQGTKLEIK RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 139 |
| hz3F10-3.2 | Heavy chain | QVQLVQSGAEVKKPGSSVKVSCKAS<u>GYTFSRYWIE</u>WVRQAPGQGLEWM<u>GEFIPGSD</u>TTNYAQKFQGRVTITAEISTNTAYMELSSLRSEDTAVYYCAR<u>GGLRRMD</u>YWGQGTLVTVSS ASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEFLGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK | 141 |
| | Light chain | DIQMTQSPSSLSASVGDRVTITC<u>RASSSVSSTYLH</u>WYQQKPGKAPKLLIY<u>TTSTLAS</u>GVPSRFSGSGSGTSYTLTISSLQPEDFATYYC<u>QQFSDSTWT</u>FGQGTKLEIK RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 143 |

(continued)

| Name | Region | Amino acid sequence | SEQ ID NO: |
|---|---|---|---|
| hz3F10-4.2 | Heavy chain | QVQLVQSGAEVKKPGSSVKVSCKASGYTFSRYWIEWVRQAPGQGLEWM GEFIPGSDTTNYAQKFQGRVTITAEISTNTAYMELSSLRSEDTAVYYCARG GLRRMDYWGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 145 |
| | Light chain | DIQMTQSPSSLSASVGDRVTITCRASSSVSSTYLHWYQQKPGKAPKLLIYT TSTLASGVPSRFSGSGSGTSYTLTISSLQPEDFATYYCQQFSESTWTFGQGT KLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 147 |
| hz3F10-5.2 | Heavy chain | QVQLVQSGAEVKKPGSSVKVSCKASGYTFSRYWIEWVRQAPGQGLEWM GEFIPGSDTTNYAQKFQGRVTITAEISTNTAYMELSSLRSEDTAVYYCARG GLRRMDYWGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 149 |
| | Light chain | DIQMTQSPSSLSASVGDRVTITCRASSSVSSTYLHWYQQKPGKAPKLWIY TTSTLASGVPSRFSGSGSGTSYTLTISSLQPEDFATYYCQQFSDSTWTFGQ GTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 151 |
| hz3F10-6.2 | Heavy chain | QVQLVQSGAEVKKPGSSVKVSCKASGYTFSRYWIEWVRQAPGQGLEWM GEFIPGSDTTNYAQKFQGRVTITAEISTNTAYMELSSLRSEDTAVYYCARG GLRRMDYWGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 153 |
| | Light chain | DIQMTQSPSSLSASVGDRVTITCRASSSVSSTYLHWYQQKPGKAPKLWIY TTSTLASGVPSRFSGSGSGTSYTLTISSLQPEDFATYYCQQFSESTWTFGQG TKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 155 |

(continued)

| Name | Region | Amino acid sequence | SEQ ID NO: |
|---|---|---|---|
| hz16E5-1.1 | Heavy chain | QVQLVQSGAEVKKPGASVKVSCKAS<u>GYTFTNYGMN</u>WVRQAPGQGLEW MG<u>WINTNTGEPT</u>YAQKLQGRVTMTLDTSTRTAYMELRSLRSDDTAVYY CARF<u>SHLRGPMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 157 |
| | Light chain | DIQMTQSPSSLSASVGDRVTITC<u>RSSQSLVHSNGYTYLH</u>WYQQKPGKAPK LLIY<u>KVSNRFS</u>GVPSRFSGSGSGTDFTLTISSLQPEDFATYYC<u>SQSTHVPPT</u> FGQGTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 159 |
| hz16E5-1.3 | Heavy chain | QVQLVQSGAEVKKPGASVKVSCKAS<u>GYTFTNYGMN</u>WVRQAPGQGLKW MG<u>WINTNTGEPT</u>YAQELQGRVTMTLDTSTRTAYMELRSLRSDDTAVYY CTRF<u>SHLRGPMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 161 |
| | Light chain | DIQMTQSPSSLSASVGDRVTITC<u>RSSQSLVHSNGYTYLH</u>WYQQKPGKAPK LLIY<u>KVSNRFS</u>GVPSRFSGSGSGTDFTLTISSLQPEDFATYYC<u>SQSTHVPPT</u> FGQGTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 163 |
| hz16E5-1.2 | Heavy chain | QVQLVQSGAEVKKPGASVKVSCKAS<u>GYTFTNYGMN</u>WVRQAPGQGLKW MG<u>WINTNTGEPT</u>YAQELQGRVTMTLDTSTRTAYMELRSLRSDDTAVYY CARF<u>SHLRGPMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 165 |
| | Light chain | DIQMTQSPSSLSASVGDRVTITC<u>RSSQSLVHSNGYTYLH</u>WYQQKPGKAPK LLIY<u>KVSNRFS</u>GVPSRFSGSGSGTDFTLTISSLQPEDFATYYC<u>SQSTHVPPT</u> FGQGTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 167 |

(continued)

| Name | Region | Amino acid sequence | SEQ ID NO: |
|---|---|---|---|
| hz16E5-3.2 | Heavy chain | QVQLVQSGAEVKKPGASVKVSCKAS<u>GYTFTNYGMN</u>WVRQAPGQGLKW MG<u>WINTNTGEPT</u>YAQELQGRVTMTLDTSTRTAYMELRSLRSDDTAVYY CARF<u>SHLRGPMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 169 |
| | Light chain | DAQMTQSPSSLSASVGDRVTITC<u>RSSQSLVHSNGYTYLH</u>WYQQKPGKAP KLLIY<u>KVSNRFSG</u>VPSRFSGSGSGTDFTLTISSLQPEDFATYYC<u>SQSTHVPP T</u>FGQGTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 171 |
| hz16E5-3.1 | Heavy chain | QVQLVQSGAEVKKPGASVKVSCKAS<u>GYTFTNYGMN</u>WVRQAPGQGLEW MGWINTNTGEPTYAQKLQGRVTMTLDTSTRTAYMELRSLRSDDTAVYY CARF<u>SHLRGPMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 173 |
| | Light chain | DAQMTQSPSSLSASVGDRVTITC<u>RSSQSLVHSNGYTYLH</u>WYQQKPGKAP KLLIY<u>KVSNRFSG</u>VPSRFSGSGSGTDFTLTISSLQPEDFATYYC<u>SQSTHVPP T</u>FGQGTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 175 |
| hz16E5-3.3 | Heavy chain | QVQLVQSGAEVKKPGASVKVSCKAS<u>GYTFTNYGMN</u>WVRQAPGQGLKW MG<u>WINTNTGEPT</u>YAQELQGRVTMTLDTSTRTAYMELRSLRSDDTAVYY CTRF<u>SHLRGPMDY</u>WGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 177 |
| | Light chain | DAQMTQSPSSLSASVGDRVTITC<u>RSSQSLVHSNGYTYLH</u>WYQQKPGKAP KLLIY<u>KVSNRFSG</u>VPSRFSGSGSGTDFTLTISSLQPEDFATYYC<u>SQSTHVPP T</u>FGQGTKLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 179 |

(continued)

| Name | Region | Amino acid sequence | SEQ ID NO: |
|------|--------|---------------------|------------|
| hz14A9-2.3 | Heavy chain | QVQLVQSGAEVKKPGASVKVSCKVSGFNIEDDYIEWVRQAPGQGLEWM GRIDPANDKTKYAQKFQGRVTMTGDTSTNTVYMELSSLRSEDTAVYYCA RPGLRRYYSMDYWGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 181 |
| | Light chain | DIQMTQSPSSLSASVGDRVTITCKASENVVSYVSWYQQKPGKAPKLLIYG ASNRYTGVPSRFIGSGSSTDFTLTISSLQPEDFATYYCGQSYSYPLTFGQGT KLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 183 |
| hz14A9-2.4 | Heavy chain | QVQLVQSGAEVKKPGASVKVSCKVSGFNIEDDYIEWVRQAPGQGLEWM GRIDPANDKTKYAQKFQGRVTMTGDTSTNTVYMELSSLRSEDTAVYYCT RPGLRRYYSMDYWGQGTLVTVSS ASTKGPSVFPLAPCSRSTSESTAALGCLVKDYFPEPVTVSWNSGALTSGV HTFPAVLQSSGLYSLSSVVTVPSSSLGTKTYTCNVDHKPSNTKVDKRVES KYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKE YKCKVSNKGLPSSIEKTISKAKGQPREPQVYTLPPSQEEMTKNQVSLTCLV KGFYPSDIAVEWESNGQPENNYKTTPPVLDSDGSFFLYSRLTVDKSRWQE GNVFSCSVMHEALHNHYTQKSLSLSLGK | 185 |
| | Light chain | DIQMTQSPSSLSASVGDRVTITCKASENVVSYVSWYQQKPGKAPKLLIYG ASNRYTGVPSRFIGSGSSTDFTLTISSLQPEDFATYYCGQSYSYPLTFGQGT KLEIK RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC | 187 |

*Immunoconjugate*

In some embodiments, the present disclosure provides an immunoconjugate of an antigen-binding polypeptide that binds to CD47 or an antigen-binding portion thereof. Therapeutic agents that can be linked or conjugated to the antigen-binding polypeptide that binds to CD47 or the antigen-binding portion thereof may include, but are not limited to, cytotoxic drugs, radioisotopes, immunomodulators or antibodies. In some embodiments, the antigen-binding polypeptide that binds to CD47 or the antigen-binding portion thereof is directly conjugated to the therapeutic agent. In some embodiments, the antigen-binding polypeptide that binds to CD47 or the antigen-binding portion thereof is conjugated to the therapeutic agent through a linker.

*Composition*

The present disclosure provides a pharmaceutical composition comprising an isolated antigen-binding polypeptide that binds to CD47 or an antigen-binding portion thereof, or a nucleic acid encoding the antibody or fragment, or the immunoconjugate described herein, and further one or more pharmaceutically acceptable carriers. In some embodiments, the composition comprises any monoclonal antibody of the chimeric and humanized antibodies of antibodies 2B2, 2H8, 3F10, 16E5 and 14A9, and a pharmaceutically acceptable carrier. The pharmaceutically acceptable carriers include, for example, excipients, diluents, encapsulating materials, fillers, buffers or other agents.

*Hybridoma*

The present disclosure provides an isolated hybridoma cell line selected from the group consisting of 2B2, 2H8, 3F10, 16E5 and 14A9. The hybridoma cell lines 2B2, 2H8, 3F10, 16E5 and 14A9 secrete monoclonal antibodies that bind to CD47.

*Isolated Nucleic Acid*

The present disclosure provides an isolated nucleic acid comprising a sequence encoding the antigen-binding polypeptide that binds to CD47 or the antigen-binding portion thereof described herein. In some embodiments, the nucleic acid may encode an amino acid sequence comprising the antigen-binding polypeptide heavy chain variable region or/and light chain variable region described herein. In some other embodiments, the nucleic acid may encode an amino acid sequence comprising the antigen-binding polypeptide heavy chain or/and the antigen-binding polypeptide light chain described herein. The nucleotide sequences of some of the variable regions and the heavy and light chains of the antigen-binding polypeptide are listed in the sequence listing.

*Vector*

The present disclosure provides a vector comprising the isolated nucleic acid. In some embodiments, the vector is a cloning vector; in some other embodiments, the vector is an expression vector.
The expression vector is optionally any expression vector capable of expressing the antigen-binding polypeptide or the antigen-binding portion thereof described herein.

*Host cell*

In some embodiments, the present disclosure provides a host cell comprising the vector, the host cell being a suitable host cell for cloning or encoding an antigen-binding polypeptide that binds to CD47 or an antigen-binding portion thereof. In some embodiments, the host cell is a prokaryotic cell. In other embodiments, the host cell is a eukaryotic cell. In some embodiments, the host cell is selected from yeast cells, mammalian cells or other cells suitable for preparing an antigen-binding polypeptide or an antigen-binding portion thereof. Mammalian cells are, for example, Chinese hamster ovary (CHO) cells.

*Method for Preparing Antigen-binding Polypeptides that Bind to CD47*

In some embodiments, the present disclosure provides a method for preparing an isolated antigen-binding polypeptide that binds to CD47, which comprises: culturing a host cell comprising a nucleic acid encoding the antigen-binding polypeptide under conditions suitable for expression of the antigen-binding polypeptide, and recovering the antigen-binding polypeptide from the host cell or host cell culture medium. To produce an antigen-binding polypeptide that binds to CD47, a nucleic acid encoding the antibody is isolated and inserted into one or more vectors for further cloning and/or expression in a host cell. The nucleic acid can be obtained using various methods known in the art, such as gene cloning, gene splicing and chemical synthesis.

*Use*

The present disclosure provides use of an isolated antigen-binding polypeptide that binds to CD47 or an antigen-binding portion thereof. In some embodiments, administering to a subject a therapeutically effective amount of the isolated antigen-binding polypeptide that binds to CD47 or the antigen-binding portion thereof described herein can reduce or inhibit tumor cell growth. In some embodiments, administering to a subject a therapeutically effective amount of the isolated antigen-binding polypeptide that binds to CD47 or the antigen-binding portion thereof described herein can treat cancer. Subjects in need of treatment include those with a disease or condition, as well as those who may have the disease or condition and whose purpose is to prevent, delay or alleviate the disease or condition. As used herein, "cancer" refers to a physiological condition in mammals that is typically characterized by unregulated cell growth. Examples of cancer include, but are not limited to leukemia, lymphoma, ovarian cancer, breast cancer, endometrial cancer, colon cancer, rectal cancer, bladder cancer, urothelial cancer, lung cancer, bronchial cancer, bone cancer, prostate cancer, pancreatic cancer, gastric cancer, hepatocellular cancer, gallbladder cancer, bile duct cancer, esophageal cancer, renal cell cancer, thyroid cancer, head and neck cancer, testicular cancer, endocrine adenocarcinoma, adrenal cancer, pituitary gland cancer, skin cancer, soft tissue cancer, vascular cancer, brain cancer, neural cancer, eye cancer, meningeal cancer, oropharyngeal cancer, hypopharynx cancer, cervical cancer, and uterine cancer, glioblastoma, medulloblastoma, astrocytoma, glioma, meningioma, gastrinoma, neuroblastoma, melanoma, acute myeloid leukemia, myelodysplastic syndrome and sarcoma.
In some embodiments, administering to the subject an effective amount of the isolated antigen-binding polypeptide that binds to CD47 or the antigen-binding portion thereof described herein promotes macrophage phagocytosis in the subject.

Although the foregoing disclosure has been described in some detail by providing illustrations and examples for the purpose of clear understanding, it will be apparent to those of ordinary skill in the art in light of the teachings of the present disclosure that certain changes and modifications can be made to the present disclosure without departing from the spirit or scope of the appended claims. The following examples are provided for illustrative purposes only and are not intended to be limiting. Those skilled in the art will readily identify a variety of noncritical parameters that may be changed or modified to produce substantially similar results. The positive control Hu5F9 used in the specific examples herein is a humanized antibody and has the same sequence as the antibody "5F9" of U.S. Pat. US2015/0183874A1.

Unless otherwise stated, the practice of the present disclosure uses techniques in the conventional molecular biology, cell biology, biochemistry and immunology well known in the art and described in the following: e.g., Methods in Molecular Biology, Humana Press; Molecular Cloning: A Laboratory Manual, 2nd Edition (Sambrook et al., 1989); Current Protocols in Immunology (J. E. Coligan et al., 1991); Immunobiology (C. A. Janeway and P. Travers, 1997); Antibodies (P. Finch, 1997); Antibodies: a practical approach (Ed. D. Catty, IRL Press, 1988-1989); Monoclonal antibodies: a practical approach (Ed. P. Shepherd and C. Dean, Oxford University Press, 2000); Phage display: a laboratory manual (C. Barbas III et al., Cold Spring Harbor Laboratory Press, 2001); and Using antibodies: a laboratory manual (E. Harlow and D. Lane, Cold Spring Harbor Laboratory Press, 1999).

## Example 1: Production of Anti-CD47 Monoclonal Antibodies

**Immunization of mice with CD47 protein:**

To produce antibodies against CD47, Balb/c mice were immunized subcutaneously with equal volumes of Freund's complete adjuvant (first immunization) or Freund's incomplete adjuvant (booster immunization) emulsified recombinant hCD47-His Tag protein (ACRObiosystems, Cat. No. CD7-H5227) (50 $\mu$g/Balb/c mice) or hCD47-mFc (ACRObiosystems, Cat. No. CD7-H52A5) every 2 weeks over 6 weeks, making a total of 3 immunizations. In the fourth immunization, each mouse was injected intramuscularly with 20 $\mu$g of hCD47-mFc (ACRObiosystems, Cat. No. CD7-H52A5) protein (in aqueous adjuvant). Three days prior to cell fusion, mice were injected intravenously with an antigen without an adjuvant to boost the immunization. Spleen cells ($1 \times 10^8$) from the immunized mice were fused with SP2/0 myeloma cells ($1.5 \times 10^7$) using polyethylene glycol 1500 (Roche, Cat. No. 10783641001). After fusion, the cells were distributed into the wells of a 96-well plate at 0.1 mL/well and incubated in an incubator at 37 °C with 5% $CO_2$. On day 1, the cells were fed by adding an additional 0.1 mL of HAT medium containing serum and supplemented with $2\times$ methotrexate to each well. On days 3 and 7, 0.1 mL of the medium from each well was replaced with 0.1 mL of fresh HAT medium. Screening is generally performed between day 9 and day 14, and screening targeted at the antibody test culture supernatants that reacted with hCD47-His Tag (ACRObiosystems, Cat. No. CD7-H5227) was performed by ELISA for positive wells.

Cloning of hybridoma cells was performed by limiting dilution. The selected positive mixed clones were subjected to limiting dilution. One strain of positive mixed clones was plated in two 96-well plates (at 100 cells/plate, 200 cells/plate). The hybridoma cells were cultured in RPMI 1640 (Hyclone, Cat. No. SH30809.01) containing 10% fetal bovine serum. After 10 days, subclones after limiting dilution were also subjected to ELISA assay to test the culture supernatants, and positive subclones were picked. The cloned hybridoma cells were preserved and used for extraction of cDNA.

**cDNA extraction and variable region sequence acquisition of anti-human CD47 antibodies:**

Total RNA was isolated from the selected hCD47 antibody-producing hybridoma cell line using an RNA extraction kit (Takara, Cat. No. 9767), and first-strand cDNA was synthesized using a reverse transcription kit (Thermo Fisher, Cat. No. K1652) according to the manufacturer's instructions with the total RNA as a template. Then the synthesized cDNA was used to amplify antibody-related sequences *in vitro* by PCR using degenerate mouse IgG primers.

The PCR amplification products were electrophoretically separated in a 1% agarose/Tris-acetate gel. A DNA fragment of the expected size (about 450 bp for heavy and light chains) was removed from the gel and purified. 3 $\mu$L of the purified PCR product was cloned into pMD-19T vector (Takara, Cat. No. 6013) and transformed into DH5$\alpha$ chemically competent *Escherichia coli* (Takara, Cat. No. 9057). In each ligation reaction, 10 positive clones were randomly selected and DNA sequencing was performed using the M13 forward primer.

The heavy chain variable region (VH) sequences and light chain variable region (VL) sequences of five murine monoclonal antibodies (2B2, 2H8, 3F10, 16E5 and 14A9 antibodies) were obtained from the corresponding hybridoma clones (2B2, 2H8, 3F10, 16E5 and 14A9 by amplification and sequencing, and are shown as follow:

| Name of antibody | VH (SEQ ID NO: ) | | VL (SEQ ID NO: ) | |
|---|---|---|---|---|
| | Amino acid sequence | Nucleotide sequence | Amino acid sequence | Nucleotide sequence |
| 2H8 | 33 | 189 | 34 | 190 |
| 16E5 | 35 | 191 | 36 | 192 |
| 2B2 | 37 | 193 | 38 | 194 |
| 3F10 | 39 | 195 | 40 | 196 |
| 14A9 | 41 | 197 | 42 | 198 |

**Construction and expression of chimeric antibodies:**

Through the chemical synthesis of genes of mouse antibody variable regions, the VL region of each mouse antibody was linked to the human κ chain constant region to construct a chimeric light chain, and the mouse VH region was linked to the IgG4 (with EU number S228P) constant region to construct a chimeric heavy chain. Expression plasmids for the chimeric light chain and the chimeric heavy chain were constructed by conventional genetic engineering means. CHO cells (a 200 mL system at $10^6$ cells/mL) were transfected with 100 μg of each type of the chimeric heavy chain and the chimeric light chain expression plasmids and cultured for 6 days. Then the chimeric antibody in the supernatant was purified using a protein A column.

Five chimeric antibodies were obtained by construction and expression, and their full-length sequences of the chimeric heavy chains and the chimeric light chains and the nucleic acids encoding same are as follows: Xi2H8 (chimeric heavy chain and nucleic acid encoding same: SEQ ID NOs: 89-90; chimeric light chain and nucleic acid encoding same: SEQ ID NOs: 91-92), Xi2B2 (chimeric heavy chain and nucleic acid encoding same: SEQ ID NOs: 97-98; chimeric light chain and nucleic acid encoding same: SEQ ID NOs: 99-100), Xi3F10 (chimeric heavy chain and nucleic acid encoding same: SEQ ID NOs: 101-102; chimeric light chain and nucleic acid encoding same: SEQ ID NOs: 103-104), Xi16E5 (chimeric heavy chain and nucleic acid encoding same: SEQ ID NOs: 93-94; chimeric light chain and nucleic acid encoding same: SEQ ID NOs: 95-96), and Xi14A9 (chimeric heavy chain and nucleic acid encoding same: SEQ ID NOs: 105-106; chimeric light chain and nucleic acid encoding same: SEQ ID NOs: 107-108).

**Humanization design of antibodies:**

Human murine chimeric antibodies of 3F10, 14A9 and 16E5 were humanized by CDR grafting (see, e.g., U.S. Pat. No. 5,225,539). Murine antibodies 3F10, 14A9 and 16E5 were homologously modeled using MOE (Molecular Operating Environment) to produce protein structural models of the Fv domains. The VH and VL amino acid sequences of murine 3F10, 14A9 and 16E5 antibodies were input into the international ImMunoGeneTics information system-related tool website (http://www.imgt.org/3Dstructure-DB/cgi/DomainGapAlign.cgi), from which human germline antibody sequences highly homologous to the variable regions of the mouse 3F10, 14A9 and 16E5 antibodies were selected. Complementarity determining regions (CDRs) in the VH and VL of the murine 3F10, 14A9 and 16E5 antibodies were grafted to template human antibodies. For 3F10, the selected template human VH was a combination of IGHV1-69*01 and IGHJ4*01, and the selected template human VL was a combination of IGKV1-39*01 and IGKJ2*01. For 14A9, the selected template human VH was a combination of IGHV1-46*01 and IGHJ4*01, and the selected template human VL was a combination of IGKV1-39*01 and IGKJ2*01. For 16E5, the selected template human VH was a combination of IGHV1-18*01 and IGHJ4*01, and the selected template human VL was a combination of IGKV1-39*01 and IGKJ2*01.

The CDR amino acid sequences of the above template human antibodies were substituted with the CDRs of the hybridoma (mouse) 3F10, 14A9 and 16E5 antibodies. The essential amino acid sequences from the VH and VL of the mouse 3F10, 14A9 and 16E5 antibodies were grafted to the VH and VL frameworks of the above template human germline antibodies to obtain functional humanized antibodies. For the VH and VL of the 3F10, 14A9 and 16E5 antibodies, several sites of framework amino acids of the template human antibodies described above were reverted to the corresponding amino acid sequences in the mouse 3F10, 14A9 and 16E5 antibodies. Mutation was performed in some CDR regions.

For the heavy chain variable region of the humanized 3F10 antibody, the selection and collocation of types of mutation were from the following sites: position 73 where the amino acid was mutated from Asp (D) to Glu (E), position 74 where the amino acid was mutated from Glu (E) to Ile (I), and position 77 where the amino acid was mutated from Ser (S) to Asn (N); and for the light chain variable region of the humanized 3F10 antibody, the selection and collocation of types of mutation were from the following sites: position 48 where the amino acid was mutated from Leu (L) to Trp (W), position 71 where the amino acid was mutated from Asp (D) to Ser (S), position 72 where the amino acid was mutated from Phe (F) to Tyr (Y), and position 94 where the amino acid was mutated from Asp (D) to Glu (E). For the heavy chain variable region of the humanized 14A9 antibody, the selection and collocation of types of mutation were from the following sites: position 24 where the amino acid was mutated from Ala (A) to Val (V), position 72 where the amino acid was mutated from Arg (R) to Gly (G), position 77 where the amino acid was mutated from Ser (S) to Asn (N), and position 97 where the amino acid was mutated from Ala (A) to Thr (T); and for the light chain variable region of the humanized 14A9 antibody, the selection and collocation of types of mutation were from the following sites: position 63 where the amino acid was mutated from Ser (S) to Ile (I), and position 68 where the amino acid was mutated from Gly (G) to Ser (S). For the heavy chain variable region of the humanized 16E5 antibody, the selection and collocation of types of mutation were from the following sites: position 46 where the amino acid was mutated from Glu (E) to Lys (K), position 63 where the amino acid was mutated from Lys (K) to Glu (E), position 72 where the amino acid was mutated from Thr (T) to Leu (L), position 77 where the amino acid was mutated from Ser (S) to Arg (R), and position 97 where the amino acid was mutated from Ala (A) to Thr (T); and for the light chain variable region of the humanized 16E5 antibody, the selection and collocation of types of mutation were from the following sites: position 2 where the amino acid was mutated from Ile (I) to Ala (A), and position 33 where the amino acid was mutated from Asn (N) to Gln (Q). The mutation sites described herein are numbered by counting from the first amino acid of the variable region in order.

The sequences of the humanized 3F10, 14A9 and 16E5 antibodies and the nucleic acids encoding same were as follows: hz3F10-1.1 (heavy chain and nucleic acids encoding same: SEQ ID NOs. 109-110; light chain and nucleic acids encoding same: SEQ ID NOs. 111-112), hz3F10-2.1 (heavy chain and nucleic acids encoding same: SEQ ID NOs. 113-114; light chain and nucleic acids encoding same: SEQ ID NOs. 115-116), hz3F10-3.1 (heavy chain and nucleic acids encoding same: SEQ ID NOs. 117-118; light chain and nucleic acids encoding same: SEQ ID NOs. 119-120), hz3F10-4.1 (heavy chain and nucleic acids encoding same: SEQ ID NOs. 121-122; light chain and nucleic acids encoding same: SEQ ID NOs. 123-124), hz3F10-5.1 (heavy chain and nucleic acids encoding same: SEQ ID NOs. 125-126; light chain and nucleic acids encoding same: SEQ ID NOs. 127-128), hz3F10-6.1 (heavy chain and nucleic acids encoding same: SEQ ID NOs. 129-130; light chain and nucleic acids encoding same: SEQ ID NOs.131-132), hz3F10-1.2 (heavy chain and nucleic acids encoding same: SEQ ID NOs.133-134; light chain and nucleic acids encoding same: SEQ ID NOs. 135-136), hz3F10-2.2 (heavy chain and nucleic acids encoding same: SEQ ID NOs. 137-138; light chain and nucleic acids encoding same: SEQ ID NOs. 139-140), hz3F10-3.2 (heavy chain and nucleic acids encoding same: SEQ ID NOs. 141-142; light chain and nucleic acids encoding same: SEQ ID NOs. 143-144), hz3F10-4.2 (heavy chain and nucleic acids encoding same: SEQ ID NOs. 145-146; light chain and nucleic acids encoding same: SEQ ID NOs.147-148), hz3F10-5.2 (heavy chain and nucleic acids encoding same: SEQ ID NOs.149-150; light chain and nucleic acids encoding same: SEQ ID NOs.151-152), hz3F10-6.2 (heavy chain and nucleic acids encoding same: SEQ ID NOs.153-154; light chain and nucleic acids encoding same: SEQ ID NOs.155-156), hz16E5-1.1 (heavy chain and nucleic acids encoding same: SEQ ID NOs.157-158; light chain and nucleic acids encoding same: SEQ ID NOs.159-160), hz16E5-1.3 (heavy chain and nucleic acids encoding same: SEQ ID NOs.161-162; light chain and nucleic acids encoding same: SEQ ID NOs.163-164), hz16E5-1.2 (heavy chain and nucleic acids encoding same: SEQ ID NOs.165-166; light chain and nucleic acids encoding same: SEQ ID NOs.167-168), hz16E5-3.2 (heavy chain and nucleic acids encoding same: SEQ ID NOs. 169-170; light chain and nucleic acids encoding same: SEQ ID NOs. 171-172), hz16E5-3.1 (heavy chain and nucleic acids encoding same: SEQ ID NOs. 173-174; light chain and nucleic acids encoding same: SEQ ID NOs.175-176), hz16E5-3.3 (heavy chain and nucleic acids encoding same: SEQ ID NOs. 177-178; light chain and nucleic acids encoding same: SEQ ID NOs.179-180), hz14A9-2.3 (heavy chain and nucleic acids encoding same: SEQ ID NOs.181-182; light chain and nucleic acids encoding same: SEQ ID NOs. 183-184), and hz14A9-2.4 (heavy chain and nucleic acids encoding same: SEQ ID NOs. 185-186; light chain and nucleic acids encoding same: SEQ ID NOs. 187-188).

**Construction and expression of humanized 3F10, 14A9 and 16E5 antibodies:**

DNA encoding the full-length light chain and the full-length heavy chain of the humanized 3F10, 14A9 and 16E5 antibodies was synthesized and cloned into expression vectors (including, for example, pcDNA3.1 vector disclosed in CN107001463A, and pCHO1.0 vector disclosed in CN109422811A). CHO cells (a 200 mL system at $10^6$ cells/mL) were transfected with 100 $\mu$g of each type of the humanized antibody heavy chain expression plasmids and light chain expression plasmids and cultured in ExpiCHO medium (Gibco, Cat. No. A29100-01) in an incubator at 37 °C with 5% $CO_2$ for 6 days. After centrifugation, a supernatant was obtained, and the humanized antibody in the supernatant was purified using a protein A column.

**Example 2: Biacore-Based Affinity Analysis of Anti-CD47 Antibodies**

The equilibrium dissociation constants $K_D$ (in unit M) were obtained by Biacore measuring of the kinetics of the binding of human CD47 protein (human CD47 protein, His Tag; ACRObiosystems, Cat. No. CD7-H5227) and cynomolgus monkey CD47 protein (cynomolgus/rhesus macaque CD47 protein, His Tag; ACRObiosystems, Cat. No. CD7-C52H1) to anti-CD47 antibodies (chimeric or humanized); the Biacore analysis was performed at 25 °C and data were recorded at a rate of 1 Hz.

Polyclonal rabbit anti-mouse IgG (GE, BR-1008-38) was diluted with 10 mM pH 5.0 sodium acetate and immobilized onto the reference flowcell and experimental flowcell of a CM5 biosensor chip using an amine coupling kit (GE, BR10050), and about 15,000 RM was reached. At the beginning of each cycle, diluted test antibody (1.5 $\mu$g/mL) was injected onto the experimental flowcell and maintained for 1 min so that it was captured. These data show that anti-CD47 antibodies (chimeric and humanized) bound to CD47 as measured by Biacore.

Table 1: Binding affinities of anti-CD47 chimeric antibodies for human CD47 and cynomolgus monkey CD47

| Name of antibody | Biacore: equilibrium dissociation constant $K_D$ (in unit M) | |
|---|---|---|
| | Human CD47 protein | Cynomolgus monkey CD47 protein |
| Xi2B2 | 7.48E-09 | 1.20E-09 |
| Xi2H8 | 3.50E-10 | 1.25E-09 |
| Xi3F 10 | 7.67E-10 | 1.04E-09 |
| Xi16E5 | 9.06E-09 | 2.92E-09 |
| Xi14A9 | 2.35E-09 | 1.58E-09 |

Table 2: Binding affinities of anti-CD47 humanized antibodies for human CD47 and cynomolgus monkey CD47

| Name of antibody | Biacore: equilibrium dissociation constant $K_D$ (in unit M) | |
|---|---|---|
| | Human CD47 protein | Cynomolgus monkey CD47 protein |
| hz3F10-1.1 | 9.46E-10 | 1.31E-09 |
| hz3F10-2.1 | 8.36E-10 | 1.17E-09 |
| hz3F10-3.1 | 1.53E-09 | 1.92E-09 |
| hz3F10-4.1 | 1.42E-09 | 1.62E-09 |
| hz3F10-5.1 | 9.60E-10 | 1.30E-09 |
| hz3F10-6.1 | 5.92E-10 | 2.47E-09 |
| hz3F10-1.2 | 9.04E-10 | 1.33E-09 |
| hz3F10-2.2 | 7.79E-10 | 1.14E-09 |
| hz3F10-3.2 | 1.15E-09 | 1.47E-09 |
| hz3F10-4.2 | 1.34E-09 | 1.86E-09 |
| hz3F10-5.2 | 8.96E-10 | 1.40E-09 |
| hz3F10-6.2 | 7.65E-10 | 1.15E-09 |
| hz16E5-1.1 | 1.89E-08 | 4.77E-09 |
| hz16E5-3.1 | 6.28E-09 | 3.28E-09 |
| hz16E5-1.2 | 9.31E-09 | 2.26E-09 |
| hz16E5-3.2 | 1.71E-08 | 3.10E-09 |
| hz16E5-1.3 | 1.53E-08 | 2.73E-09 |
| hz16E5-3.3 | 7.13E-09 | 1.87E-09 |
| hz14A9-2.3 | 2.04E-10 | 3.07E-09 |
| hz14A9-2.4 | 8.55E-09 | 2.00E-09 |
| Hu5F9 | 8.33E-10 | 1.92E-09 |

**Example 3: ELISA-Based Binding Assay of Anti-CD47 antibodies**

An ELISA binding assay was performed based on human CD47 protein and anti-CD47 chimeric antibodies or anti-CD47 humanized antibodies. A 96-well plate (Costar, Cat. No. 9018) was coated overnight at 4 °C with 100 $\mu$L/well

coating buffer of 2 μg/mL CD47-His (human CD47 protein, His Tag; ACRObiosystems, Cat. No. CD7-H5227) in PBS. The wells were aspirated, and non-specific binding sites were blocked by adding 100 μL/well of a blocking solution (PBS with 1% (w/v) bovine serum albumin (BSA, Sigma, Cat. No. B2064-100G)) and incubating the plate at 37 °C for 1 h. After the 96-well plate was washed three times with a washing buffer (PBS with 0.01% (w/v) Tween 20 (Sigma, Cat. No. P9416-100ML)), 100 μL/well of 1:4 serial dilutions (from 30 μg/mL) of anti-CD47 antibodies (see Table 3 for chimeric antibodies, and see Table 4 for humanized antibodies) in blocking solution were added, and the plate was incubated at room temperature for 2 h. The 96-well plate was washed, and then incubated with a secondary antibody HRP-anti-Human IgG (BD, Cat. No. 555788) for 20 min in the dark. After the 96-well plate was washed, 100 μL/well of a substrate solution TMB (TIANGEN, Cat. No. PA107-01) was added, and the 96-well plate was incubated at room temperature for 10 min. 100 μL/well of a stop solution (1 M $H_2SO_4$) was added to stop the reaction. Colorimetric signals were generated and read at 450 nm using a multifunctional microplate reader (manufacturer: PerkinElmer; model: EnVision). Data were analyzed using GraphPad Prism5, and $EC_{50}$ was calculated. These data show that the anti-CD47 antibodies bound to CD47 and the humanized antibodies performed well, as measured by ELISA.

Table 3: ELISA-based binding $EC_{50}$ of anti-CD47 chimeric antibodies to human CD47 protein

| Name of antibody | $EC_{50}$ (ng/mL) |
|---|---|
| Xi2B2 | 349.5 |
| Xi2H8 | NA |
| Xi3F10 | 1106 |
| Xi16E5 | 87.89 |
| Xi14A9 | 273.4 |
| Note: NA indicates that the limit of detection was not reached. | |

Table 4: ELISA-based binding $EC_{50}$ of anti-CD47 humanized antibodies to human CD47 protein

| Name of antibody | $EC_{50}$ (ng/mL) | Name of antibody | $EC_{50}$ (ng/mL) |
|---|---|---|---|
| hz3F10-1.1 | 70.02 | hz3F10-5.2 | 105.6 |
| hz3F10-2.1 | 80.94 | hz3F10-6.2 | 129.3 |
| hz3F10-3.1 | 126.9 | hz16E5-1.1 | 306 |
| hz3F10-4.1 | 115.1 | hz16E5-3.1 | 139.7 |
| hz3F10-5.1 | 99.33 | hz16E5-1.2 | 159.5 |
| hz3F10-6.1 | 56.33 | hz16E5-3.2 | 215.1 |
| hz3F10-1.2 | 124.8 | hz16E5-1.3 | 138.1 |
| hz3F10-2.2 | 105.8 | hz16E5-3.3 | 240.9 |
| hz3F10-3.2 | 165.9 | hz14A9-2.3 | 156.8 |
| hz3F10-4.2 | 180.7 | hz14A9-2.4 | 339.3 |

**Example 4: Cell-Based Binding Assay of Anti-CD47 antibodies**

A cell binding assay of anti-CD47 antibodies (chimeric or humanized) was performed based on the binding to a CHO-K1 cell line stably expressing CD47. CHO-K1-CD47 cells (CHO-K1 cells expressing human CD47 protein) were added to each well of a 96-well plate (Eppendorf, Cat. No. C030730.119) at $1.2 \times 10^4$ cells per well and incubated with anti-CD47 antibodies (20 μg/mL, 1:4 dilution in PBS) at room temperature for 1-2 h. Then the cells were washed three times with PBS buffer, and a secondary antibody Alexa Fluor 488-labeled goat anti-human IgG (Jackson ImmunoResearch Inc, Cat. No. 109-545-088) was diluted 1:200 with PBS and added to the cells at 100 μL/well. The cells were incubated at room temperature for 30 min. The cells were washed three times with PBS buffer. A nuclear dye Hoechst (Sigma, Cat. No. B2261) was diluted with PBS buffer at a dilution ratio of 1:500 and then added to each well at 100 μL/well for staining for 10 min. The cells were washed 3 times with PBS and analyzed using a high content screening system (manufacturer: PerkinElmer; model: Operetta). The results are shown in Tables 5 and 6. These data show that anti-CD47 antibodies bound to CD47 as indicated by the cell-based binding assay.

Table 5: Cell-based binding $EC_{50}$ of anti-CD47 chimeric antibodies to CD47

| Name of antibody | $EC_{50}$ (ng/mL) |
|---|---|
| Xi2B2 | 30790 |
| Xi2H8 | NA |
| Xi3F10 | 19803 |
| Xi16E5 | 2026 |
| Xi14A9 | 540.7 |
| Note: NA indicates that the limit of detection was not reached. | |

Table 6: Cell-based binding $EC_{50}$ of anti-CD47 humanized antibodies to CD47

| Name of antibody | $EC_{50}$ (ng/mL) | Name of antibody | $EC_{50}$ (ng/mL) |
|---|---|---|---|
| hz3F10-1.1 | 875.7 | hz3F10-6.2 | 1486 |
| hz3F10-2.1 | 1068 | hz16E5-1.1 | 3866 |
| hz3F10-3.1 | 1121 | hz16E5-3.1 | 5883 |
| hz3F10-4.1 | 1162 | hz16E5-1.2 | NA |
| hz3F10-5.1 | 1480 | hz16E5-3.2 | NA |
| hz3F10-6.1 | 1184 | hz16E5-1.3 | 18845 |
| hz3F10-1.2 | 1267 | Hz16E5-3.3 | 40479 |
| hz3F10-2.2 | 1119 | hz14A9-2.3 | 498.6 |
| hz3F10-3.2 | 1195 | hz14A9-2.4 | 470.7 |
| hz3F10-4.2 | 1265 | Hu5F9 | 1076 |
| hz3F10-5.2 | 1260 | | |
| Note: NA indicates that the limit of detection was not reached. | | | |

## Example 5: Cell-Based Antibody Blocking Analysis of Anti-CD47 Chimeric Antibodies

The blocking effects of the anti-CD47 chimeric antibodies on the binding of CD47 to SIRPα were evaluated based on flow cytometry assay.

Jurkat cells (the Cell Bank of the Chinese Academy of Sciences, Cat. No. TCHU123) were added to a 96-well U-plate at $5 \times 10^5$ cells per well, and 100 μL of a 1 μg/mL dilution of CD47 ligand Human-SIRPα-mFC (Human SIRP alpha/CD172a Protein, Mouse IgG1 Fc Tag, ACRObiosystems, Cat. No. SIA-H52A8) in PBS buffer was added to each well. The plate was incubated at room temperature for 1 h. After the plate was washed twice with PBS buffer, 100 μL of anti-CD47 chimeric antibodies (Table 7) was added to each well. 4-fold dilution was performed with PBS buffer from a starting concentration of 20 μg/mL, with 8 gradients obtained. The plate was incubated at room temperature for 1-2 h. After the plate was washed 3 times with PBS buffer, Alexa Fluor 488-labeled Goat anti-mouse IgG (Jackson ImmunoResearch Inc., Cat. No. 115-545-062) was used as an assay secondary antibody, and 100 μL of the assay secondary antibody diluted at a dilution ratio of 1:200 with PBS buffer was added to each well. The plate was incubated at room temperature for 1 h and washed 3 times with PBS buffer. The blocking effects of the anti-CD47 chimeric antibodies on the binding of CD47 to SIRPα were determined using a flow cytometer (BD, model: C6). The results are shown in Table 7. The anti-CD47 chimeric antibodies can block the binding of CD47 to SIRPα. Among the antibodies, Xi3F10, Xi16E5 and Xi14A9 had better blocking effects.

Table 7: Cell-based antibody blocking analysis of anti-CD47 chimeric antibodies

| Name of antibody | Xi2B2 | Xi2H8 | Xi3F10 | Xi16E5 | Xi14A9 |
|---|---|---|---|---|---|
| $IC_{50}$ (ng/mL) | NA | 26966 | 358.5 | 4861 | 1283 |
| Note: NA indicates that the limit of detection was not reached. | | | | | |

**Example 6: ELISA-Based Antibody Blocking Analysis of Anti-CD47 Antibodies**

The blocking effects of the anti-CD47 antibodies on the binding of CD47 to SIRPα were evaluated based on ELISA assay.

In a microplate 96-well plate (Costar, Cat. No. 9018), with CD47-His (human CD47 protein, His Tag; ACRObiosystems, Cat. No. CD7-H5227) as a capture antigen, 100 μL coating buffer PBS containing 2 μg/mL capture antigen was added to each well. The plate was let stand at 2-8 °C overnight. The wells were aspirated to remove the coating buffer and blocked with 100 μL/well of a blocking solution (PBS with 1% (w/v) bovine serum albumin (BSA, Sigma, Cat. No. B2064-100G)) by incubation at 37 °C for 1 h. The 96-well plate was washed with a washing buffer (PBS with 0.01% (w/v) Tween 20 (Sigma, Cat. No. P9416-100ML)). Then 100 μL/well of 1 μg/mL CD47 ligand Human-SIRPα-mFC (Human SIRP alpha/CD172a Protein, Mouse IgG1 Fc Tag, ACRObiosystems, Cat. No. SIA-H52A8) was added, and the plate was incubated at 25±2 °C for 1.5 h. After the 96-well plate was washed with PBS washing buffer, anti-CD47 chimeric antibodies or anti-CD47 humanized antibodies (see Table 8 for chimeric antibodies, and see Table 9 for humanized antibodies) were added. 4-fold dilution was performed with PBS buffer from a starting concentration of 20 μg/mL, with 8 gradients obtained. The plate was incubated at room temperature for 1.5 h. After the plate was washed with PBS buffer, 100 μL/well of HRP-labeled goat anti-mouse IgG (BD, Cat. No. 554002) diluted 1:2000 with PBS buffer was added, and the plate was incubated at 25±2 °C for 1 h. After the 96-well plate was washed with PBS buffer, 100 μL/well of a substrate solution TMB (TIANGEN, Cat. No. PA107-01) was added, and the plate was incubated at room temperature for color development for 10 min. 100 μL/well of 1 M $H_2SO_4$ was added to stop the reaction. Colorimetric signals were generated and read at 450 nm using a multifunctional microplate reader (manufacturer: PerkinElmer; model: EnVision). Data were analyzed using GraphPad Prism5, and $EC_{50}$ was calculated. The results are shown in Table 8 and Table 9. The chimeric antibodies (Xi3F10, Xi16E5, Xi14A9) and humanized antibodies of 14A9, 16E5 and 3F10 had good blocking effects as indicated by the ELISA-based assay.

Table 8: ELISA-based antibody blocking analysis of anti-CD47 chimeric antibodies

| Name of antibody | $IC_{50}$ (ng/mL) |
|---|---|
| Xi2B2 | NA |
| Xi2H8 | 4.682 |
| Xi3F10 | 7910 |
| Xi16E5 | 946.1 |
| Xi14A9 | 444.4 |
| Note: NA indicates that the limit of detection was not reached. | |

Table 9: ELISA-based antibody blocking analysis of anti-CD47 humanized antibodies

| Name of antibody | $IC_{50}$ (ng/mL) | Name of antibody | $IC_{50}$ (ng/mL) |
|---|---|---|---|
| hz3F10-1.1 | 1534 | hz3F10-6.2 | 1839 |
| hz3F10-2.1 | 1612 | hz16E5-1.1 | NA |
| hz3F10-3.1 | 1678 | hz16E5-3.1 | 15246 |
| hz3F10-4.1 | 1952 | hz16E5-1.2 | 2939 |
| hz3F10-5.1 | 1701 | hz16E5-3.2 | 2051 |
| hz3F10-6.1 | 1768 | hz16E5-1.3 | 2323 |
| hz3F10-1.2 | 1689 | hz16E5-3.3 | 1998 |
| hz3F10-2.2 | 1725 | hz14A9-2.3 | 703.2 |
| hz3F10-3.2 | 1741 | hz14A9-2.4 | 621.1 |
| hz3F10-4.2 | 1738 | Hu5F9 | 2398 |
| hz3F10-5.2 | 1824 | | |
| Note: NA indicates that the limit of detection was not reached. | | | |

**Example 7: Analysis of Anti-CD47 Antibodies Based on Promotion of Macrophage Phagocytosis**

The ability of the anti-CD47 antibodies to promote the phagocytosis of tumor cells by macrophages having SIRP$\alpha$ on the cell surface was determined based on flow cytometry.

Monocytes were isolated from human peripheral blood mononuclear cells (PBMC, Sailybio, Cat. No. 190056) using a monocyte isolation kit (STEMCELL, Cat. No. 19058), plated into a 24-well plate (Greiner bio-one, Cat. No. 662-160) at $5 \times 10^5$ cells per well, and induced under 100 ng/mL stimulating factor M-CSF (R&D Systems, Cat. No. 216-MC-010) for 7 days to form macrophages. $1 \times 10^6$ target cells HL60 (Shanghai Institute of Biochemistry and Cell Biology, Chinese Academy of Sciences, Cat. No. TCHu23) were suspended in 5 $\mu$mol/L CFSE (BD, Cat. No. 565082) fluorescence dye (diluted with PBS buffer to the desired concentration), incubated in a water bath at 37 °C in the dark for 15 min, and washed with PBS buffer to prepare CFSE-labeled HL60 cells, which was then incubated with each of 10 $\mu$g/mL and 1 $\mu$g/mL anti-CD47 antibodies (see Table 10 for chimeric antibodies, and see Table 11 for humanized antibodies) in an incubator at 37 °C with 5% $CO_2$ for 1-2 h. The cultured HL60 cells were washed 3 times with PBS buffer and added to the macrophages. The cells were incubated in an incubator at 37 °C with 5% $CO_2$ for 2-4 h. After the cells were washed 3 times with PBS buffer, a mixture of 260 $\mu$L of PBS, 20 $\mu$L of anti-APC-CD14 (APC Mouse Anti-Human CD14, BD, Cat. No. 555399) and 20 $\mu$L of PE-CD11b (PE Mouse Anti-Human CDI11b/Mac-1, BD, Cat. No. 555388) antibody was added to each well. The cells were incubated at 4 °C in the dark for 30 min, washed 3 times with PBS buffer, and digested with trypsin (Gibco, Cat. No. 12604-013). The rate of phagocytosis (%) by the macrophages was determined using a flow cytometer (BD, model: C6).

The effects of the anti-CD47 chimeric antibodies and anti-CD47 humanized antibodies on macrophage phagocytosis are shown in Tables 10 and 11. Among the antibodies, the chimeric antibodies Xi3F10, Xi16E5 and Xi14A9 and the anti-CD47 humanized antibodies had good promoting effects on phagocytosis, and the performance of the humanized antibodies hz14A9-2.3 and hz14A9-2.4 was more excellent.

Table 10: Analysis of anti-CD47 chimeric antibodies based on promotion of macrophage phagocytosis (rate of phagocytosis, %)

| Name of antibody | 10 $\mu$g/mL | 1 $\mu$g/mL |
|---|---|---|
| Xi2B2 | 4.1 | 7.3 |
| Xi2H8 | 3.5 | 7.1 |
| Xi3F10 | 59.8 | 65.7 |
| Xi16E5 | 28.8 | 11.5 |
| Xi14A9 | 44.67 | 54.17 |

Table 11: Analysis of anti-CD47 humanized antibodies based on promotion of macrophage phagocytosis (rate of phagocytosis, %)

| Name of antibody | 10 $\mu$g/mL | 1 $\mu$g/mL | Name of antibody | 10 $\mu$g/mL | 1 $\mu$g/mL |
|---|---|---|---|---|---|
| hz3F10-1.1 | 43.10 | 41.25 | hz3F10-6.2 | 44.19 | 49.32 |
| hz3F10-2.1 | 55.23 | 47.24 | hz16E5-1.1 | 4.02 | 14.79 |
| hz3F10-3.1 | 58.72 | 50.60 | hz16E5-3.1 | 24.19 | 6.37 |
| hz3F10-4.1 | 58.26 | 50.26 | hz16E5-1.2 | 17.83 | 5.1 |
| hz3F10-5.1 | 58.46 | 50.45 | hz16E5-3.2 | 2.03 | 3.02 |
| hz3F10-6.1 | 60.91 | 47.57 | hz16E5-1.3 | 4.33 | 3.03 |
| hz3F10-1.2 | 38.86 | 55.52 | hz16E5-3.3 | 4.7 | 11.62 |
| hz3F10-2.2 | 34.72 | 44.19 | hz14A9-2.3 | 74.93 | 71.48 |
| hz3F10-3.2 | 37.09 | 47.04 | hz14A9-2.4 | 81.66 | 81.65 |
| hz3F10-4.2 | 31.67 | 46.24 | Hu5F9 | 72.35 | 67.5 |
| hz3F10-5.2 | 29.6 | 47.06 | | | |

**Example 8: Effects of Anti-CD47 Antibodies on CD4+ T Cells**

The effects of the anti-CD47 antibodies on CD4+ T cells were determined based on flow cytometry assay. CD4+ T cells were isolated from human peripheral blood mononuclear cells (PBMC, Sailybio, Cat. No. 190056) using a T cell isolation kit (Miltenyi Biotech, Cat. No. 130-094-131) and plated into a 24-well plate (Greiner bio-one, Cat. No. 662106) at 5 $\times$ 10$^5$ cells per well. Anti-CD47 antibodies (see Table 12 for chimeric antibodies, and see Table 13 for humanized antibodies) were separately added at the same time so that the final concentrations were 10 µg/mL and 1 µg/mL. 1640 complete medium was added, and the cells were incubated in an incubator at 37 °C with 5% $CO_2$ for 20 h and stained using an apoptosis kit (STEMCELL, Cat. No. 556547) at room temperature for 15 min. The rate of cell apoptosis (%) was determined using a flow cytometer (BD, model: C6). The rate of apoptosis was calculated based on the sum of the early apoptosis and later apoptosis. The results show that the anti-CD47 antibodies (see Table 12 for chimeric antibodies, and see Table 13 for humanized antibodies) had no significant effect on T cell apoptosis.

Table 12: Effects of anti-CD47 chimeric antibodies on CD4+ T cells (rate of apoptosis %)

| Name of antibody | 10 µg/mL | 1 µg/mL |
|---|---|---|
| Xi2B2 | 10.3 | 12.1 |
| Xi2H8 | 10.5 | 12.1 |
| Xi3F10 | 13.6 | 12.6 |
| Xi16E5 | 23.1 | 0.4 |
| Xi14A9 | 1.34 | 1.42 |

Table 13: Effects of anti-CD47 humanized antibodies on CD4+ T cells (rate of apoptosis %)

| Name of antibody | 10 µg/mL | 1 µg/mL | Name of antibody | 10 µg/mL | 1 µg/mL |
|---|---|---|---|---|---|
| hz3F10-1.1 | 1.94 | 1.82 | hz3F10-6.2 | 1.68 | 2.18 |
| hz3F10-2.1 | 1.7 | 1.38 | hz16E5-1.1 | 1.88 | 1.62 |
| hz3F10-3.1 | 1.88 | 2 | hz16E5-3.1 | 2.04 | 1.8 |
| hz3F10-4.1 | 1.86 | 1.94 | hz16E5-1.2 | 1.66 | 1.08 |
| hz3F10-5.1 | 1.74 | 1.94 | hz16E5-3.2 | 1.8 | 1.52 |
| hz3F10-6.1 | 2.12 | 1.34 | hz16E5-1.3 | 1.28 | 1.38 |
| hz3F10-1.2 | 1.74 | 1.7 | hz16E5-3.3 | 1.54 | 1.54 |
| hz3F10-2.2 | 1.82 | 1.94 | hz14A9-2.3 | 1.56 | 1.59 |
| hz3F10-3.2 | 1.84 | 1.66 | hz14A9-2.4 | 1.89 | 1.38 |
| hz3F10-4.2 | 1.84 | 1.78 | Hu5F9 | 1.35 | 1.58 |
| hz3F10-5.2 | 1.94 | 1.78 | IgG4 | 1.72 | 1.81 |

**Example 9: Thermostability Assay of Anti-CD47 Antibodies Based on Nano DSF**

The high-throughput protein stability analyzer Prometheus NT.48 is an instrument for obtaining various types of data such as protein molecular structure stability, aggregation stability, and colloid dispersion stability. The melting temperatures (Tm) and the aggregation temperatures (Tagg) of the anti-CD47 chimeric antibodies and the anti-CD47 humanized antibodies were measured using this instrument. The results are shown in Tables 14 and 15, and show that the anti-CD47 antibodies have good thermostability.

Table 14: Thermostability of anti-CD47 chimeric antibodies

| Name of antibody | Tm (°C) | Tagg (°C) |
|---|---|---|
| Xi2B2 | 62.99 | 61.34 |
| Xi2H8 | 69.77 | 68.57 |
| Xi3F 10 | 70.51 | 70.59 |

(continued)

| Name of antibody | Tm (°C) | Tagg (°C) |
|---|---|---|
| Xi16E5 | 69.89 | 73.39 |
| Xi14A9 | 66.73 | 75.75 |

Table 15: Thermostability of anti-CD47 humanized antibodies

| Name of antibody | Tm (°C) | Tagg (°C) | Name of antibody | Tm (°C) | Tagg (°C) |
|---|---|---|---|---|---|
| hz3F10-1.1 | 68.44 | 72.68 | hz14A9-2.4 | 68.3 | 68.3 |
| hz3F10-2.1 | 68.44 | 72.68 | hz3F10-4.2 | 68.48 | 61.13 |
| hz3F10-3.1 | 68.15 | 61.53 | hz3F10-5.2 | 68.69 | 62.67 |
| hz3F10-4.1 | 68.35 | 62.54 | hz3F10-6.2 | 68.64 | 61.91 |
| hz3F10-5.1 | 68.05 | 62.12 | hz16E5-1.1 | 68.21 | 61.87 |
| hz3F10-6.1 | 68.6 | 68.6 | hz16E5-3.1 | 69.20 | 61.84 |
| hz3F10-1.2 | 68.71 | 61.92 | hz16E5-1.2 | 69.54 | 61.90 |
| hz3F10-2.2 | 68.92 | 62.40 | hz16E5-3.2 | 69.10 | 61.44 |
| hz3F10-3.2 | 68.30 | 61.80 | hz16E5-1.3 | 68.90 | 73.77 |
| hz14A9-2.3 | 68.10 | 73.42 | hz16E5-3.3 | 68.72 | 73.81 |

## Example 10: Effects of Anti-CD47 Antibodies on Red Cell Agglutination

Some published studies have shown that the surface of red cells contains a certain amount of CD47 protein, which will cause some anti-CD47 antibodies to bind to red cells after they are added and thus may cause red cell agglutination. Thus, the effects of the anti-CD47 antibodies on red cell agglutination were tested.

Anticoagulated blood was collected and well mixed with an equal amount of normal saline. The mixture was centrifuged at 2000 rpm/min for 5 min, and the supernatant was removed. The red cells were washed 3 times with calcium and magnesium-free Hank's balanced salt solution (Thermo Fisher Scientific, Cat. No. 14175-095). In the first two washings, the mixture was centrifuged at 2000 rpm/min for 5 min, and in the last washing, the mixture was centrifuged at 2000 rpm/min for 10 min, and the supernatant was removed. The red cells were diluted 10-fold with PBS buffer to form a 10% red cell suspension, which was added to a 96-well U-plate (eppendorf, Cat. No. 003073119) at 100 µL/well. Different concentrations of anti-CD47 antibodies were added at 100 µL/well. The antibodies were diluted 3-fold with PBS buffer from a starting concentration of 10 µg/mL, with 8 concentration gradients obtained. The plate was incubated at 37 °C for 2-6 h. The red cells were observed and photographed. Where agglutination occurred, red cells completely clumped together, settled to the bottom, and spread as a piece of net; where agglutination did not occur, red cells settled to the bottom of the well and were present as dots.

The effects of the anti-CD47 chimeric antibodies on red cell agglutination are shown in FIG. 1. The effects of the anti-CD47 humanized antibodies on red cell agglutination are shown in FIGs. 2 and 3. The chimeric antibodies Xi3F10, Xi16E5 and Hu5F9 all caused blood agglutination. Xi3F10 caused blood agglutination from 1111.1 ng/mL, and Xi16E5 and Hu5F9 caused blood agglutination from 123.46 ng/mL. The chimeric antibodies Xi2B2, Xi2H8 and Xi14A9 all did not cause blood agglutination at all the concentrations tested. The humanized antibodies hz3F10-3.1, hz3F10-4.1, hz3F10-3.2 and hz3F10-4.2 caused blood agglutination from 3.3 µg/mL, and hz16E5-1.1, hz16E5-3.1, hz16E5-1.2, hz16E5-3.2, hz16E5-1.3 and hz16E5-3.3 caused blood agglutination from 3.3 µg/mL. The other humanized antibodies did not cause blood agglutination at all the concentrations tested.

## Example 11: Analysis of Binding of Anti-CD47 Antibodies to Red Cells

Based on the fact that a certain amount of CD47 on the red cell surface may bind to some anti-CD47 antibodies, the binding of the anti-CD47 antibodies to red cells was assessed.

Anticoagulated blood was collected and well mixed with an equal amount of normal saline. The mixture was centrifuged at 2000 rpm/min for 5 min, and the supernatant was removed. The red cells were washed 3 times with calcium and magnesium-free Hank's balanced salt solution (Thermo Fisher Scientific, Cat. No. 14175-095). In the first two washings, the mixture was centrifuged at 2000 rpm/min for 5 min, and in the last washing, the mixture was centrifuged at 2000 rpm/min for 10 min, and the supernatant was removed. The isolated red cells were fixed with 4% polyoxymethylene (Sangon Biotech (Shanghai) Co., Ltd., Cat. No. E672002-0500) overnight, incubated with 10 $\mu$g/mL or 1 $\mu$g/mL anti-CD47 antibodies at room temperature the next day for 1-2 h, washed with PBS buffer, incubated with a secondary antibody Alexa Fluor 488-labeled goat anti-human IgG (Jackson ImmunoResearch Inc, Cat. No. 109-545-088) that was diluted 1:200 with PBS buffer at room temperature for 1 h, and washed with PBS buffer, and the binding of the red cells to the anti-CD47 chimeric antibodies (see Table 16) or humanized antibodies (see Table 17) was assessed using a flow cytometer (BD, model: C6) in terms of the mean fluorescent intensity (MFI).

Table 16: Analysis of the binding of Anti-CD47 chimeric antibodies to red cells (MFI)

| Name of antibody | 10 $\mu$g/mL | 1 $\mu$g/mL |
|---|---|---|
| Xi2B2 | 608 | 656 |
| Xi2H8 | 5303 | 952 |
| Xi3F10 | 61821 | 36114 |
| Xi16E5 | 3318 | 1309 |
| Xi14A9 | 32831 | 26477 |

Table 17: Analysis of the binding of Anti-CD47 humanized antibodies to red cells (MFI)

| Name of antibody | 10 $\mu$g/mL | 1 $\mu$g/mL | Name of antibody | 10 $\mu$g/mL | 1 $\mu$g/mL |
|---|---|---|---|---|---|
| hz3F10-1.1 | 50,463 | 4,449 | hz3F10-6.2 | 55,886 | 3,435 |
| hz3F10-2.1 | 53,218 | 8,454 | hz16E5-1.1 | 6,461 | 945 |
| hz3F10-3.1 | 41,362 | 6,703 | hz16E5-3.1 | 5,145 | 945 |
| hz3F10-4.1 | 41,900 | 5,976 | hz16E5-1.2 | 4,729 | 913 |
| hz3F10-5.1 | 50,634 | 7,403 | hz16E5-3.2 | 4,204 | 860 |
| hz3F10-6.1 | 53,048 | 7,583 | hz16E5-1.3 | 4,885 | 894 |
| hz3F10-1.2 | 56,720 | 6,247 | hz16E5-3.3 | 3,874 | 848 |
| hz3F10-2.2 | 56,086 | 5,612 | hz14A9-2.3 | 10,979 | 2,506 |
| hz3F10-3.2 | 45,305 | 5,096 | hz14A9-2.4 | 14,954 | 5,237 |
| hz3F10-4.2 | 45,616 | 4,814 | IgG4 | 484 | 449 |
| hz3F10-5.2 | 53,329 | 5,134 | | | |

**Example 12. Pharmacodynamic Activity of Anti-CD47 Antibodies in Mouse Hematological Tumor Model of Lymphoma Raji Cells**

A mouse hematological tumor model was established using hematological tumor Raji cells. The anti-CD47 antibodies were applied to the model, and the pharmacodynamic activity of the antibodies was evaluated.

A suspension of Raji cells in PBS buffer was prepared at a concentration of $5 \times 10^6$ cells/mL, and was injected to female NOD/SCID mice via the tail vein at 0.1 mL/mouse. After 7 days, the mice were randomized into 4 groups of 10, which were group 1: IgG4 group; group 2: Hu5F9 group; group 3: hz3F10-6.1; and group 4: hz14A9-2.3. The administration was performed by intraperitoneal injection in doses of 10 mg/kg for 21 consecutive days. The survival of the animals was observed.

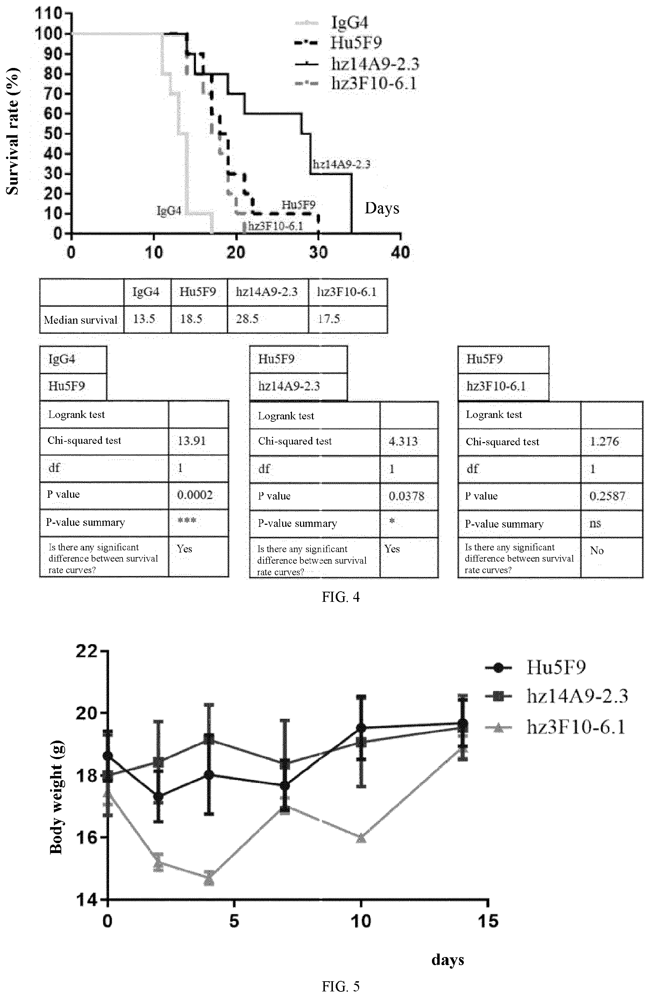

As shown in FIG. 4, the anti-CD47 antibodies hz14A9-2.3 and hz3F10-6.1 showed anti-tumor activity in the Raji hematological tumor model. The survival rates of the mice show that: all the animals in the IgG4 group died on day 17, with a median survival of 13.5 days; all the animals in the Hu5F9 group died on day 30, with a median survival of 18.5 days; all the animals in the hz14A9-2.3 group died on day 34, with a median survival of 28.5 days; all the animals in the hz3F10-6.1 group died on day 21, with a median survival of 17.5 days. These data indicate that the antibody hz14A9-2.3 has better efficacy in prolonging the survival of the animals than the other groups, and the antibodies hz3F10-6.1 and Hu5F9 had basically equivalent effects.

**Example 13. Toxicity Assay of Anti-CD47 Antibodies in Single Administration**

Since CD47 is expressed on red cells and plays a role in clearing aged red cells, the toxic effects of the anti-CD47 antibodies in B-hCD47 mice were compared.

B-hCD47 mice were randomized into 3 groups: an Hu5F9 group of 3 mice, an hz14A9-2.3 group of 4 mice, and an hz3F10-6.1 group of 3 mice. The B-hCD47 mice were injected with Hu5F9 antibody, hz14A9-2.3 antibody or hz3F10-6.1 antibody via the tail vein in a single dose of 10 mg/kg, and changes in red blood cell count (RBC), Hemoglobin (HGB) level and body weight were measured 2, 4, 7, 10 and 14 days after administration.

Table 18: Rates of change in body weight of mice after administration (vs before administration)

| Days after administration | Hu5F9 10mpk | hz14A9-2.3 10mpk | hz3F10-6.1 10mpk |
|---|---|---|---|
| 0 | 0.0% | 0.0% | 0.0% |
| 2 | -7.0% | 2.3% | -13.0% |
| 4 | -3.2% | 6.3% | -15.8% |
| 7 | -5.1% | 2.0% | -2.5% |
| 10 | 4.8% | 5.9% | -8.4% |
| 14 | 5.6% | 8.6% | 8.2% |

Table 19: Rates of change in RBC of mice after administration (vs before administration)

| Days after administration | Hu5F9 10mpk | hz14A9-2.3 10mpk | hz3F10-6.1 10mpk |
|---|---|---|---|
| 0 | 0.0% | 0.0% | 0.0% |
| 2 | -52.8% | -13.6% | -56.7% |
| 4 | -61.8% | -16.7% | -75.6% |
| 7 | -22.2% | -9.8% | -38.7% |
| 10 | -21.6% | -8.5% | -6.6% |
| 14 | -7.4% | -2.2% | -2.0% |

Table 20: Rates of change in HGB level of mice after administration (vs before administration)

| Days after administration | Hu5F9 10mpk | hz14A9-2.3 10mpk | hz3F10-6.1 10mpk |
|---|---|---|---|
| 0 | 0.0% | 0.0% | 0.0% |
| 2 | -52.7% | -13.3% | -57.1% |
| 4 | -57.8% | -18.5% | -74.7% |
| 7 | -16.5% | -9.9% | -39.2% |
| 10 | -12.8% | -10.0% | -6.0% |
| 14 | -1.2% | -3.9% | -3.0% |

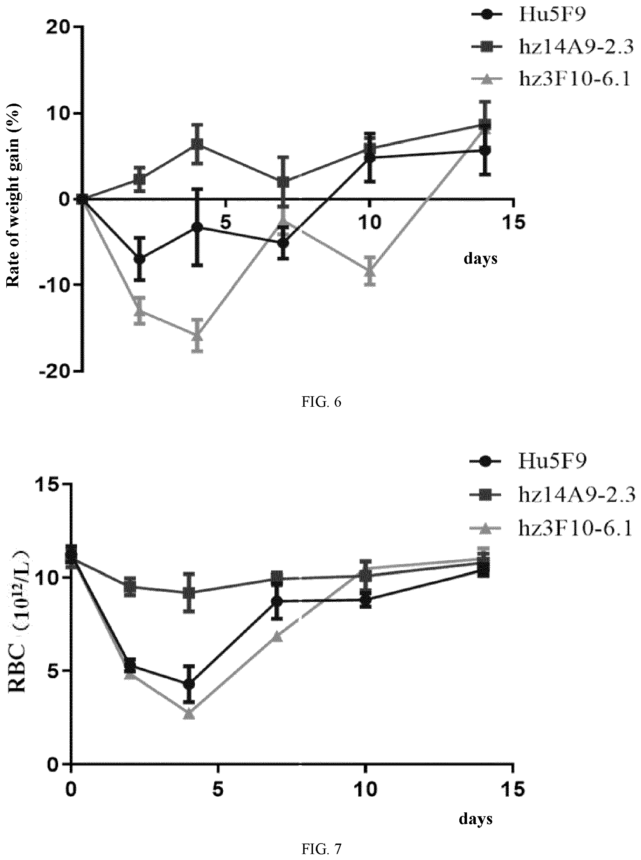

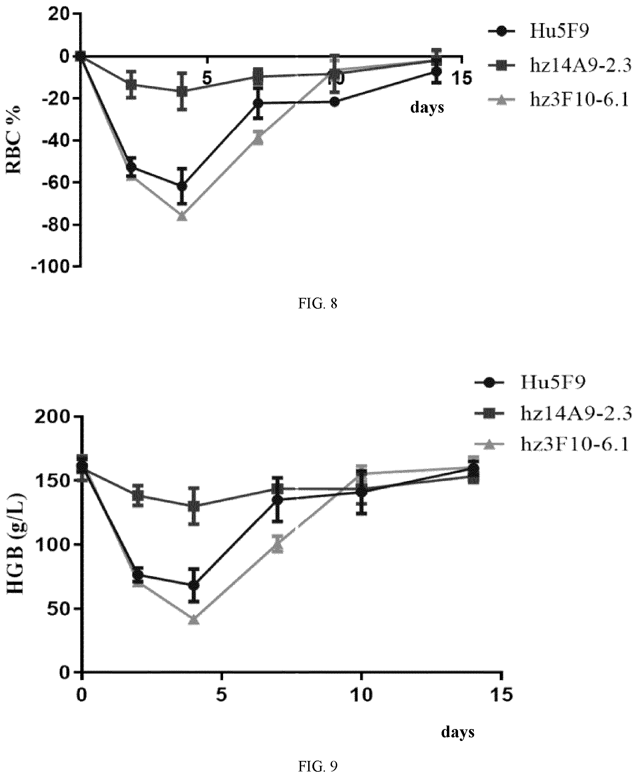

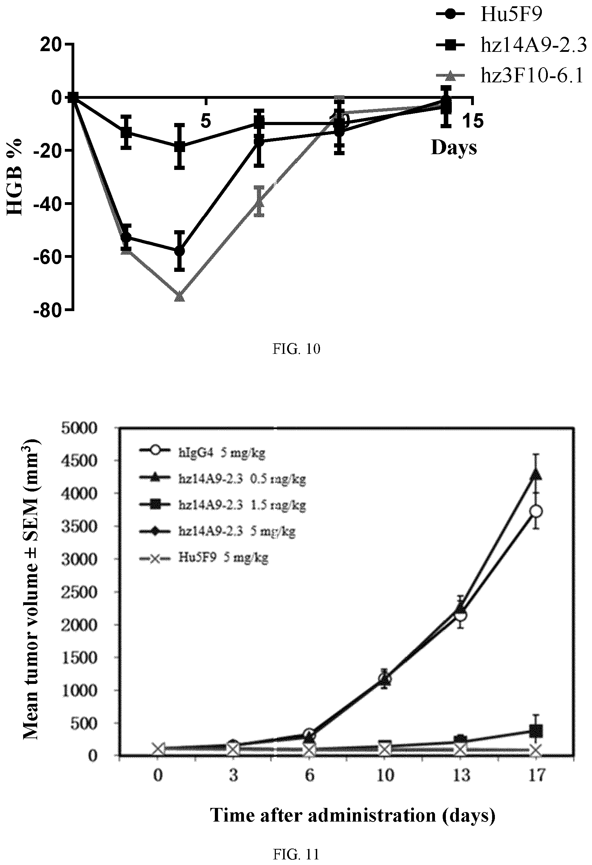

As shown in Table 18 and FIGs. 5 and 6, only the body weight of the mice in the hz14A9-2.3 group continuously increased, and the body weight of the mice in the Hu5F9 and hz3F10-6.1 groups decreased first and then increased after administration; the hz3F10-6.1 group had greater decreases than the Hu5F9 group. As shown in Tables 19-20 and FIGs. 7-10, the RBC and HGB levels in the animals of all the three groups decreased, to the minimums 4 days after administration, and then began to increase. The RBC and HGB levels in the hz14A9-2.3 group substantially returned to normal 7 days after administration, and those in the other two groups substantially returned to normal at least 10 days after administration. Arranged in descending order according to the decreases in RBC and HGB level 4 days after administration, the three groups are: hz3F10-6.1 > Hu5F9 > hz14A9-2.3. It can be seen that the antibody hz14A9-2.3 has no significant toxicity and is superior to the positive antibody Hu5F9.

**Example 14**

The anti-tumor activity of the antibodies was tested using a mouse model of human acute myeloid leukemia cells MOLM-16 cells. Each NOD-SCID mouse was inoculated subcutaneously with MOLM-16 cells, and after tumors grew to 80-150 mm$^3$, the animals were randomized into 5 groups: (1) model group (negative control group, given human IgG4, 5 mg/kg), n = 10 mice; (2) hz14A9-2.3 0.5 mg/kg group, n = 6 mice; (3) hz14A9-2.3 1.5 mg/kg group, n = 6 mice; (4) hz14A9-2.3 5 mg/kg group, n = 6 mice; and (5) Hu5F9 5 mg/kg group, n = 6 mice. On day 0 (D0), mice were divided into groups and dosed by intravenous injection, once every 3 days, for a total of two doses. Tumor diameters were measured twice a week, and the therapeutic effects were determined based on the tumor volume.

The tumor volume (V) was calculated as follows: $V = (a \times b^2)/2$, where a and b represent length and width, respectively.

$T/C\,(\%) = (T - T_0)/(C - C_0) \times 100$, where T and C represent the mean tumor volumes at the end of the experiment in the treatment group and negative control group, respectively; $T_0$ and $C_0$ represent the mean tumor volumes at the start of the experiment in the treatment group and negative control group, respectively.

$$\text{Tumor growth inhibition (TGI) (\%)} = 100\% - \text{T/C (\%)}.$$

The results are shown in Tables 21 and 11. The antibody hz14A9-2.3 can effectively inhibit the tumor growth in the dose of 1.5 mg/kg, and the tumor growth inhibition (or TGI) reached 93% on day 17 (D17). The tumor growth inhibition of the antibody hz14A9-2.3 reached 125% in the dose of 5 mg/kg.

Table 21

| Grouping | Administration | Route | Mean tumor volume (mm³) D0 | Standard error of the mean (SEM) D0 | Mean tumor volume (mm³) D17 | Standard error of the mean (SEM) D17 | T/C(%) D17 | Tumor growth inhibition (%) D17 | P value D17 | Partial regression | Number of animals per group (mice) at the beginning of the experiment | Number of animals per group (mice) at the end of the experiment |
|---|---|---|---|---|---|---|---|---|---|---|---|---|
| hIgG4 5mg/kg | D0,3 | IV | 107.8 | ±1.1 | 3732.0 | ±272.8 | - | - | - | 0 | 10 | 10 |
| hz14A9-2.3 0.5mg/kg | D0,3 | IV | 111.2 | ±2.5 | 4298.1 | ±291.1 | 116 | -16 | 0.200 | 0 | 6 | 6 |
| hz14A9-2.3 1.5mg/kg | D0,3 | IV | 110.6 | ±1.8 | 377.5 | +244.7 | 7 | 93 | 0.000 | 3 | 6 | 6 |
| hz14A9-2.3 5mg/kg | D0,3 | IV | 111.8 | ±2.0 | 84.2 | ±2.8 | -25 | 125 | 0.000 | 6 | 6 | 6 |
| Hu5F9 5mg/kg | D0,3 | IV | 110.8 | ±1.3 | 91.4 | ±2.4 | -18 | 118 | 0.000 | 6 | 6 | 6 |

**Claims**

1. An isolated antigen-binding polypeptide that binds to CD47 or an antigen-binding portion thereof, wherein the antigen-binding polypeptide or an antigen-binding portion thereof comprises the following complementarity determining regions:

   a heavy chain CDR1 comprising an amino acid sequence set forth in SEQ ID NO: 5;
   a heavy chain CDR2 comprising an amino acid sequence set forth in SEQ ID NO: 10;
   a heavy chain CDR3 comprising an amino acid sequence set forth in SEQ ID NO: 15;
   a light chain CDR1 comprising an amino acid sequence set forth in SEQ ID NO: 20;
   a light chain CDR2 comprising an amino acid sequence set forth in SEQ ID NO: 25; and
   a light chain CDR3 comprising an amino acid sequence set forth in SEQ ID NO: 30,
   wherein the antigen-binding polypeptide is a monoclonal antibody.

2. The antigen-binding polypeptide or the antigen-binding portion thereof according to claim 1, wherein the antigen-binding polypeptide or an antigen-binding portion thereof is selected from the group consisting of:

   (a) an antigen-binding polypeptide or an antigen-binding portion thereof comprising a heavy chain variable region comprising an amino acid sequence having at least 80% identity to the amino acid sequence of SEQ ID NO: 41, and a light chain variable region comprising an amino acid sequence having at least 80% identity to the amino acid sequence of SEQ ID NO: 42; and
   (b) an antigen-binding polypeptide or an antigen-binding portion thereof comprising a heavy chain variable region comprising an amino acid sequence having at least 80% identity to the amino acid sequence of SEQ ID NO: 87, and a light chain variable region comprising an amino acid sequence having at least 80% identity to the amino acid sequence of SEQ ID NO: 88; preferably, the antigen-binding polypeptide or an antigen-binding portion thereof comprises a heavy chain variable region and a light chain variable region, wherein the heavy chain variable region comprises the amino acid sequence of SEQ ID NO: 79, and the light chain variable region comprises the amino acid sequence of SEQ ID NO: 80;

   the heavy chain variable region comprises the amino acid sequence of SEQ ID NO: 41, and the light chain variable region comprises the amino acid sequence of SEQ ID NO: 42;
   the heavy chain variable region comprises the amino acid sequence of SEQ ID NO: 87, and the light chain variable region comprises the amino acid sequence of SEQ ID NO: 88; or
   the heavy chain variable region comprises the amino acid sequence of SEQ ID NO: 81, and the light chain variable region comprises the amino acid sequence of SEQ ID NO: 82.

3. The antigen-binding polypeptide or the antigen-binding portion thereof according to claim 1, wherein the antigen-binding polypeptide or an antigen-binding portion thereof comprises a heavy chain variable region with the amino acid sequence of SEQ ID NO: 79 and
   a light chain variable region with the amino acid sequence of SEQ ID NO: 80.

4. The antigen-binding polypeptide according to claim 1, wherein the antigen-binding polypeptide comprises a heavy chain set forth in SEQ ID NO: 181 and a light chain set forth in SEQ ID NO: 183;

   the antigen-binding polypeptide comprises a heavy chain set forth in SEQ ID NO: 105 and a light chain set forth in SEQ ID NO: 107; or
   the antigen-binding polypeptide comprises a heavy chain set forth in SEQ ID NO: 185 and a light chain set forth in SEQ ID NO: 187.

5. The antigen-binding polypeptide or the antigen-binding portion thereof according to any one of claims 1-4, wherein the antigen-binding polypeptide or the antigen-binding portion thereof is selected from the group consisting of multispecific antibodies, Fab fragments, Fab' fragments, F(ab')2 fragments, Fv fragments and scFv.

6. The antigen-binding polypeptide according to any one of claims 1-5. wherein the antigen-binding polypeptide is of IgG1, IgG2, IgG3 or IgG4 subclass.

7. The antigen-binding polypeptide according to claim 6, the antigen-binding polypeptide of IgG4 subclass has an S228P mutation according to the EU numbering index.

8. An isolated antigen-binding polypeptide that binds to CD47 according to claims 1 or 4, wherein the antigen-binding polypeptide comprises a heavy chain set forth in SEQ ID NO: 181 and a light chain set forth in SEQ ID NO: 183.

9. An immunoconjugate comprising the antigen-binding polypeptide or the antigen-binding portion thereof according to any one of claims 1-8 and a therapeutic agent linked or conjugated to the antigen-binding polypeptide or the antigen-binding portion thereof;
preferably, the therapeutic agent is a cytotoxic drug, a radioisotope, an immunomodulator or an antibody.

10. A composition comprising a component A and a pharmaceutically acceptable carrier, wherein the component A is the antigen-binding polypeptide or the antigen-binding portion thereof according to any one of claims 1-8 or the immunoconjugate according to claim 9.

11. An isolated nucleic acid encoding the antigen-binding polypeptide or the antigen-binding portion thereof according to any one of claims 1-8.

12. A vector comprising the isolated nucleic acid according to claim 11.

13. A host cell comprising the vector according to claim 12 or into whose genome the isolated nucleic acid according to claim 11 is integrated.

14. An antigen-binding polypeptide or an antigen-binding portion thereof according to any one of claims 1-8, the immunoconjugate according to claim 9, or the composition according to claim 10 for use in a method for reducing a tumor or inhibiting tumor cell growth in a subject, in a method for treating cancer in a subject in need thereof, or in a method for promoting macrophage phagocytosis in a subject.

15. The antigen-binding polypeptide or an antigen-binding portion thereof, the immunoconjugate, or the composition for use according to claim 14, wherein the cancer is selected from the group consisting of leukemia, lymphoma, ovarian cancer, breast cancer, endometrial cancer, colon cancer, rectal cancer, bladder cancer, urothelial cancer, lung cancer, bronchial cancer, bone cancer, prostate cancer, pancreatic cancer, gastric cancer, hepatocellular cancer, gallbladder cancer, bile duct cancer, esophageal cancer, renal cell cancer, thyroid cancer, head and neck cancer, testicular cancer, endocrine adenocarcinoma, adrenal cancer, pituitary gland cancer, skin cancer, soft tissue cancer, vascular cancer, brain cancer, neural cancer, eye cancer, meningeal cancer, oropharyngeal cancer, hypopharynx cancer, cervical cancer, and uterine cancer, glioblastoma, medulloblastoma, astrocytoma, glioma, meningioma, gastrinoma, neuroblastoma, melanoma, acute myeloid leukemia, myelodysplastic syndrome and sarcoma.

**Patentansprüche**

1. Isoliertes Antigen-bindendes Polypeptid, das an CD47 bindet, oder ein Antigen-bindender Teil davon, wobei das Antigen-bindende Polypeptid oder ein Antigen-bindender Teil davon die folgenden komplementaritätsbestimmenden Regionen umfasst:

eine CDR1 einer schweren Kette, die eine in SEQ ID NO: 5 angegebene Aminosäuresequenz umfasst;
eine CDR2 einer schweren Kette, die eine in SEQ ID NO: 10 angegebene Aminosäuresequenz umfasst;
eine CDR3 einer schweren Kette, die eine in SEQ ID NO: 15 angegebene Aminosäuresequenz umfasst;
eine CDR1 einer leichten Kette, die eine in SEQ ID NO: 20 angegebene Aminosäuresequenz umfasst;
eine CDR2 einer leichten Kette, die eine in SEQ ID NO: 25 angegebene Aminosäuresequenz umfasst; und
eine CDR3 einer leichten Kette, die eine in SEQ ID NO: 30 angegebene Aminosäuresequenz umfasst,
wobei das Antigen-bindende Polypeptid ein monoklonaler Antikörper ist.

2. Antigen-bindendes Polypeptid oder Antigen-bindender Teil davon gemäß Anspruch 1, wobei das Antigen-bindende Polypeptid oder ein Antigen-bindender Teil davon ausgewählt ist aus der Gruppe bestehend aus:

(a) einem Antigen-bindenden Polypeptid oder einem Antigen-bindenden Teil davon, umfassend eine variable Region einer schweren Kette, die eine Aminosäuresequenz mit mindestens 80% Identität zur Aminosäuresequenz von SEQ ID NO: 41 umfasst, und eine variable Region einer leichten Kette, die eine Aminosäuresequenz mit mindestens 80% Identität zur Aminosäuresequenz von SEQ ID NO: 42 umfasst; und
(b) einem Antigen-bindenden Polypeptid oder einem Antigen-bindenden Teil davon, umfassend eine variable Region einer schweren Kette, die eine Aminosäuresequenz mit mindestens 80% Identität zur Aminosäuresequenz von SEQ ID NO: 87 umfasst, und eine variable Region einer leichten Kette, die eine Aminosäuresequenz mit mindestens 80% Identität zur Aminosäuresequenz von SEQ ID NO: 88 umfasst;

wobei das Antigen-bindende Polypeptid oder ein Antigen-bindender Teil davon vorzugsweise eine variable Region einer schweren Kette und eine variable Region einer leichten Kette umfasst, wobei
die variable Region der schweren Kette die Aminosäuresequenz von SEQ ID NO: 79 umfasst und die variable Region der leichten Kette die Aminosäuresequenz von SEQ ID NO: 80 umfasst;
die variable Region der schweren Kette die Aminosäuresequenz von SEQ ID NO: 41 umfasst und die variable Region der leichten Kette die Aminosäuresequenz von SEQ ID NO: 42 umfasst;
die variable Region der schweren Kette die Aminosäuresequenz von SEQ ID NO: 87 umfasst und die variable Region der leichten Kette die Aminosäuresequenz von SEQ ID NO: 88 umfasst; oder
die variable Region der schweren Kette die Aminosäuresequenz von SEQ ID NO: 81 umfasst und die variable Region der leichten Kette die Aminosäuresequenz von SEQ ID NO: 82 umfasst.

3. Antigen-bindendes Polypeptid oder Antigen-bindender Teil davon gemäß Anspruch 1, wobei das Antigen-bindende Polypeptid oder ein Antigen-bindender Teil davon eine variable Region einer schweren Kette mit der Aminosäuresequenz von SEQ ID NO: 79 und eine variable Region einer leichten Kette mit der Aminosäuresequenz von SEQ ID NO: 80 umfasst.

4. Antigen-bindendes Polypeptid gemäß Anspruch 1, wobei

das Antigen-bindende Polypeptid eine in SEQ ID NO: 181 angegebene schwere Kette und eine in SEQ ID NO: 183 angegebene leichte Kette umfasst;
das Antigen-bindende Polypeptid eine in SEQ ID NO: 105 angegebene schwere Kette und eine in SEQ ID NO: 107 angegebene leichte Kette umfasst; oder
das Antigen-bindende Polypeptid eine in SEQ ID NO: 185 angegebene schwere Kette und eine in SEQ ID NO: 187 angegebene leichte Kette umfasst.

5. Antigen-bindendes Polypeptid oder Antigen-bindender Teil davon gemäß einem der Ansprüche 1 bis 4, wobei das Antigen-bindende Polypeptid oder der Antigen-bindende Teil davon aus der Gruppe ausgewählt ist, die aus multispezifischen Antikörpern, Fab-Fragmenten, Fab'-Fragmenten, F(ab')2-Fragmenten, Fv-Fragmenten und scFv besteht.

6. Antigen-bindendes Polypeptid gemäß einem der Ansprüche 1 bis 5, wobei das Antigen-bindende Polypeptid zur Unterklasse IgG1, IgG2, IgG3 oder IgG4 gehört.

7. Antigen-bindendes Polypeptid gemäß Anspruch 6, wobei das Antigen-bindende Polypeptid der IgG4-Unterklasse eine S228P-Mutation gemäß dem EU-Nummerierungsindex aufweist.

8. Isoliertes Antigen-bindendes Polypeptid, das an CD47 bindet, gemäß Anspruch 1 oder 4, wobei das Antigen-bindende Polypeptid eine in SEQ ID NO: 181 angegebene schwere Kette und eine in SEQ ID NO: 183 angegebene leichte Kette umfasst.

9. Immunkonjugat, umfassend das Antigen-bindende Polypeptid oder den Antigen-bindenden Teil davon gemäß einem der Ansprüche 1 bis 8 und ein therapeutisches Mittel, das mit dem Antigen-bindenden Polypeptid oder mit dem Antigen-bindenden Teil davon verknüpft oder daran konjugiert ist;
wobei das therapeutische Mittel vorzugsweise ein zytotoxisches Medikament, ein Radioisotop, ein Immunmodulator oder ein Antikörper ist.

10. Zusammensetzung, umfassend eine Komponente A und einen pharmazeutisch verträglichen Träger, wobei die Komponente A das Antigen-bindende Polypeptid oder der Antigen-bindende Teil davon gemäß einem der Ansprüche 1 bis 8 oder das Immunkonjugat gemäß Anspruch 9 ist.

11. Isolierte Nukleinsäure, die das Antigen-bindende Polypeptid oder den Antigen-bindenden Teil davon gemäß einem der Ansprüche 1 bis 8 kodiert.

12. Vektor, umfassend die isolierte Nukleinsäure gemäß Anspruch 11.

13. Wirtszelle, die den Vektor gemäß Anspruch 12 umfasst oder in deren Genom die isolierte Nukleinsäure gemäß Anspruch 11 integriert ist.

14. Antigen-bindendes Polypeptid oder Antigen-bindender Teil davon gemäß einem der Ansprüche 1 bis 8, Immunkonjugat gemäß Anspruch 9 oder Zusammensetzung gemäß Anspruch 10 zur Verwendung in einem Verfahren zur Verkleinerung eines Tumors oder zur Hemmung des Tumorzellwachstums in einem Subjekt, in einem Verfahren zur Behandlung von Krebs in einem Subjekt, das dies benötigt, oder in einem Verfahren zur Förderung der Makrophagen-Phagozytose in einem Subjekt.

15. Antigen-bindendes Polypeptid oder Antigen-bindender Teil davon, Immunkonjugat oder Zusammensetzung zur Verwendung gemäß Anspruch 14, wobei der Krebs aus der Gruppe ausgewählt ist, die aus Leukämie, Lymphom, Eierstockkrebs, Brustkrebs, Endometriumkarzinom, Darmkrebs, Rektumkrebs, Blasenkrebs, Urothelkarzinom, Lungenkrebs, Bronchialkrebs, Knochenkrebs, Prostatakrebs, Bauchspeicheldrüsenkrebs, Magenkrebs, Leberzellkarzinom, Gallenblasenkrebs, Gallengangskrebs, Speiseröhrenkrebs, Nierenzellkrebs, Schilddrüsenkrebs, Kopf-Hals-Krebs, Hodenkrebs, endokrinem Adenokarzinom, Nebennierenkrebs, Hypophysenkrebs, Hautkrebs, Weichteilkrebs, Gefäßkrebs, Hirntumor, Nervenkrebs, Augenkrebs, Hirnhautkrebs, Mund-Rachen-Krebs, Hypopharynxkrebs, Gebärmutterhalskrebs und Gebärmutterkrebs, Glioblastom, Medulloblastom, Astrozytom, Gliom, Meningeom, Gastrinom, Neuroblastom, Melanom, akuter myeloischer Leukämie, myelodysplastischem Syndrom und Sarkom besteht.

## Revendications

1. Polypeptide de liaison à l'antigène isolé qui se lie au CD47 ou une partie de liaison à l'antigène de celui-ci, dans lequel le polypeptide de liaison à l'antigène ou une partie de liaison à l'antigène de celui-ci comprend les régions déterminant la complémentarité suivantes :

   une CDR1 de chaîne lourde comprenant une séquence d'acides aminés décrite dans SEQ ID NO : 5 ;
   une CDR2 de chaîne lourde comprenant une séquence d'acides aminés décrite dans SEQ ID NO : 10 ;
   une CDR3 de chaîne lourde comprenant une séquence d'acides aminés décrite dans SEQ ID NO : 15 ;
   une CDR1 de chaîne légère comprenant une séquence d'acides aminés décrite dans SEQ ID NO : 20 ;
   une CDR2 de chaîne légère comprenant une séquence d'acides aminés décrite dans SEQ ID NO : 25 ; et
   une CDR3 de chaîne légère comprenant une séquence d'acides aminés décrite dans SEQ ID NO : 30,
   dans lequel le polypeptide de liaison à l'antigène est un anticorps monoclonal.

2. Polypeptide de liaison à l'antigène ou la partie de liaison à l'antigène de celui-ci selon la revendication 1, dans lequel le polypeptide de liaison à l'antigène ou une partie de liaison à l'antigène de celui-ci est choisi dans le groupe constitué par :

   (a) un polypeptide de liaison à l'antigène ou une partie de liaison à l'antigène de celui-ci comprenant une région variable de chaîne lourde comprenant une séquence d'acides aminés ayant au moins 80 % d'identité avec la séquence d'acides aminés de SEQ ID NO : 41, et une région variable de chaîne légère comprenant une séquence d'acides aminés ayant au moins 80 % d'identité avec la séquence d'acides aminés de SEQ ID NO : 42 ; et
   (b) un polypeptide de liaison à l'antigène ou une partie de liaison à l'antigène de celui-ci comprenant une région variable de chaîne lourde comprenant une séquence d'acides aminés ayant au moins 80 % d'identité avec la séquence d'acides aminés de SEQ ID NO : 87, et une région variable de chaîne légère comprenant une séquence d'acides aminés ayant au moins 80 % d'identité avec la séquence d'acides aminés de SEQ ID NO : 88 ;

   de préférence, le polypeptide de liaison à l'antigène ou une partie de liaison à l'antigène de celui-ci comprenant une région variable de chaîne lourde et une région variable de chaîne légère, dans laquelle
   la région variable de chaîne lourde comprend la séquence d'acides aminés de SEQ ID NO : 79, et la région variable de chaîne légère comprend la séquence d'acides aminés de SEQ ID NO : 80 ;
   la région variable de la chaîne lourde comprend la séquence d'acides aminés de SEQ ID NO : 41, et la région variable de la chaîne légère comprend la séquence d'acides aminés de SEQ ID NO : 42 ;
   la région variable de la chaîne lourde comprend la séquence d'acides aminés de SEQ ID NO : 87, et la région variable de la chaîne légère comprend la séquence d'acides aminés de SEQ ID NO : 88 ; ou
   la région variable de la chaîne lourde comprend la séquence d'acides aminés de SEQ ID NO : 81, et la région variable de la chaîne légère comprend la séquence d'acides aminés de SEQ ID NO : 82.

3. Polypeptide de liaison à l'antigène ou partie de liaison à l'antigène de celui-ci selon la revendication 1, dans lequel le polypeptide de liaison à l'antigène ou une partie de liaison à l'antigène de celui-ci comprend une région variable de chaîne lourde avec la séquence d'acides aminés de SEQ ID NO : 79 et une région variable de chaîne légère avec la séquence d'acides aminés de SEQ ID NO : 80.

4. Polypeptide de liaison à l'antigène selon la revendication 1, dans lequel

le polypeptide de liaison à l'antigène comprend une chaîne lourde décrite dans SEQ ID NO : 181 et une chaîne légère décrite dans SEQ ID NO : 183 ;
le polypeptide de liaison à l'antigène comprend une chaîne lourde décrite dans SEQ ID NO : 105 et une chaîne légère décrite dans SEQ ID NO : 107 ; ou
le polypeptide de liaison à l'antigène comprend une chaîne lourde décrite dans SEQ ID NO : 185 et une chaîne légère décrite dans SEQ ID NO : 187.

5. Polypeptide de liaison à l'antigène ou partie de liaison à l'antigène de celui-ci selon l'une quelconque des revendications 1 à 4, dans lequel le polypeptide de liaison à l'antigène ou la partie de liaison à l'antigène de celui-ci est choisi dans le groupe constitué par les anticorps multispécifiques, les fragments Fab, les fragments Fab', les fragments F(ab')2, les fragments Fv et les scFv.

6. Polypeptide de liaison à l'antigène selon l'une quelconque des revendications 1 à 5, dans lequel le polypeptide de liaison à l'antigène est de la sous-classe IgG1, IgG2, IgG3 ou IgG4.

7. Polypeptide de liaison à l'antigène selon la revendication 6, le polypeptide de liaison à l'antigène de la sous-classe IgG4 présentant une mutation S228P selon l'index de numérotation de l'EU.

8. Polypeptide de liaison à l'antigène isolé qui se lie au CD47 selon les revendications 1 ou 4, dans lequel le polypeptide de liaison à l'antigène comprend une chaîne lourde décrite dans SEQ ID NO: 181 et une chaîne légère décrite dans SEQ ID NO : 183.

9. Immunoconjugué comprenant le polypeptide de liaison à l'antigène ou la partie de liaison à l'antigène de celui-ci selon l'une quelconque des revendications 1 à 8 et un agent thérapeutique lié ou conjugué au polypeptide de liaison à l'antigène ou à la partie de liaison à l'antigène de celui-ci ;
de préférence, l'agent thérapeutique étant un médicament cytotoxique, un radio-isotope, un immunomodulateur ou un anticorps.

10. Composition comprenant un composant A et un support pharmaceutiquement acceptable, dans laquelle le composant A est le polypeptide de liaison à l'antigène ou la partie de liaison à l'antigène de celui-ci selon l'une quelconque des revendications 1 à 8 ou l'immunoconjugué selon la revendication 9.

11. Acide nucléique isolé codant pour le polypeptide de liaison à l'antigène ou la partie de liaison à l'antigène de celui-ci selon l'une quelconque des revendications 1 à 8.

12. Vecteur comprenant l'acide nucléique isolé selon la revendication 11.

13. Cellule hôte comprenant le vecteur selon la revendication 12 ou dans le génome de laquelle l'acide nucléique isolé selon la revendication 11 est intégré.

14. Polypeptide de liaison à l'antigène ou partie de liaison à l'antigène de celui-ci selon l'une quelconque des revendications 1 à 8, immunoconjugué selon la revendication 9 ou composition selon la revendication 10 destiné(e) à être utilisé(e) dans un procédé visant à réduire une tumeur ou à inhiber la croissance de cellules tumorales chez un sujet, dans un procédé visant à traiter un cancer chez un sujet qui en a besoin, ou dans un procédé visant à favoriser la phagocytose des macrophages chez un sujet.

15. Polypeptide se liant à un antigène ou une partie de celui-ci se liant à un antigène, immunoconjugué ou composition destiné(e) à être utilisé(e) selon la revendication 14, dans laquelle le cancer est choisi parmi le groupe constitué de la leucémie, du lymphome, du cancer de l'ovaire, du cancer du sein, du cancer de l'endomètre, du cancer du côlon, du cancer du rectum, du cancer de la vessie, du cancer urothélial, cancer du poumon, cancer bronchique, cancer des os, cancer de la prostate, cancer du pancréas, cancer gastrique, cancer hépatocellulaire, cancer de la vésicule biliaire, cancer des voies biliaires, cancer de l'œsophage, cancer des cellules rénales, cancer de la thyroïde, cancer de la tête et du cou, cancer des testicules, adénocarcinome endocrinien, cancer des surrénales, cancer de l'hypophyse, cancer de la peau, cancer des tissus mous, cancer vasculaire, cancer du cerveau, cancer neural, cancer de l'œil, cancer méningé, cancer oropharyngé, cancer hypopharyngien, cancer du col de l'utérus et cancer de l'utérus, glioblastome, médulloblastome, astrocytome, gliome, méningiome, gastrinome, neuroblastome, mélanome, leucémie myéloïde aiguë, syndrome myélodysplasique et sarcome.

ng/ml 10000 3333.33 1111.11 370.37 123.46 41.15 13.72 4.57

Xi2B2

Xi2H8

Xi3F10

Xi16E5

Xi14A9

Xi16E5-2

Hu5F9

IgG4

PBS

FIG. 1

| ng/ml | 1 | 2 | 3 | 4 | 5 | 6 | 7 | 8 | 9 | 10 | 11 | 12 |
|---|---|---|---|---|---|---|---|---|---|---|---|---|
| 10000 | | | | | | | | | | | | |
| 3333.33 | | | | | | | | | | | | |
| 1111.11 | | | | | | | | | | | | |
| 370.37 | | | | | | | | | | | | |
| 123.46 | | | | | | | | | | | | |
| 41.15 | | | | | | | | | | | | |
| 13.72 | | | | | | | | | | | | |
| 4.57 | | | | | | | | | | | | |

FIG. 2

FIG. 3

| | IgG4 | Hu5F9 | hz14A9-2.3 | hz3F10-6.1 |
|---|---|---|---|---|
| Median survival | 13.5 | 18.5 | 28.5 | 17.5 |

| IgG4 | |
|---|---|
| Hu5F9 | |
| Logrank test | |
| Chi-squared test | 13.91 |
| df | 1 |
| P value | 0.0002 |
| P-value summary | *** |
| Is there any significant difference between survival rate curves? | Yes |

| Hu5F9 | |
|---|---|
| hz14A9-2.3 | |
| Logrank test | |
| Chi-squared test | 4.313 |
| df | 1 |
| P value | 0.0378 |
| P-value summary | * |
| Is there any significant difference between survival rate curves? | Yes |

| Hu5F9 | |
|---|---|
| hz3F10-6.1 | |
| Logrank test | |
| Chi-squared test | 1.276 |
| df | 1 |
| P value | 0.2587 |
| P-value summary | ns |
| Is there any significant difference between survival rate curves? | No |

FIG. 4

days

FIG. 5

FIG. 6

FIG. 7

FIG. 8

FIG. 9

FIG. 10

Time after administration (days)

FIG. 11

# EP 4 130 042 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2015183874 A1
- US 20150183874 A1
- US 5225539 A
- CN 107001463 A
- CN 109422811 A

### Non-patent literature cited in the description

- **TSENG D. et al.** *Proceedings of the National Academy of Sciences*, 20 May 2013, vol. 110 (27), 11103-11108
- **CHOLAMIN, SHARARCH et al.** *Science Translational Medicine*, 15 March 2017
- **WARD et al.** *Nature*, 1989, vol. 341, 544-546
- **BIRD et al.** *Science*, 1988, vol. 242, 423-426
- **HUSTON et al.** *Proc. Natl. Acad. USA*, 1988, vol. 85, 5879-5883
- **E. MEYERS** ; **W. MILLER**. *Comput. Appl. Biosci.*, 1988, vol. 4, 11-17
- **NEEDLEMAN** ; **WUNSCH**. *J. Mol. Biol.*, 1970, 484-453
- **WATSON et al.** Molecular Biology of the Gene. Bengamin/Cummings Publication Company, 1987
- Methods in Molecular Biology. Humana Press
- **SAMBROOK et al.** Molecular Cloning: A Laboratory Manual. 1989
- **J. E. COLIGAN et al.** *Current Protocols in Immunology*, 1991
- **C. A. JANEWAY** ; **P. TRAVERS**. *Immunobiology*, 1997
- **P. FINCH**. *Antibodies*, 1997
- Antibodies: a practical approach. IRL Press, 1988-1989
- Monoclonal antibodies: a practical approach. Oxford University Press, 2000
- **C. BARBAS III et al.** Phage display: a laboratory manual. Cold Spring Harbor Laboratory Press, 2001
- Using antibodies: a laboratory manual. Cold Spring Harbor Laboratory Press, 1999